# EUROPEAN PATENT SPECIFICATION

(11) **EP 0 618 969 B1**
(45) Date of publication and mention of the grant of the patent: **25.06.2003**
(21) Application number: 92901213.6
(22) Date of filing: 13.12.1991
(51) Int. Cl.: C12N 15/13, C12N 5/10, A61K 39/395, A61K 47/48, A61K 49/00, A61K 51/10

(54) **COMPOSITE ANTIBODIES OF HUMAN SUBGROUP IV LIGHT CHAIN CAPABLE OF BINDING TO TAG-72**
AN TAG-72 BINDENDE ZUSAMMENGESETZTE ANTIKÖRPER MIT DER HUMANEN LEICHTEN KETTE DER UNTERGRUPPE-IV
COMPOSITES DE LA CHAINE LEGERE DU SOUS-GROUPE HUMAIN IV APTES A LA LIAISON AVEC TAG-72

(43) Date of publication of application: 12.10.1994
(73) Proprietor: THE DOW CHEMICAL COMPANY, Midland, Michigan 48674 (US)
(72) Inventor: MEZES, Peter, S., Midland, MI 48640 (US); RICHARD, Ruth, A., Midland, MI 48642 (US); JOHNSON, Kim, S., Midland, MI 48645 (US)
(74) Representative: Smart, Peter John
(86) International application number: AU9100583
(87) International publication number: WO93012231

(56) References cited:
- WO-A-89/00692
- WO-A-89/01783
- AU-A- 4 354 089
- AU-A- 4 429 989
- NUCLEIC ACIDS RESEARCH, vol. 13, no. 18, 1985, pages 6531-6544, XP002026269 MARSH ET AL: "DETECTION OF A UNIQUE HUMAN VKIV GERMLINE GENE BY A CLONED CDNA PROBE"
- WHITTLE, N. et al.: "Expression in COS cells of a mouse-human chimaeric B72.3 antibody", Protein Engineering, Volume 1, No. 6, pages 499-505, 1987.
- BRADY, R.L. et al.: "Crystallization and Preliminary X-ray Diffraction Study of a Chimaeric Fab'Fragment of Antibody Binding Tumour Cells", J MOL Biol, 219, pages 603-604, 1991.

## Description

The present invention is directed to the fields of immunology and genetic engineering.

The following information is provided for the purpose of making known information believed by the applicants to be of possible relevance to the present invention. No admission is necessarily intended, nor should be construed, that any of the following information constitutes prior art against the present invention.

Antibodies are specific immunoglobulin (Ig) polypeptides produced by the vertebrate immune system in response to challenges by foreign proteins, glycoproteins, cells, or other antigenic foreign substances. The binding specificity of such polypeptides to a particular antigen is highly refined, with each antibody being almost exclusively directed to the particular antigen which elicited it.

Two major methods of generating vertebrate antibodies are presently utilized:generation *in situ* by the mammalian B lymphocytes and generation in cell culture by B-cell hybrids. Antibodies are generated *in* *situ* as a result of the differentiation of immature B lymphocytes into plasma cells (see Gough (1981), Trends in Biochem Sci, 6:203 (1981). Even when only a single antigen is introduced into the immune system for a particular mammal, a uniform population of antibodies does not result, i.e., the response is polyclonal.

The limited but inherent heterogeneity of polyclonal antibodies is overcome by the use of hybridoma technology to create "monoclonal" antibodies in cell cultures by B cell hybridomas (see Kohler and Milstein (1975), Nature, 256:495-497). In this process, a mammal is injected with an antigen, and its relatively short-lived, or mortal, splenocytes or lymphocytes are fused with an immortal tumor cell line. The fusion produces hybrid cells or "hybridomas" which are both immortal and capable of producing the genetically-coded antibody of the B cell.

In many applications, the use of monoclonal antibodies produced in non-human animals is severely restricted where the monoclonal antibodies are to be used in humans. Repeated injections in humans of a "foreign" antibody, such as a mouse antibody, may lead to harmful hypersensitivity reactions, i.e., an anti-idiotypic, or human anti-mouse antibody (HAMA) response, (see Shawler *et al*. (1985), Journal of Immunology, 135:1530-1535. and Sear *et al*., J. Biol. Resp. Modifiers, 3:138-150).

Various attempts have already been made to manufacture human-derived monoclonal antibodies by using human hybridomas (see Olsson *et al*., Proc. Natl. Acad. Sci. U.S.A., **77:**5429 (1980) and Roder *et al*. (1986), Methods in Enzymology, **121:**140-167. Unfortunately, yields of monoclonal antibodies from human hybridoma cell lines are relatively low compared to mouse hybridomas. In addition, human cell lines expressing immunoglobulins are relatively unstable compared to mouse cell lines, and the antibody producing capability of these human cell lines is transient. Thus, while human immunoglobulins are highly desirable, human hybridoma techniques have not yet reached the stage where human monoclonal antibodies with required antigenic specificities can be easily obtained.

Thus, antibodies of nonhuman origin have been genetically engineered, or "humanized". Humanized antibodies reduce the HAMA response compared to that expected after injection of a human patient with a mouse antibody. Humanization of antibodies derived from nonhumans, for example, has taken two principal forms, i.e., chimerization where non-human regions of immunoglobulin constant sequences are replaced by corresponding human ones (see for example, USP 4,816,567 to Cabilly *et al*., Genentech) and grafting of complementarity determining regions (CDR) into human framework regions (FR) (see European Patent Office Application (EPO) 0 239 400 to Winter). Some researchers have produced Fv antibodies (USP 4,642,334 to Moore, DNAX) and single chain Fv (SCFV) antibodies (see USP 4,946,778 to Ladner, Genex).

The above patent applications only show the production of antibody fragments in which some portion of the variable domains is coded for by nonhuman 7 gene regions. Humanized antibodies to date still retain various portions of light and heavy chain variable regions of nonhuman origin: the chimeric, Fv and single chain Fv antibodies retain the entire variable region of nonhuman origin and CDR-grafted antibodies retain CDR of nonhuman origin.

Such nonhuman-derived regions are expected to elicit an immunogenic reaction when administered into a human patient (see Brüggemann *et al*. (1989), J. Exp. Med., **170**:2153-2157; and Lo Buglio (1991), Sixth International Conference on Monoclonal Antibody Inaunoconjugates for Cancer, San Diego, Ca). Thus, it is most desirable to obtain a human variable region which is capable of binding to a selected antigen.

One known human carcinoma tumor antigen is tumor-associated glycoprotein-72 (TAG-72), as defined by monoclonal antibody B72.3 (see Thor *et al*. (1986) Cancer Res., **46**:3118-3124; and Johnson, *et al*. (1986), Cancer Res., **46**:850-857). TAG-72 is associated with the surface of certain tumor cells of human origin, specifically the LS174T tumor cell line (American Type Culture Collection (ATCC) No. CL 188), which is a variant of the LS180 (ATCC No. CL 187) colon adenocarcinoma line.

Numerous murine monocional antibodies have been developed which have binding specificity for TAG-72. Exemplary murine monoclonal antibodies include the "CC" (colon cancer) monoclonal antibodies, which are a library of murine monoclonal antibodies developed using TAG-72 purified on an immunoaffinity column with an immobilized anti-TAG-72 antibody, 372.3 (ATCC HB-8108) (see EP 394277, to Schlom *et al*., National Cancer Institute). Certain CC antibodies were deposited with the ATCC: CC49 (ATCC No. HB 9459); CC83 (ATCC No. HB 9453); CC46 (ATCC No. HB 9458); CC92 (ATCC No. HB 9454); CC30 (ATCC NO. HB 9457); CC11 (ATCC No. 9455) and CC15 (ATCC No. HB 9460). Various antibodies of the CC series have been chimerized (see for example EPO 0 365 997 to Mezes et al., The Dow Chemical Company).

WO 90/04410 discloses a family of chimeric antibodies with affinity to TAG-72. Here, the V_{L} is preferably human and the V_{H} can be derived from V_{H} αTAG-encoded antibodies CC46, 49, 83 or 92.

WO 89/01783 discloses a humanised antibody in which at least the CDRs and optionally the whole variable region are derived from murine anti-TAG-72 antibody B72.3 and the rest are derived from human antibodies.

It is thus of great interest to develop antibodies against TAG-72 containing a light and/or heavy chain variable region(s) derived from human antibodies. However, the prior art simply does not teach recombinant and immunologic techniques capable of routinely producing an anti-TAG-72 antibody in which the light chain and/or the heavy chain variable regions have specificity and affinity for TAG-72 and which are derived from human sequences so as to elicit expectedly low or no HAMA response. It is known that the function of an immunoglobulin molecule is dependent on its three dimensional structure which in turn is dependent on its primary amino acid sequence. A change of a few or even one amino acid can drastically affect the binding function of the antibody, i.e, the resultant antibodies are generally presumed to be a non-specific immunoglobulin (NSI), i.e. lacking in antibody character, (see for example, USP 4,816,567 Fo Cabilly et al., Genentech).

Surprisingly, the present invention is capable of meeting many of these above mentioned needs and provides a method for supplying the desired antibodies.

The invention provides a composite Hum4 V_{L}, V_{H} antibody or immunoreactive fragment thereof having binding affinity for TAG-72, comprising:-
a) a light chain variable region (V_{L}) of human kappa Subgroup IV, said V_{L} containing the human Subgroup IV germline gene (Hum4 V_{L}) encoding amino acid sequence, 
Asp Ile Val Met Thr Gln Ser Pro Asp Ser Leu Ala Val
Ser Leu Gly Glu Arg Ala Thr Ile Asn Cys Lys Ser Ser
Gln Ser Val Leu Tyr Ser Ser Asn Asn Lys Asn Tyr Leu
Ala Trp Tyr Gln Gln Lys Pro Gly Gln Pro Pro Lys Leu
Leu Ile Tyr Trp Ala Ser Thr Arg Glu Ser Gly Val Pro
Asp Arg Phe Ser Gly Ser Gly Ser Gly Thr Asp Phe Thr
Leu Thr Ile Ser Ser Leu Gln Ala Glu Asp Val Ala Val
Tyr Tyr Cys Gln Gln Tyr Tyr Ser Tyr Pro Leu Thr Phe
Gly Gly Gly Thr Lys Val Val Ile Lys,
 and
b) a heavy chain variable region (V_{H}), said V_{H} being encoded by a DNA coding sequence at least 90% homologous to the V_{H}αTAG germline gene (V_{H}αTAG) coding sequence,
wherein the V_{H} is combined with the V_{L} to form a three dimensional antigen binding structure retaining at least the same ability to bind TAG-72, as does a three dimensional antigen binding structure formed by combination of the V_{H} encoded by V_{H}αTAG and the V_{L} encoded by Hum4V_{L}.

The invention further includes the aforementioned antibody alone or conjugated to an imaging marker or therapeutic agent. The invention also includes a composition comprising the aforementioned antibody in unconjugated or conjugated form in a pharmaceutically acceptable, non-toxic, sterile carrier.

The invention is also directed to such compositions for use as diagnostic agents or therapeutics, or for the manufacture of compositions for use in methods of in vivo diagnosis or therapy of cancer. In vivo diagnosis of cancer may be carried out by administering to an animal containing a tumor expressing TAG-72 a pharmaceutically effective amount of the aforementioned composition for the in situ detection of carcinoma lesions.

Intraoperative therapy may be carried out by (a) administering to patient containing a tumour expressing TAG-72 a pharmaceutically effective amount of the aforementioned composition, whereby the tumour is localised, and (b) excising the localised tumours.

In a composite Hum4V_{L}, V_{H} antibody or immunoreactive fragment thereof of the invention, the V_{L} may be further encoded by a human J gene segment.

The V_{H} may be further encoded by a non-human animal D gene segment and an animal J gene segment.

The heavy chain variable region (V_{H}) is preferably from the heavy chain variable region of any one of CC46, CC49, CC83 or CC92.

The V_{H} preferably comprises (1) complementarity determining regions (CDR) being encoded by V_{H}αTAG and (2) framework segments, adjacent to the CDR segments, encoded by human genes.

The light chain may further comprise a human light chain constant region (C_{L}) and the heavy chain may further comprise an animal heavy chain region (C_{H}).

The C_{H} may be IgG1-4, IgM, IgA1, IgA2, IgD or IGE.

C_{L} may be kappa or lambda.

The invention further provides a composite Hum4 V_{L}, V_{H} single chain antibody or immuno-reactive fragment thereof having binding affinity for TAG-72, comprising (a) a light chain having a variable region (V_{L}) as defined above, (b) a heavy chain variable region (V_{H}) as defined above and (c) a polypeptide linker linking the V_{H} and V_{L}, wherein the polypeptide linker properly folds the V_{H} and V_{L} into a single chain antibody having the ability to bind TAG-72.

The composite Hum4 V_{L}, V_{H} antibody conjugates of the invention may comprise as an imaging marker ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re, or ^{99m}Tc.

Composite Hum4 V_{L}, V_{H} antibody conjugates of the invention may comprise as a therapeutic agent a drug or biological response modifier, radionuclide, or toxin. Such a drug may be methotrexate, adriamycin or interferon.

Composite Hum4 V_{L}, V_{H} antibody conjugates of the invention may comprise as a radionuclide ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I or ¹¹¹In.

The invention includes a cell capable of expressing a composite Hum4 V_{L}, V_{H} antibody or immunoreactive fragment thereof of the invention, said cell being transformed with
(a) a first DNA sequence encoding at least a light chain variable region (V_{L}) as defined above; and
(b) a second DNA sequence encoding at least a heavy chain variable region (V_{H}) as defined above, capable of combining with the V_{L} to form a three-dimensional structure having the ability to bind TAG-72.

In such a cell the first and second DNA sequences may be contained within at least one biologically functional expression vector.

The invention further includes a process for producing a composite Hum4 V_{L}, V_{H} antibody comprising at least the variable domains of the antibody heavy and light chains, in a single host cell, comprising the steps of:
(a) transforming at least one host cell with
   (i) a first DNA sequence encoding at least a light chain variable region (V_{L}) as defined above; and
   (ii) a second DNA sequence encoding at least a heavy chain variable region (V_{H}) as defined above which is capable of combining with the V_{L} to form a three-dimensional structure having the ability to bind TAG-72, and
(b) expressing, optionally independently expressing, said first DNA sequence and said second DNA sequence in said transformed single host cell.

Said first and second DNA sequences may be present in at least one vector.

The antibody heavy and light chains of the composite Hum4 V_{L}, V_{H} antibody may be expressed in the host cell and may then be secreted therefrom as an immunologically functional antibody molecule or antibody fragment.

Preferably, the second DNA sequence encodes the V_{H} of CC46, CC49, CC83 or CC92.

The invention further includes a process for preparing an antibody or antibody fragment conjugate which comprises contacting:
a composite Hum4 V_{L}, V_{H} antibody of the invention with an imaging marker or therapeutic agent, which may be ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re, or ^{99m}Tc as an imaging marker or a radionuclide, drug or biological response modifier, toxin or another antibody as a therapeutic agent.

### Description of the Drawings

Figure 1 illustrates a basic immunoglobulin structure.
Figure 2 illustrates the nucleotide sequences of V_{H}αTAG, CC46 V_{H}, CC49 V_{H}, CC83 V_{H} and CC92 V_{H}.
Figure 3 illustrates the amino acid sequences of V_{H}αTAG, CC46 V_{H}, CC49 V_{H}, CC83 V_{H} and CC92 V_{H}.
Figure 4 illustrates the V_{H} nucleotide and amino acid sequences of antibody B17X2.
Figure 5 illustrates the mouse germline J-H genes from pNP9.
Figure 6 illustrates the plasmid map of p49 g1-2.3.
Figure 7 illustrates the plasmid map of p83 g1-2.3.
Figure 8 illustrates the entire sequence of HUMVL (+) and HUMVL (-).
Figure 9 illustrates the human J4 (HJ4) nucleotide sequence and amino acid sequence.
Figure 10 illustrates the nucleotide sequences, and the amino acid sequence of Hum4 V_{L}, *Cla*I*-Hind*III segment.
Figure 11 illustrates a schematic representation of the human germline Subgroup IV V_{L} gene (Hum4 V_{L}), as the target for the PCR.
Figure 12 shows the results of agarose gel electrophoresis of the PCR reaction to obtain the Hum4 V_{L} gene. Lane identifications are as follows:-
   Lane 1: *λHind* III Standard; lane 2: no Taq polymerase control; lane 3: no primers added; lane 4: no human DNA template; lane 5: Gene *Amp* kit positive control; lane 6: 3 µg human DNA with primers and Taq polymerase; lane 7: same as lane 6, but with lug human DNA and lane 8: ØX174- *Hae* III DNA standard. Ethidium bromide was added to the gel and buffer. Bands were visualised by long wavelength UV light.

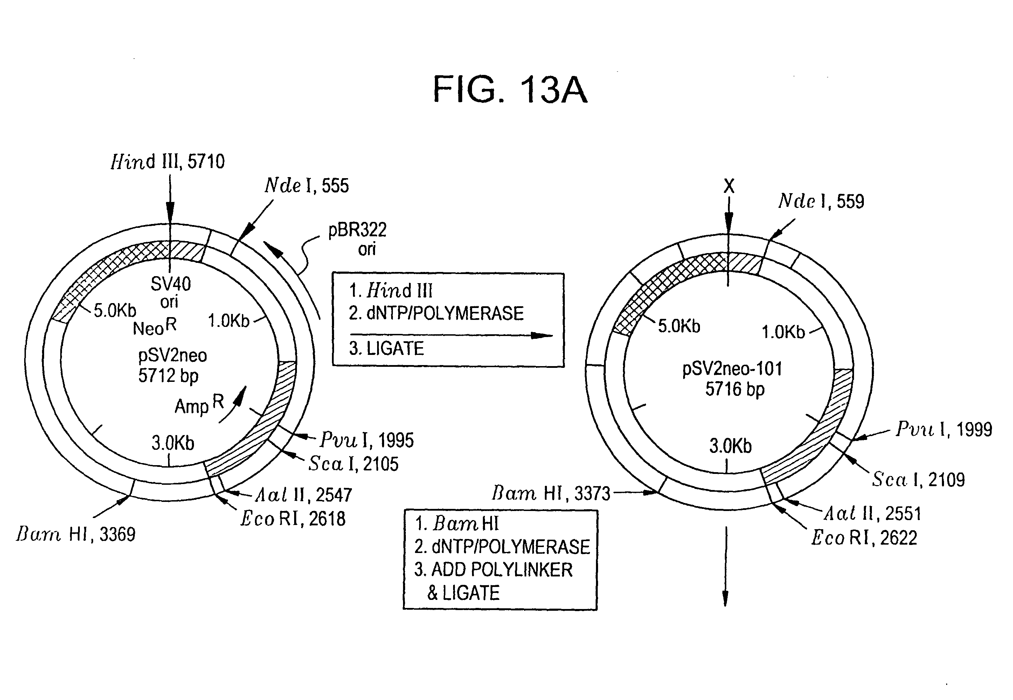

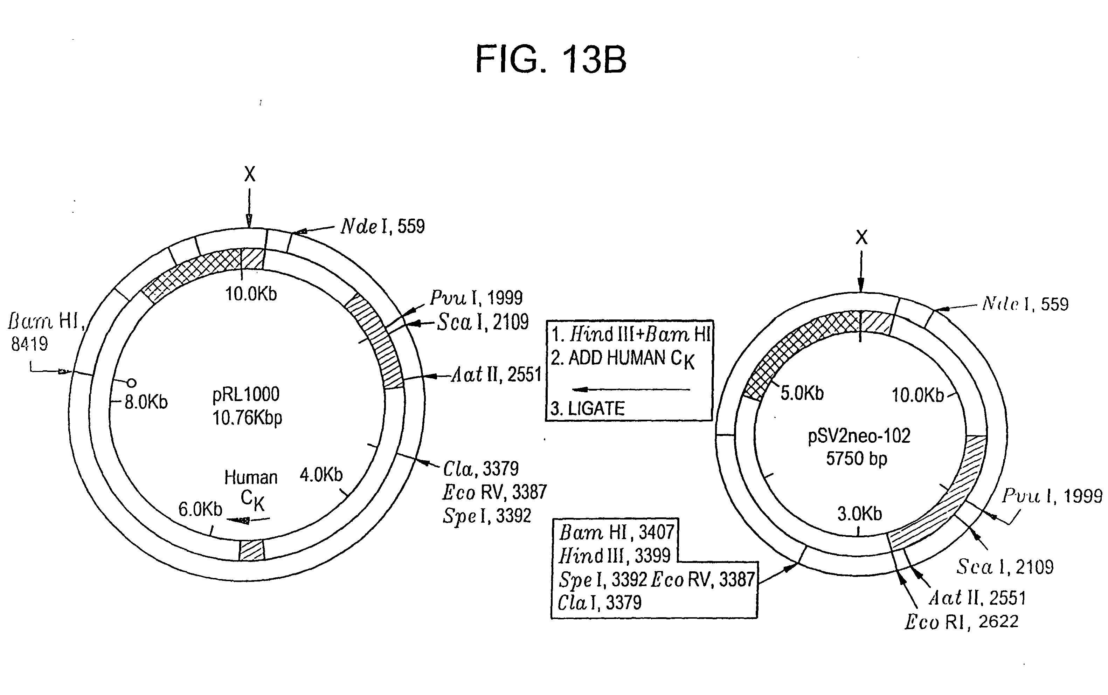

Figure 13 (A+B) illustrates the restriction enzyme map of pRL1000. and precursor plasmids pSV2neo, pSV2neo-101 and pSV2neo-102. "X" indicates where the *Hind*III site of pSV2neo has been destroyed.

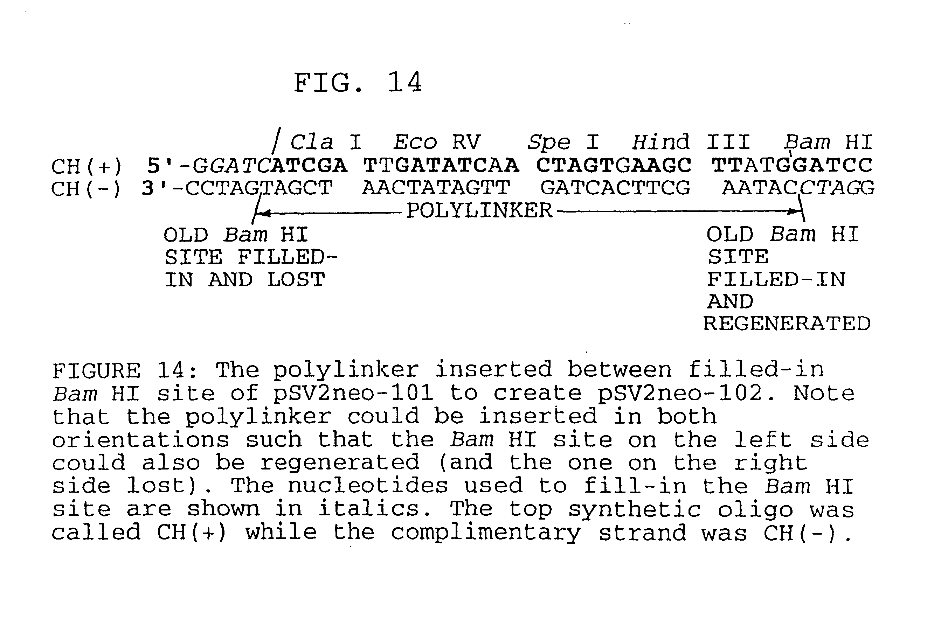

Figure 14 illustrates a polylinker segment made by snythesizing two oligonucieotides: CH(+) and CH(-).

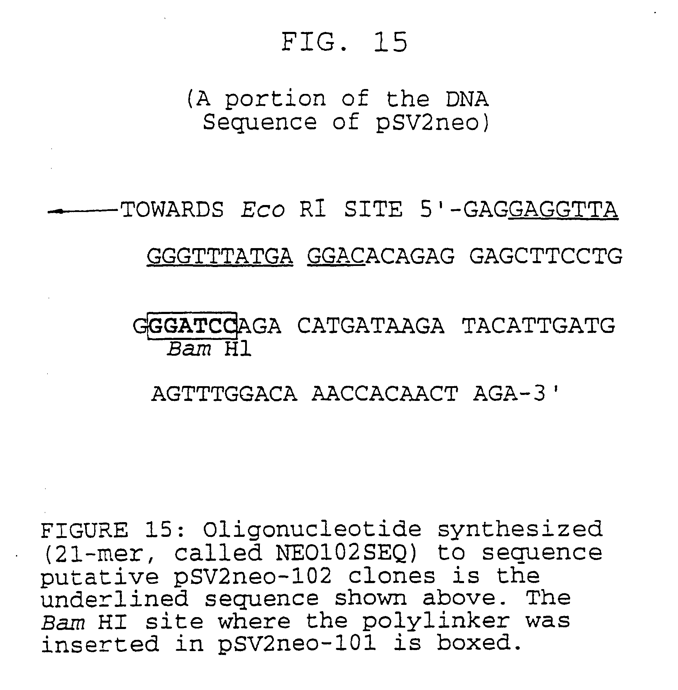

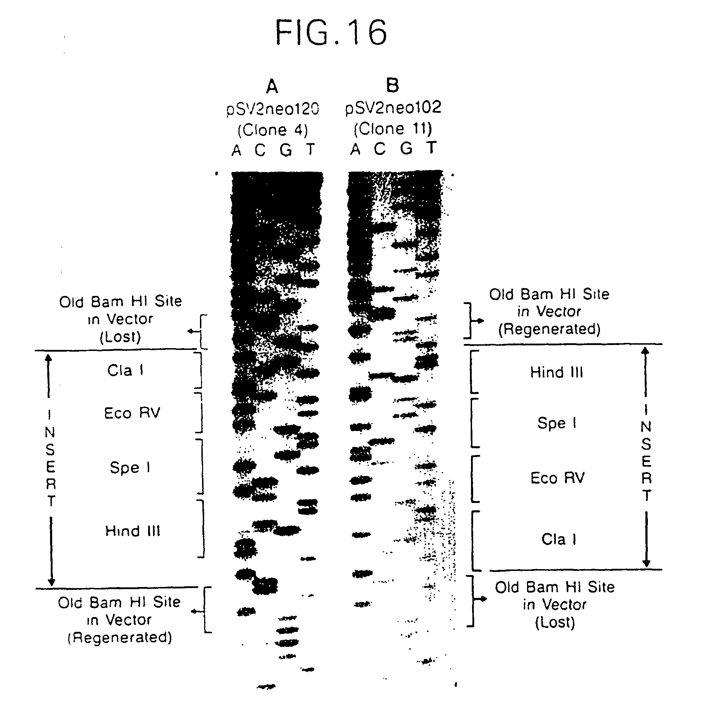

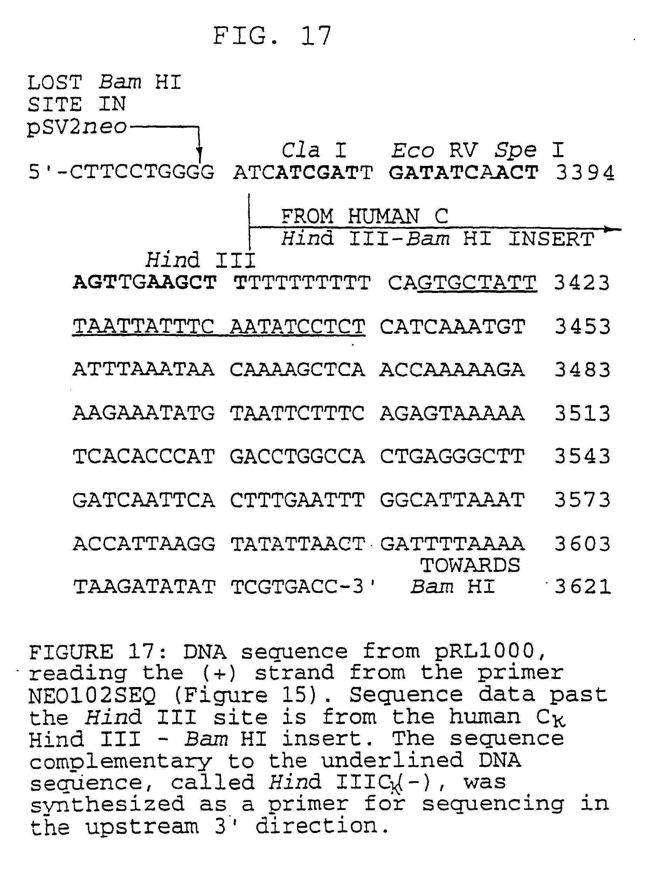

Figures 15 illustrates a primer, NEO102SEQ, used for sequencing plasmid DNA from several clones of pSV2neo-102.
Figure 16 illustrates a P-32 autoradiogram showing polylinker DNA sequence cloned in *Bam* HI site of pSV2neo-101. In both cases a single 30-base linker unit was incorporated, but in opposite orientations. Panel A-Sequence resulting in pSV2neo-120; Panel B-sequence resulting in pSV2neo-102. Reading the sequence (going up) is in the 5' to 3' direction of the (+) strand.
Figure 17 illustrates a partial mucleotide sequence segment of pRL1000.

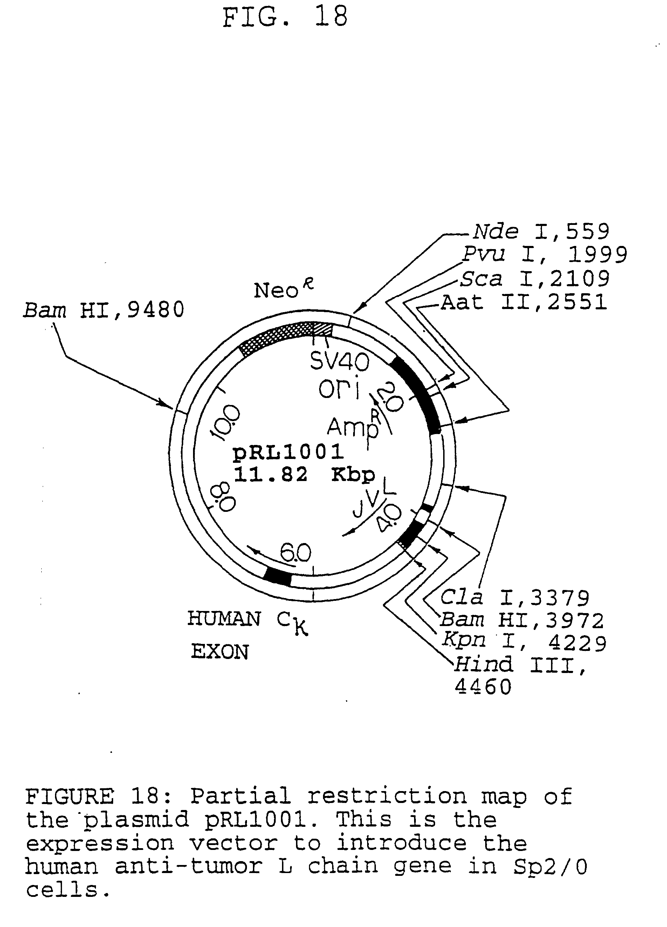

Figure 18 illustrates the restriction enzyme map of pRL1001.

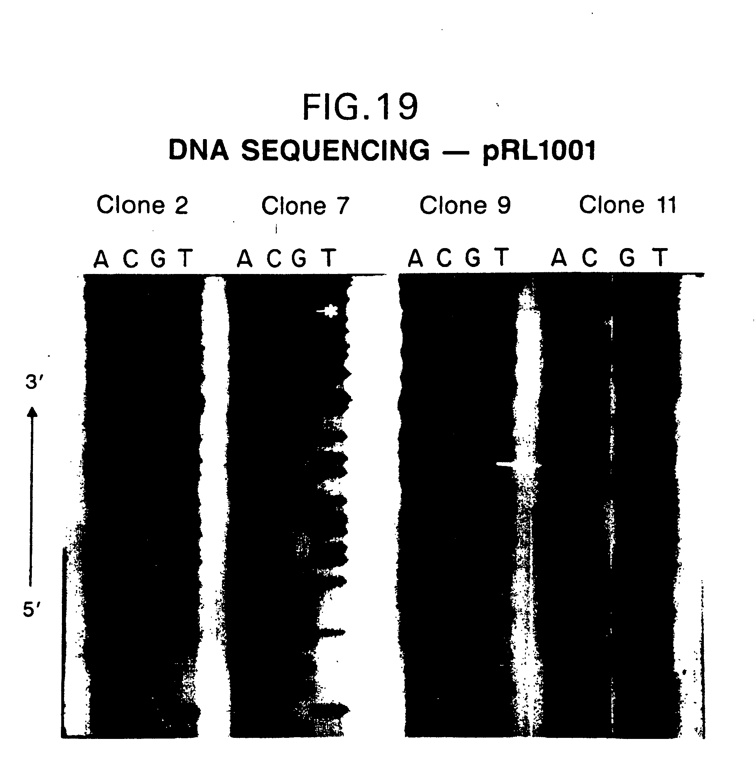

Figure 19 illustrates a DNA sequence autoradiogram of pRL1001 clones. Reading the gel is in the 5' to 3' direction on the (-) strand, from the *Hind* III Cκ(-) primer. Clones 2 and 9 were equivalent to the expected sequence, clone 7 had a single base substitution (marked by *) and clone 11 had a single base deletion (marked by -).

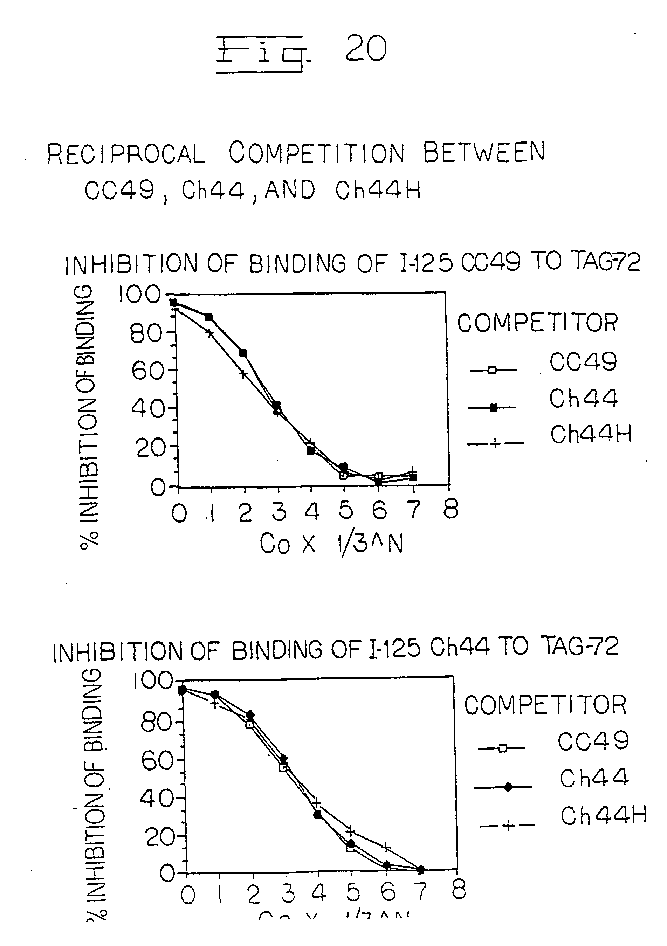

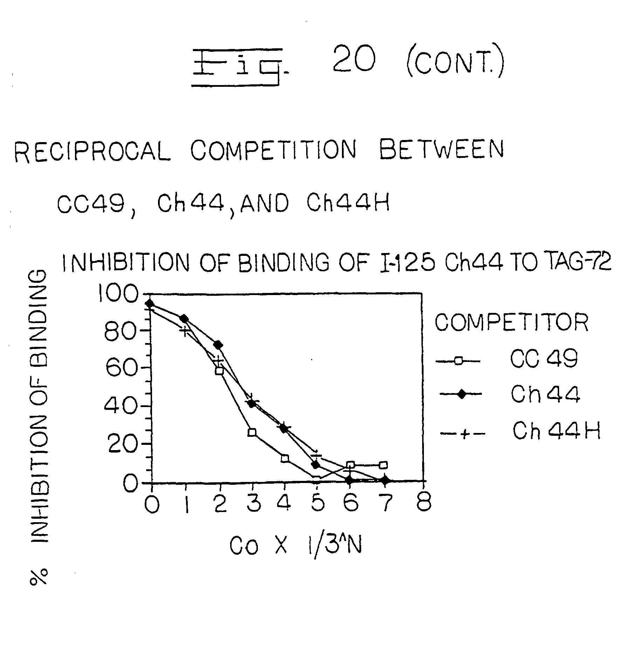

Figure 20 illustrates a competition assay for binding to TAG-using a composite Hum4 V_{L}, V_{H}αTAG antibody.

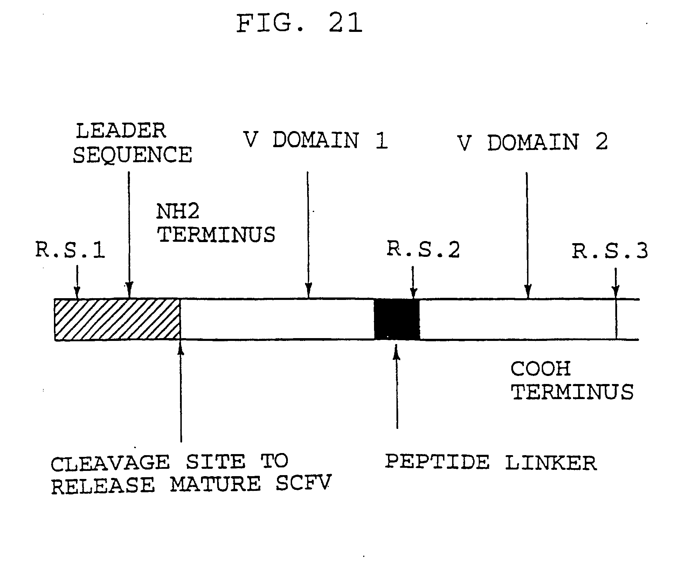

Figure 21 illustrates a general DNA construction of a single chain, composite Hum4 V_{L}, V_{H}αTAG.

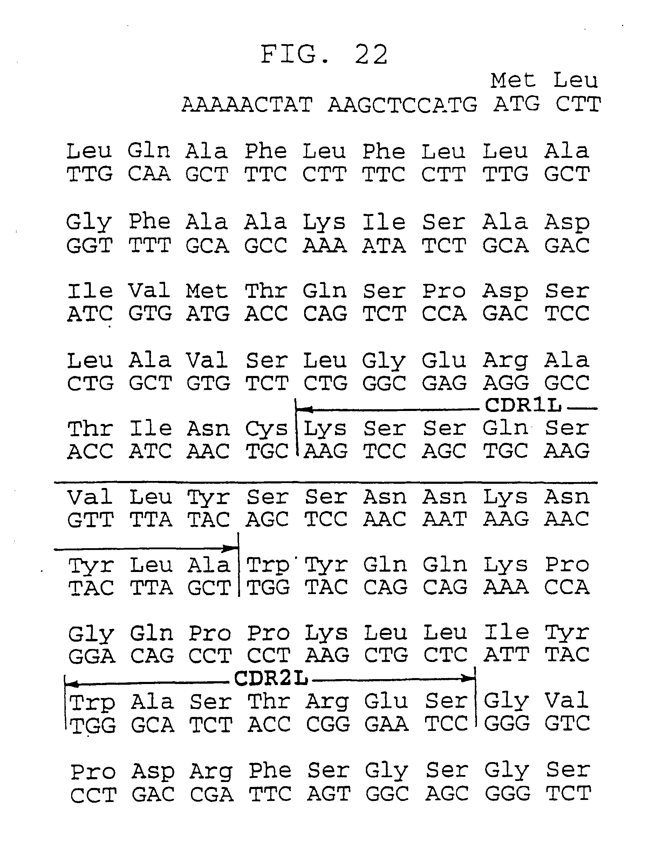

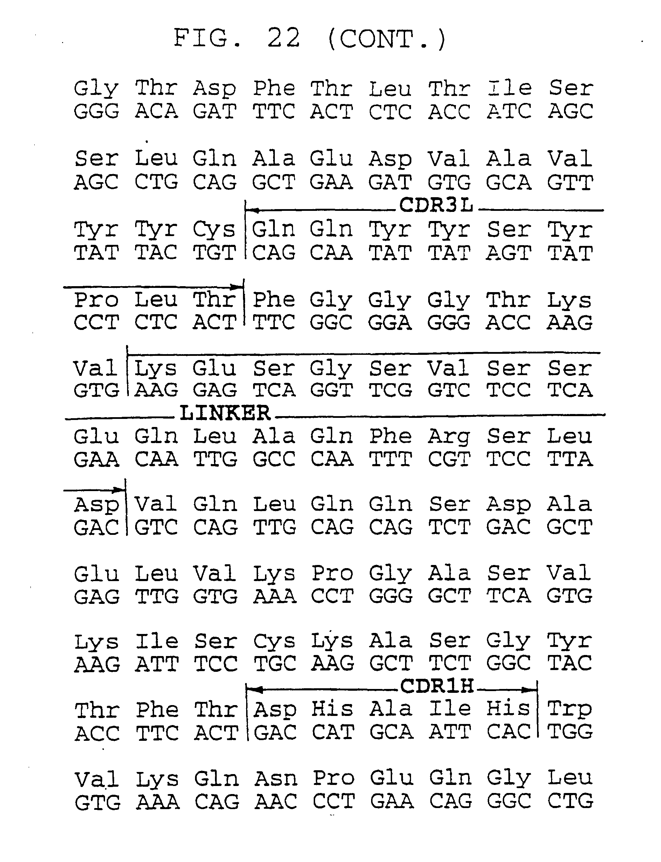

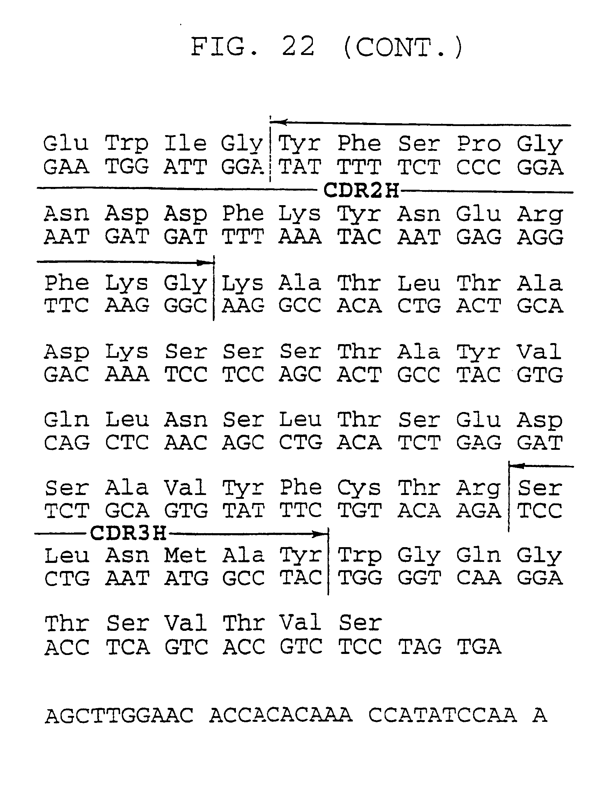

Figure 22 illustrates the nucleotide sequence and amino acid sequence of SCFV1.

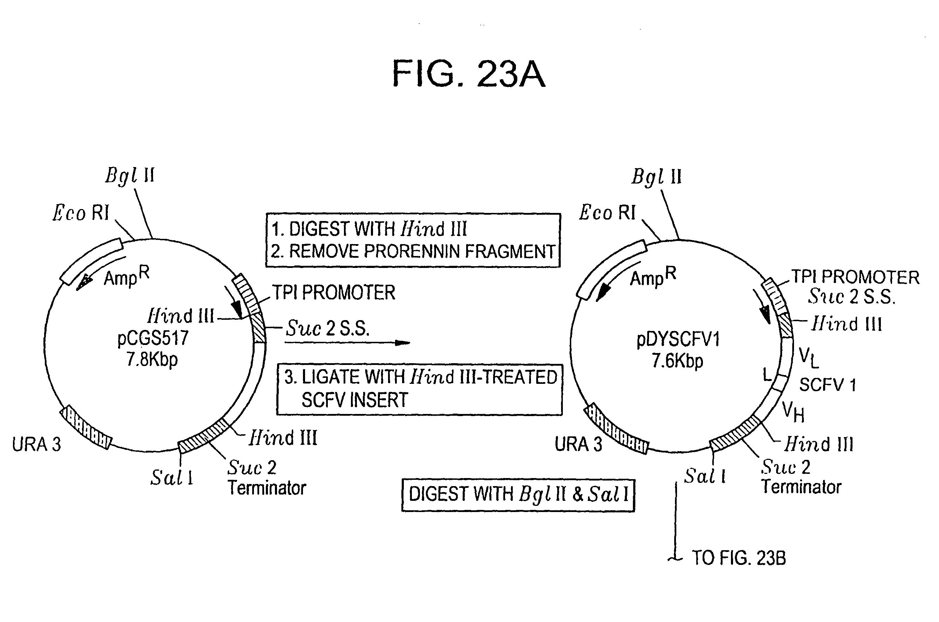

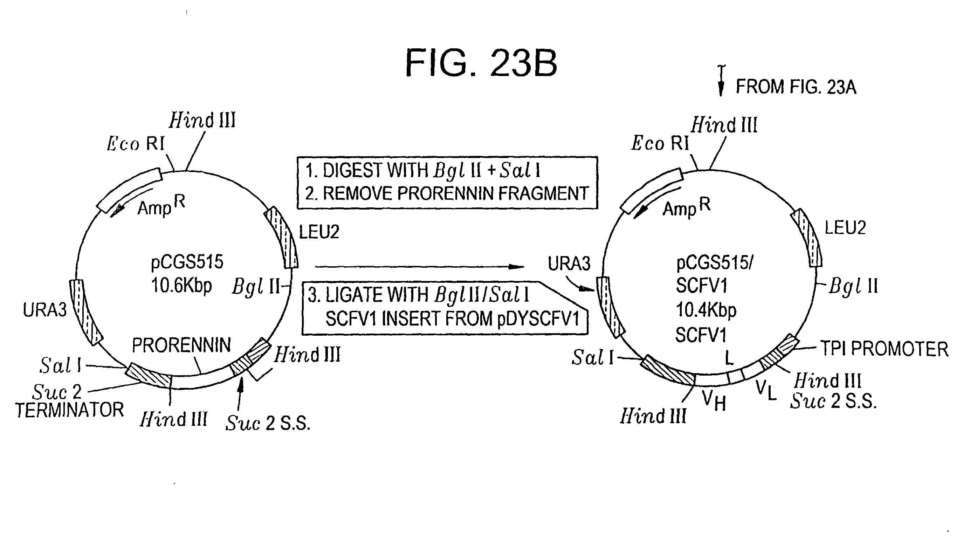

Figure 23 (A+B) shows the construction of plasmid pCGS515/SCFV1.

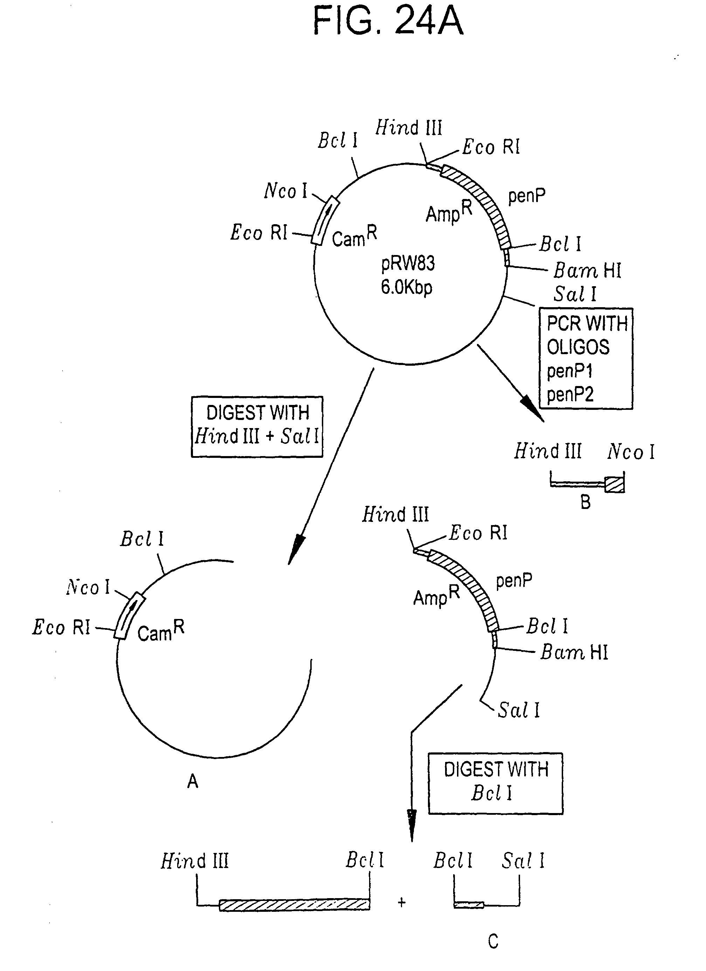

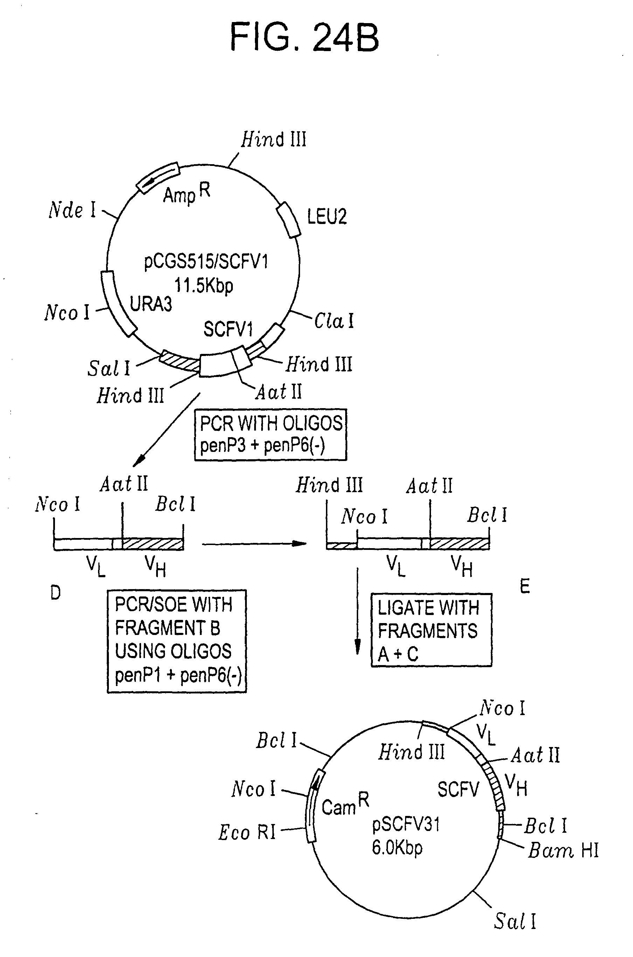

Figure 24 (A+B) shows the construction of plasmid pSCFV31.

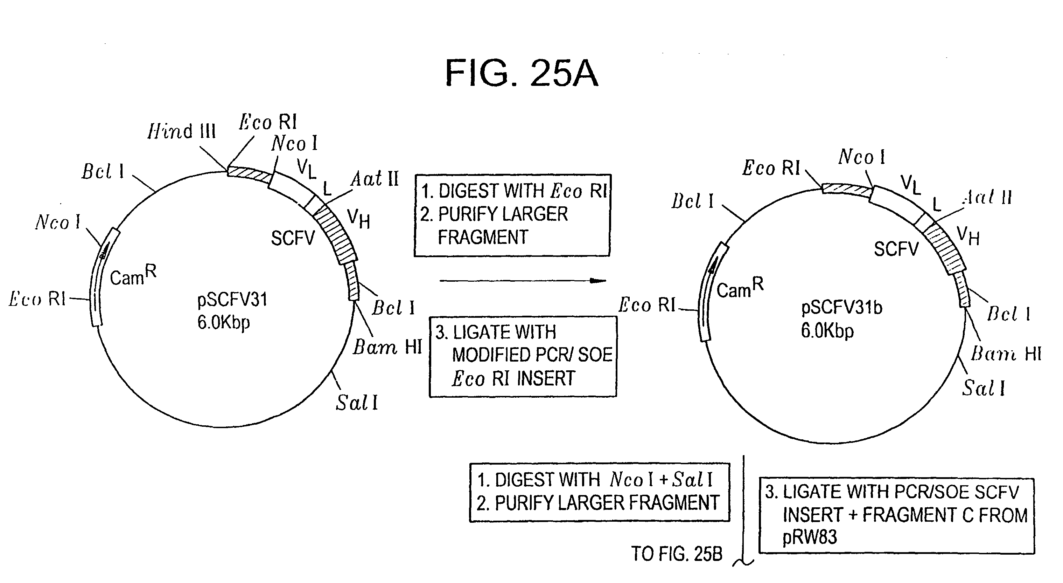

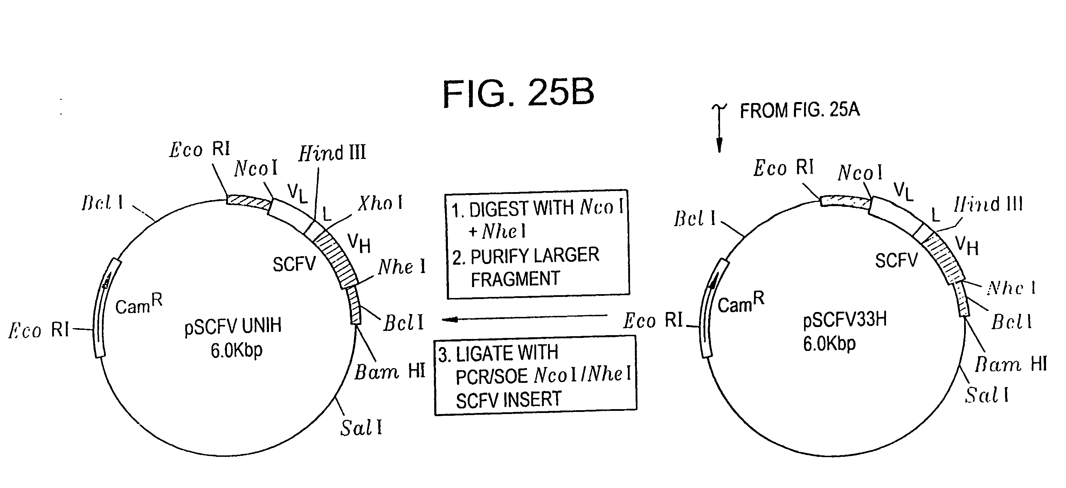

Figure 25 (A+B) shows the construction of E. coli SCFV expression plasmids containing Hum4 V_{L}.

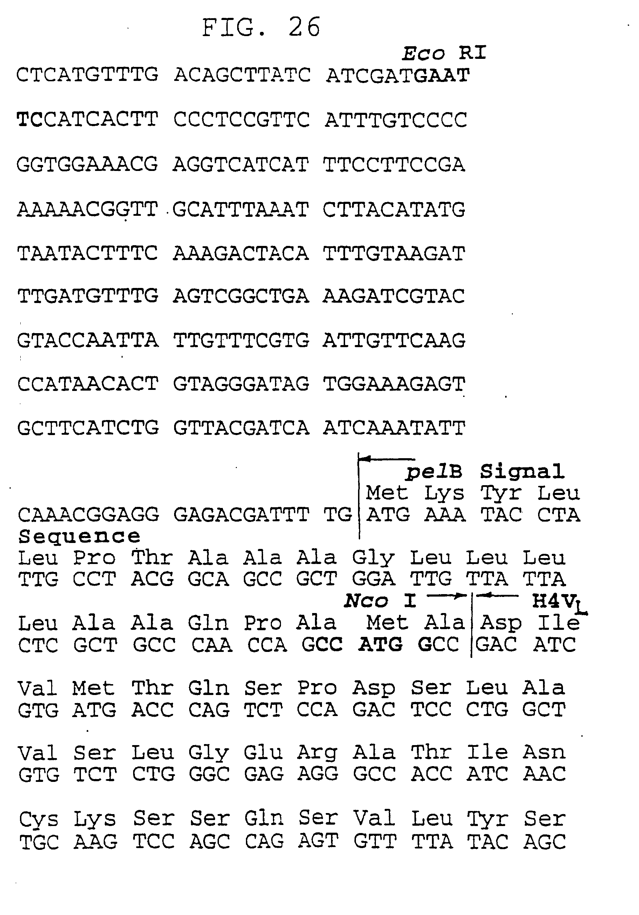

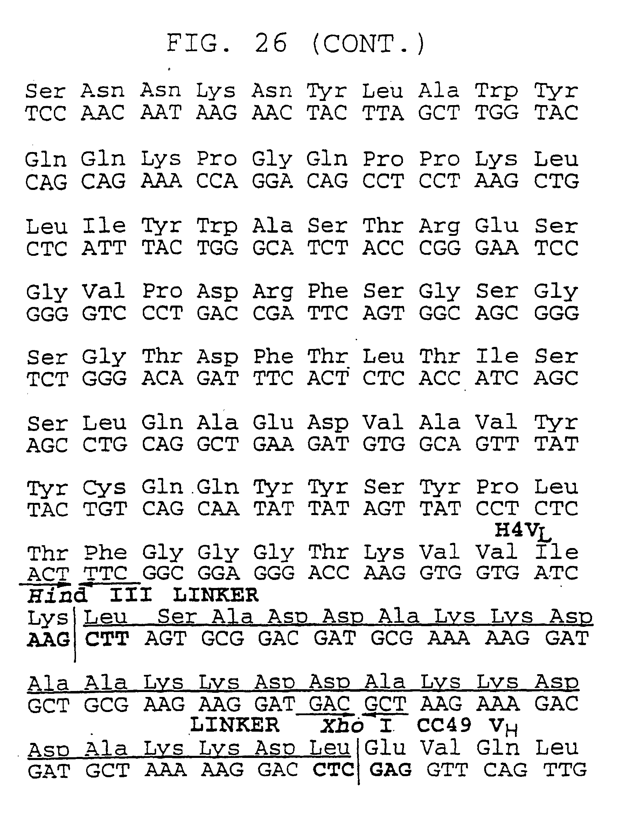

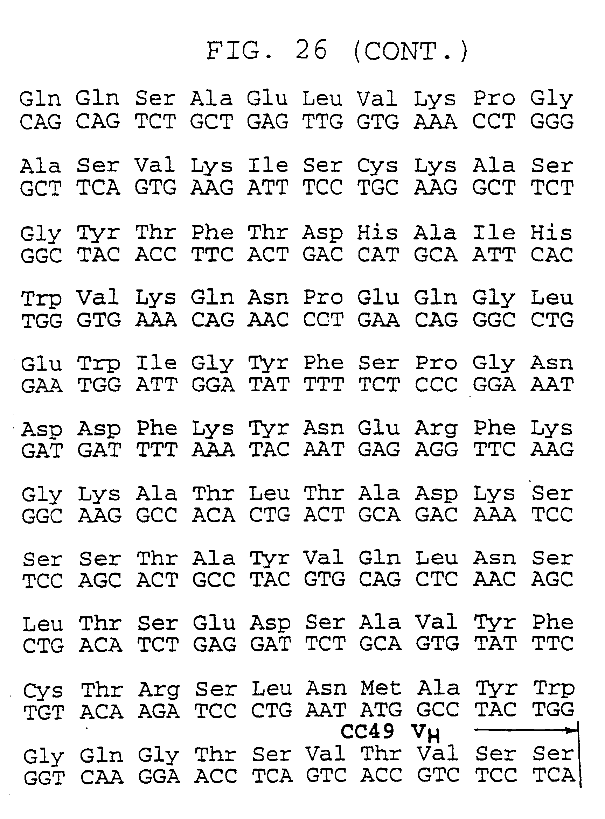

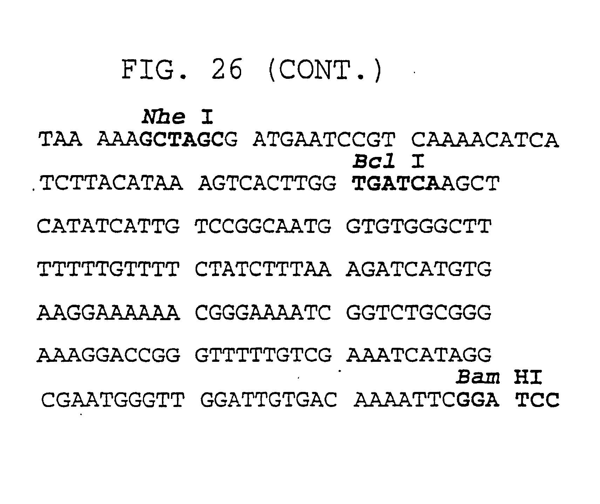

Figure 26 shows the DNA sequence and amino acid sequence of Hum4 V_{L}-CC49V_{H} SCFV present in pSCFVUHH.

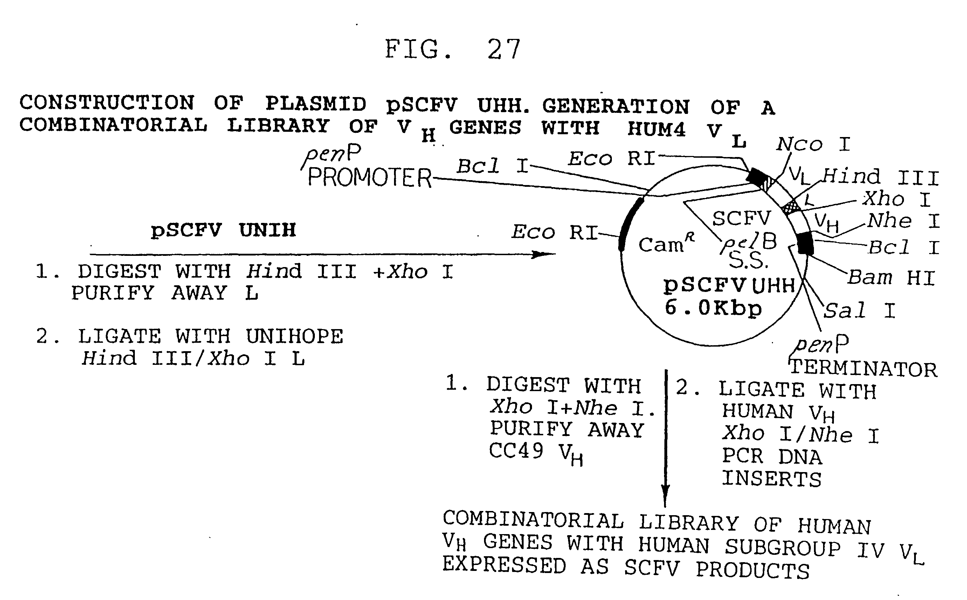

Figure 27 shows the construction plasmid pSCFV UHH and a schematic of a combinatorial library of V_{H} genes with Hum4 V_{L}.

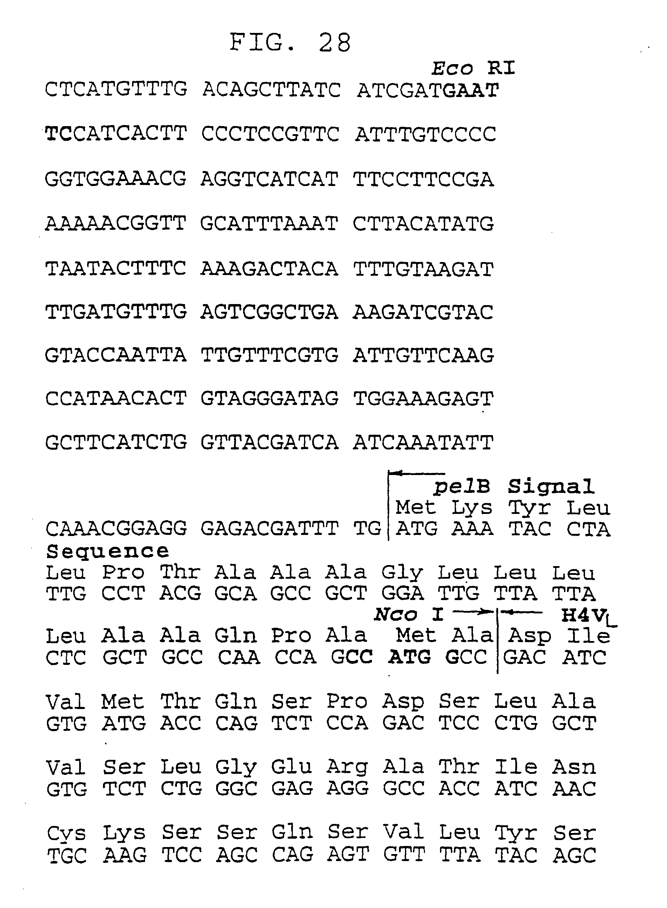

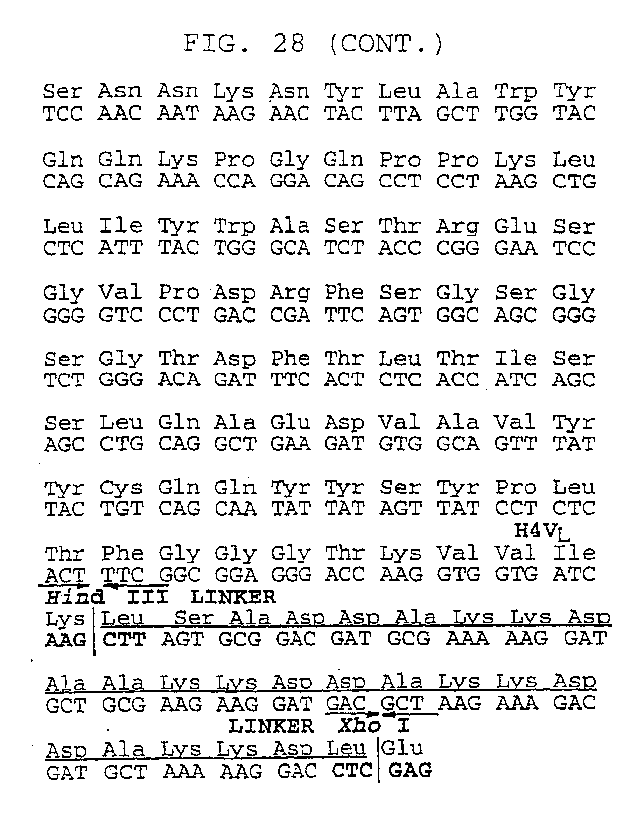

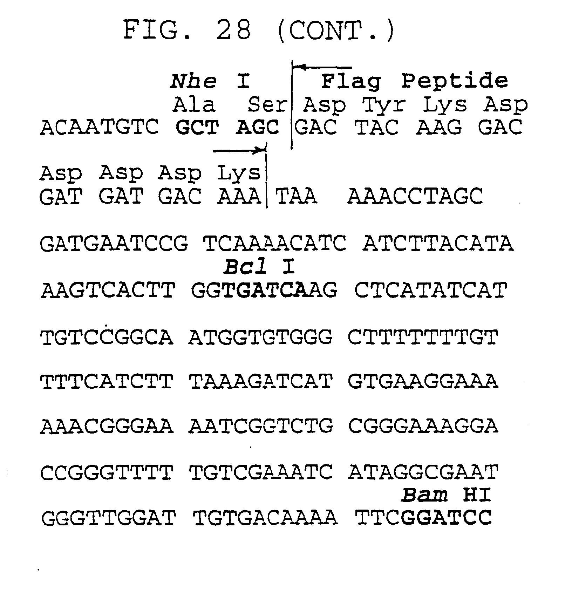

Figure 28 illustrates the nucleotide sequence of FLAG peptide adapter in pATDFLAG.

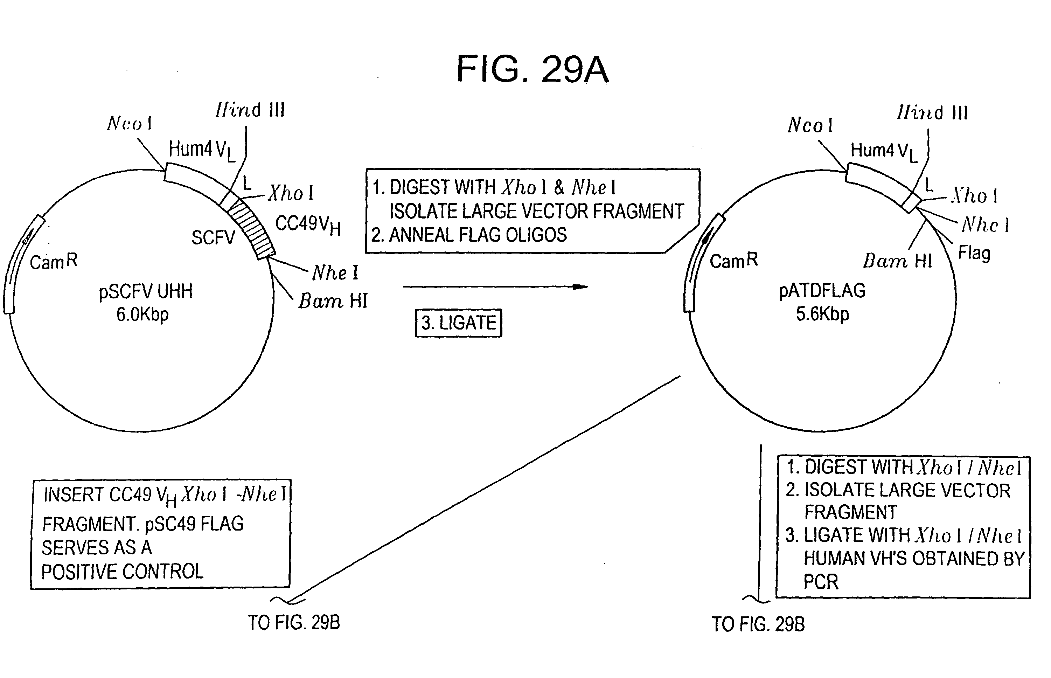

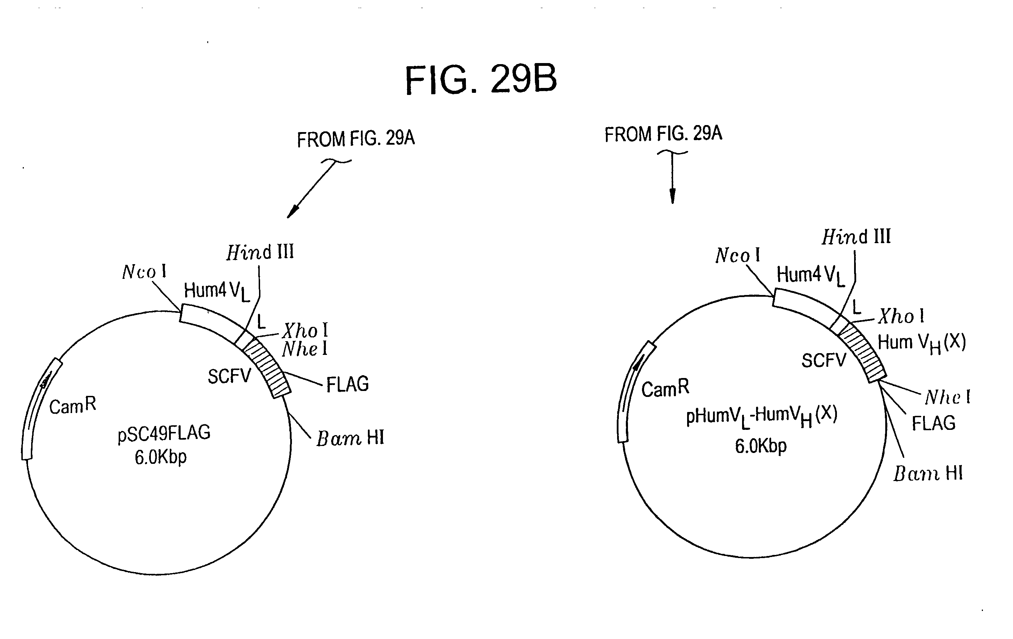

Figure 29 (A+B) illustrates the construction of pATDFLAG, pHumVL-HumVH (X) and pSC49FLAG.

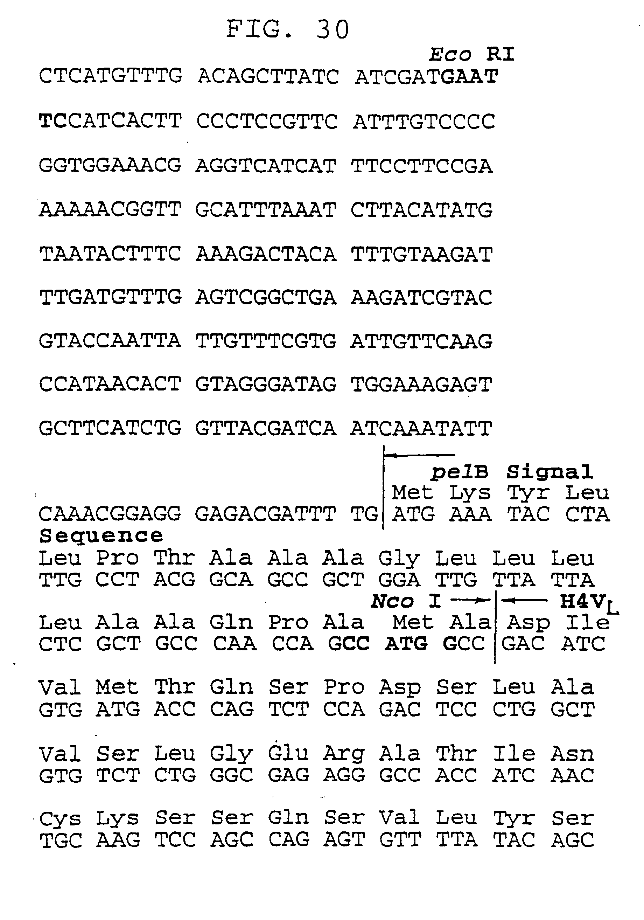

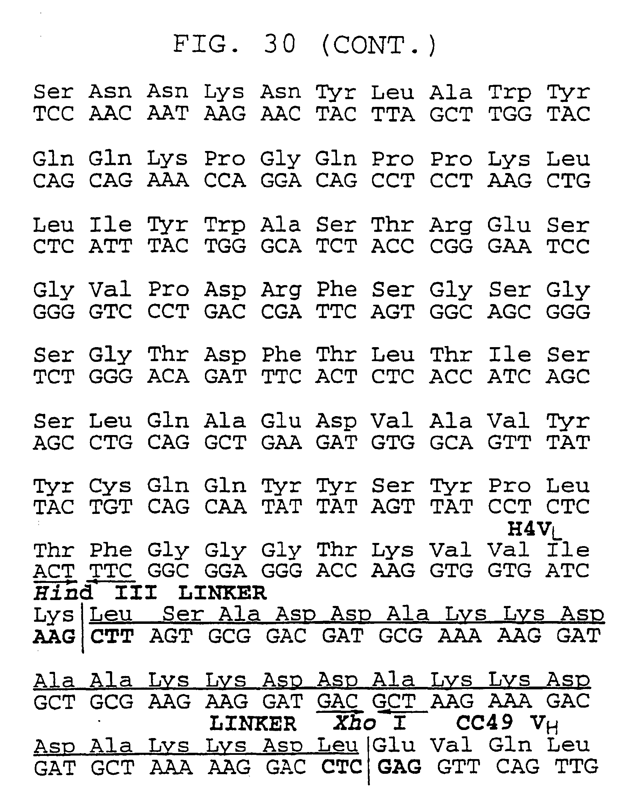

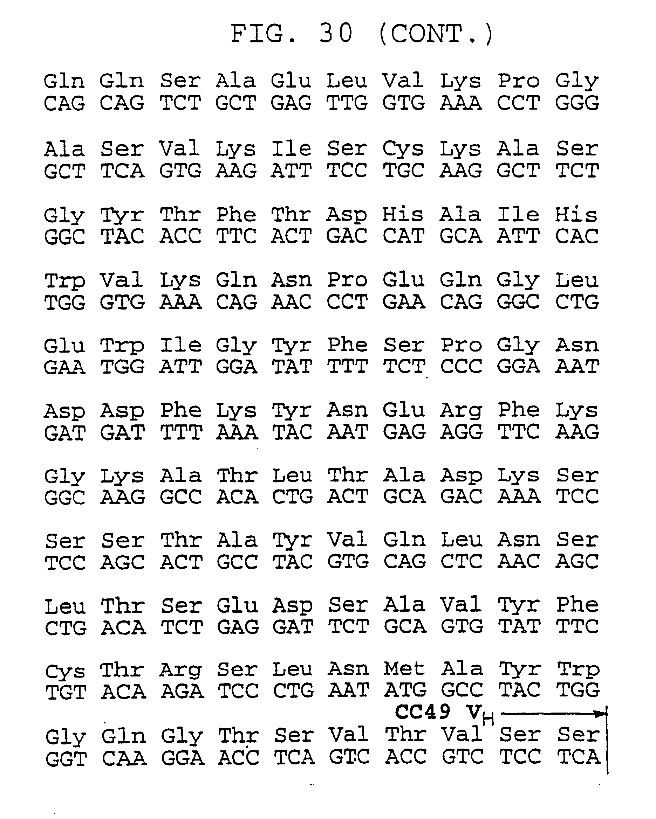

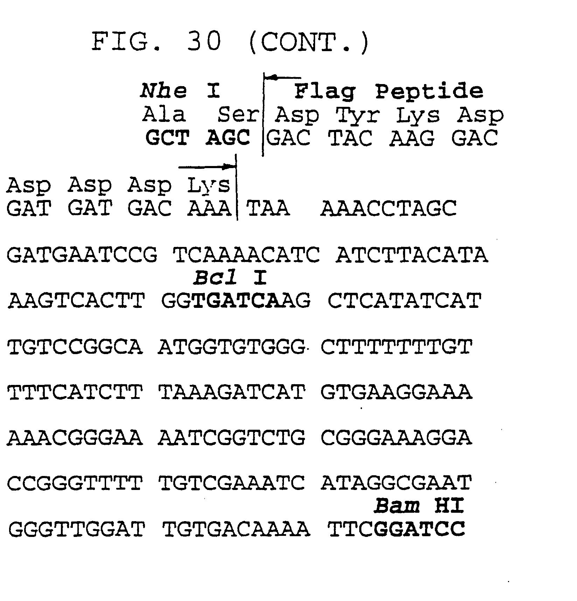

Figure 30 illustrates the nucleotide and amino acid sequences of pSC49FLAG.

### Detailed Description of the Invention

Nucleic acids, amino acids, peptides, protective groups, active groups and so on, when abbreviated, are abbreviated according to the IUPAC IUB (Commission on Biological Nomenclature) or the practice in the fields concerned.

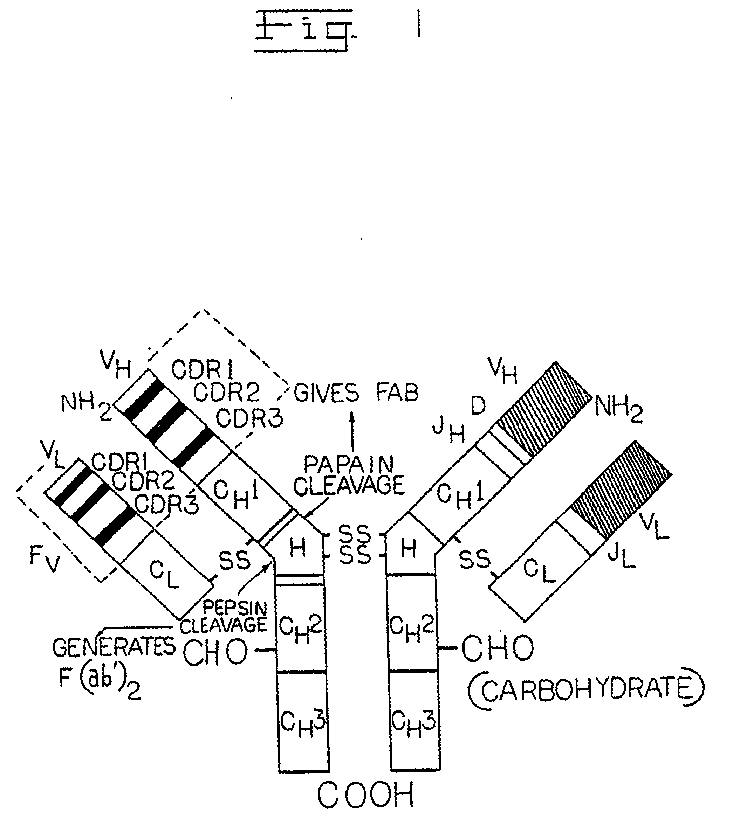

The basic immunoglobulin structural unit is set forth in Figure 1. The terms "constant" and "variable" are used functionally. The variable regions of both light (V_{L}) and heavy (V_{H}) chains determine binding recognition and specificity to the antigen. The constant region domains of light (C_{L}) and heavy (C_{H}) chains confer important biological properties such as antibody chain association, secretion, transplacental mobility, complement binding, binding to Fc receptors and the like.

The immunoglobulins of this invention have been developed to address the problems of the prior art. The methods of this invention produce, and the invention is directed to, composite antibodies. By "composite antibodies" is meant immunoglobulins comprising variable regions not hitherto found associated with each other in nature.

The composite Hum4 V_{L}, V_{H} antibodies described herein assume a conformation having an antigen binding site which binds specifically and with sufficient strength to TAG-72 to form a complex capable of being isolated by using standard assay techniques (e.g. enzyme-linked immunosorbent assay (ELISA), radio-immunoassay (RIA), or fluorescence-activated cell sorter analysis (FACS), immunohistochemistry and the like). Preferably, the composite HUM4 V_{L}, V_{H} antibodies of the present invention have an antigen binding affinity or avidity greater than 10⁵ M⁻¹, more preferably great than 10⁶ M⁻¹ and most preferably greater than 10⁸ M⁻¹. For a discussion of the techniques for generating and reviewing immunoglobulin binding affinities see Munson (1983), Methods Enzymol. 92:543-577 and Scatchard (1949), Ann. N.Y. Acad. Sci., 51:660-672.

Human antibody kappa chains have been classified into four sub-groups on the basis of invariant amino acid sequences (see, for example, Katab et al (1991), Sequences of Proteins of Immunological Interest (4th ed.), published by the U.S. Department of Health and Human Services). There appear to be approximately 80 human V_{K} genes, but only one Sub-group IV V_{K} gene has been identified in the human genome (see Klobeck, et al. (1985), Nucleic Acids Research, 13:6516-6528). The nucleotide sequence of Hum₄ is set forth in Katab et al (1991), *supra:* and Wang et al (1973), Nature, 243:126-127.

It has been found, quite surprisingly, that an immunoglobulin having a light chain containing the V_{L} encoded by a gene derived from Hum4 V_{L} may, if combined with suitable V_{H}, having binding specificity for TAG-72.

The type of J_{L} gene segment selected is not critical to the invention, in that it is expected that any J_{L}, if present, can associate with the Hum4 V_{L}. The present invention obviously contemplates that Hum4 V_{L} in association with a human J_{K} sequence. The five human J_{K} sequences are set forth in Heiter et al (1982). The Journal of Biological Chemistry, 357:1516-1522. However, the present invention is not intended to be limited to the human J_{K}. The present invention specifically contemplates the human Hum4 V_{L} in association with any of the least six human J_{K} genes (see Hollis et al (1982), Nature, 296:321-325).

An exemplary technique for engineering the Hum4 V_{L} with selected J_{L} segments includes synthesizing a primer having a so-called "wagging tail", that does not hybridise with the target DNA; thereafter, the sequences are amplified and spliced together by overlap extension (see Horton et al (1989), Gene. 77:61-68).

The C_{L} of the composite Hum4 V_{L}, V_{H} antibodies is not critical to the invention. To date, the Hum4 V_{L} has only been reported as having been naturally rearranged with the single C_{K} gene (see Heiter et al (1980), Cell. 22:197-207). However, the present invention is not intended to be limited to the C_{K} light chain constant domain. That is, the C_{L} gene segment may also be any of the at least six C_{K} genes (see Hollis et al., supra).

The DNA encoding the heavy chain variable region consists roughly of a heavy chain variable (V_{H}) gene sequence, a heavy chain diversity (D_{H}) gene sequence, and a heavy chain joining (J_{H}) gene sequence.

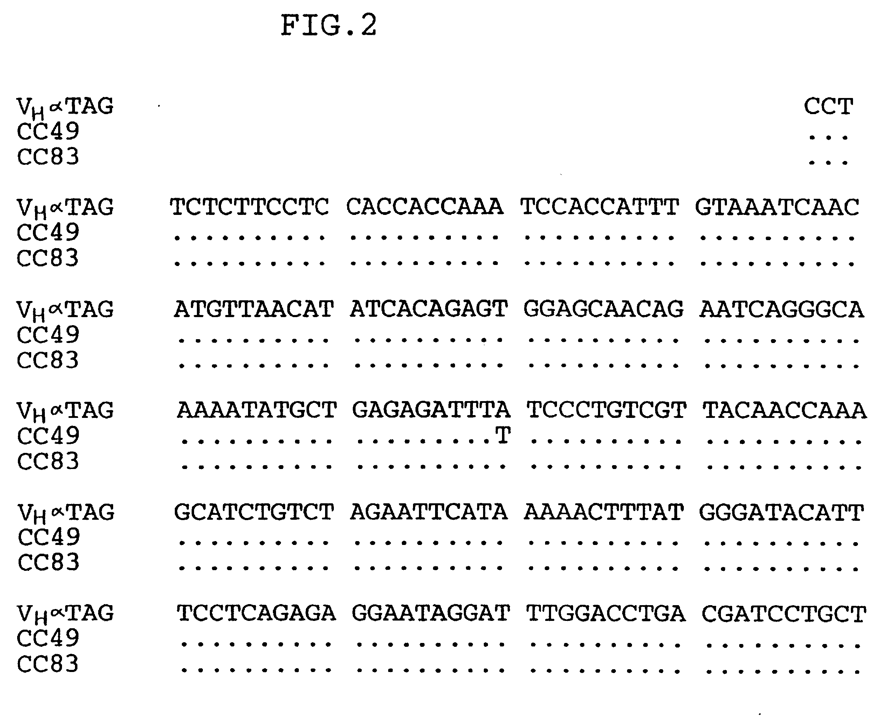

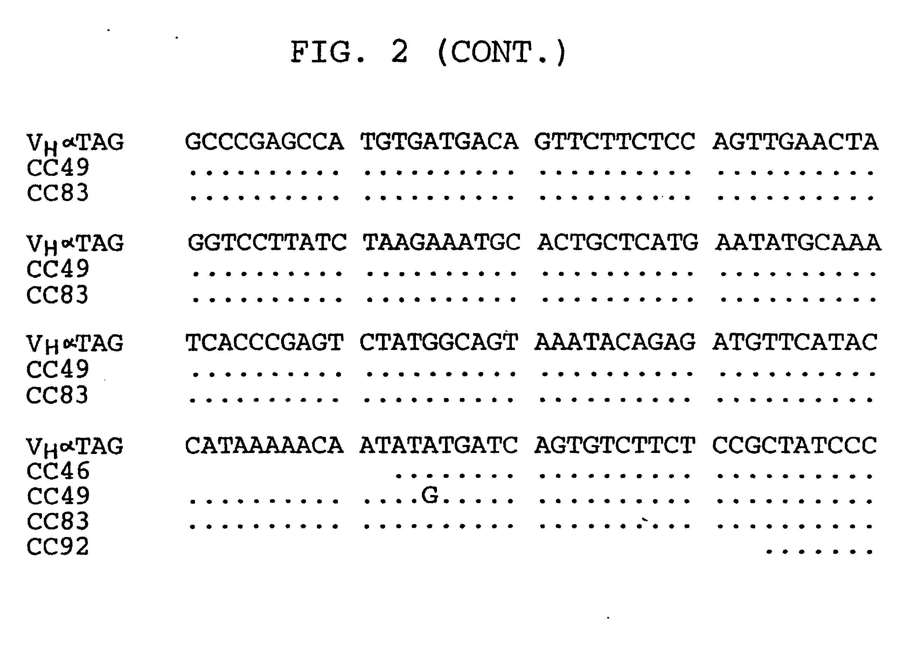

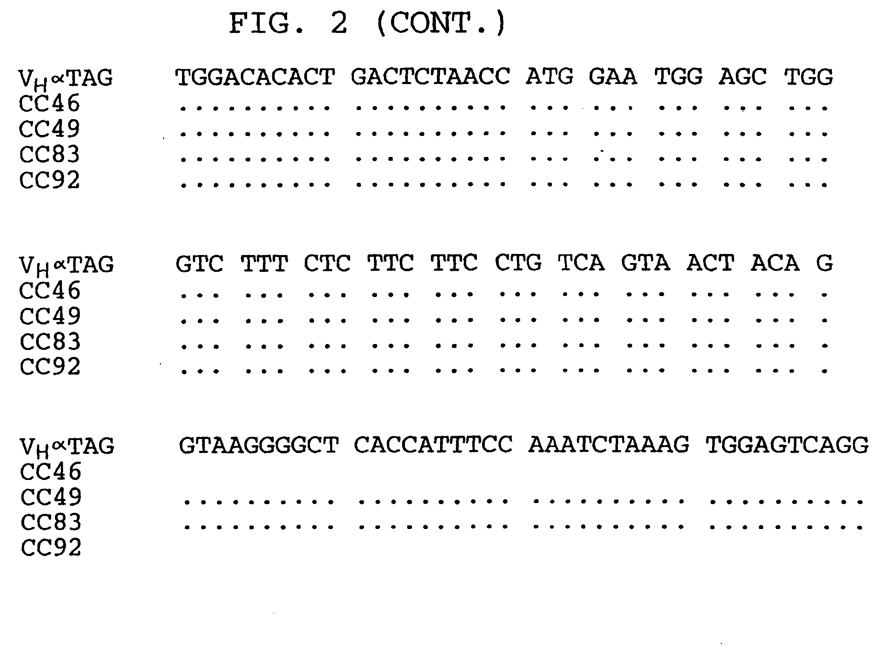

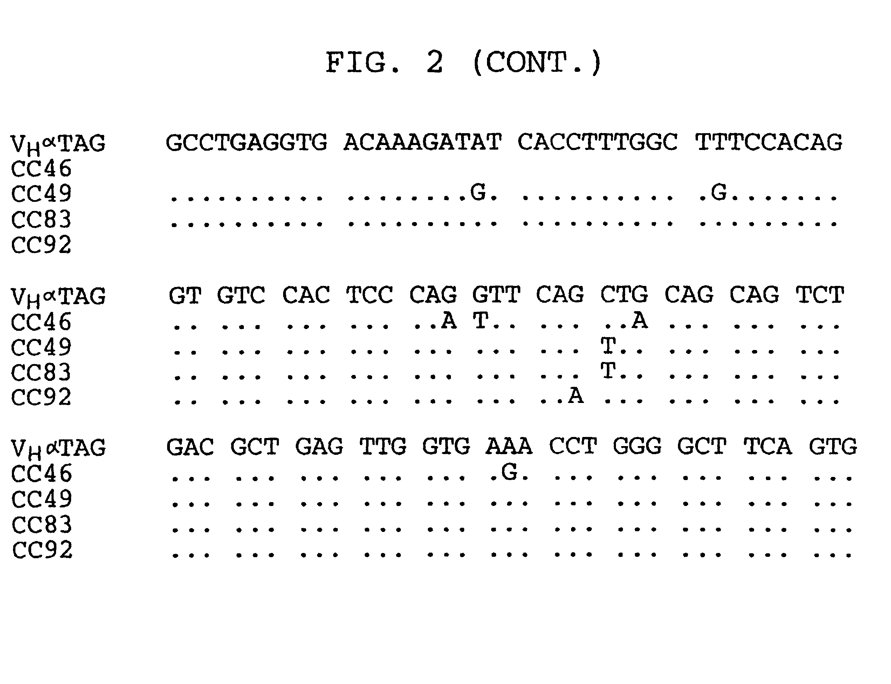

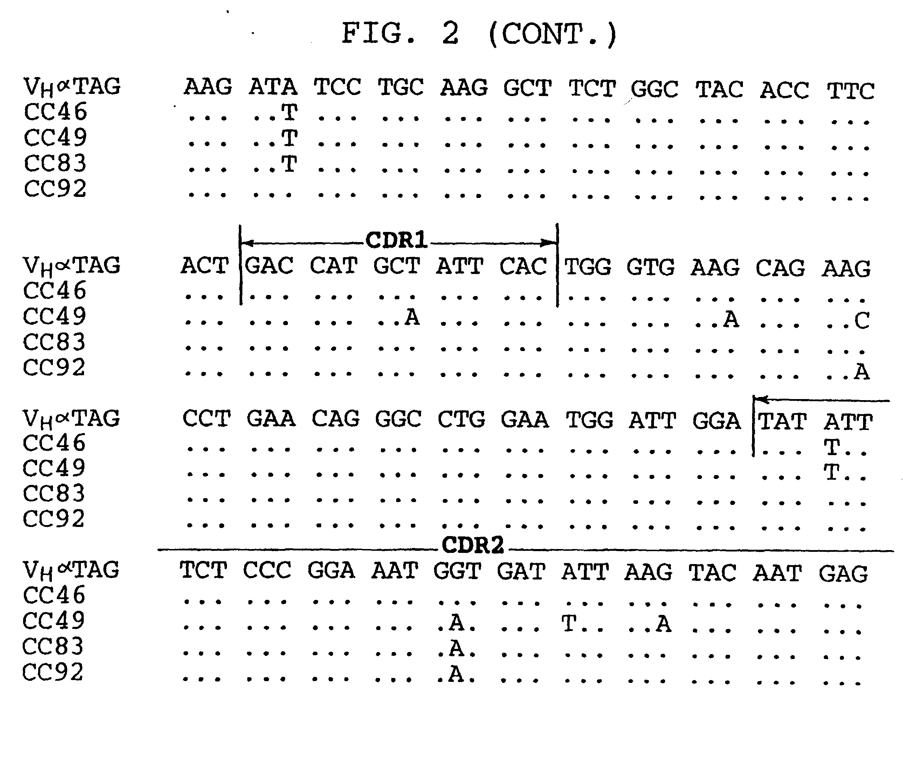

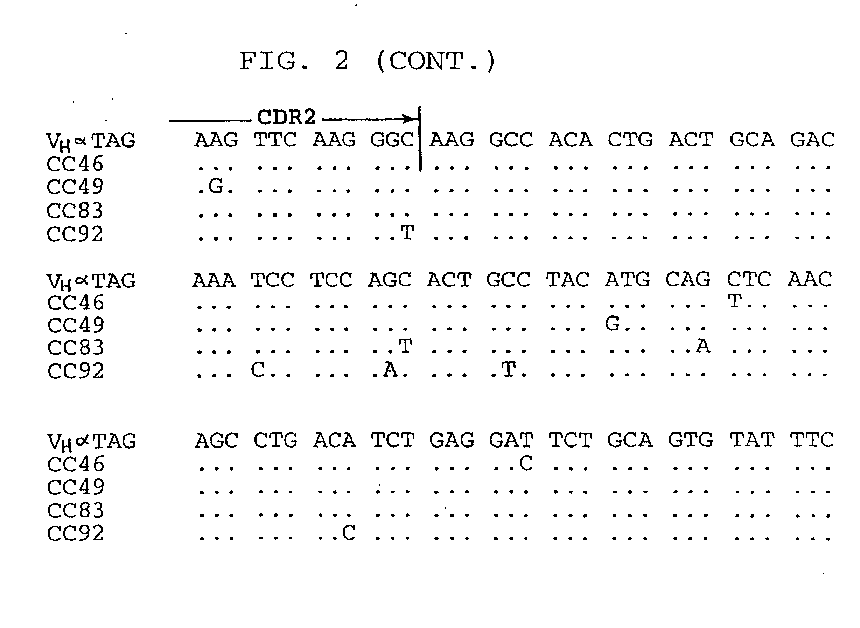

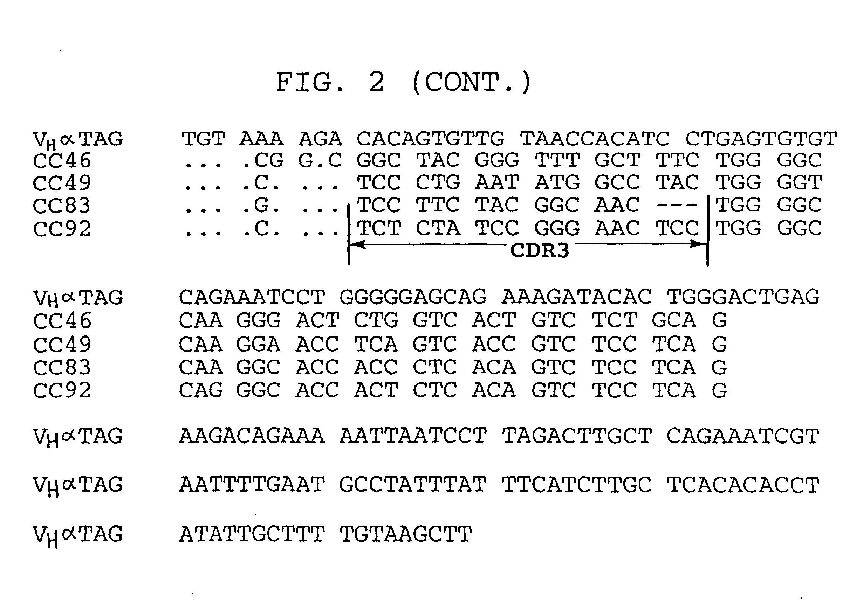

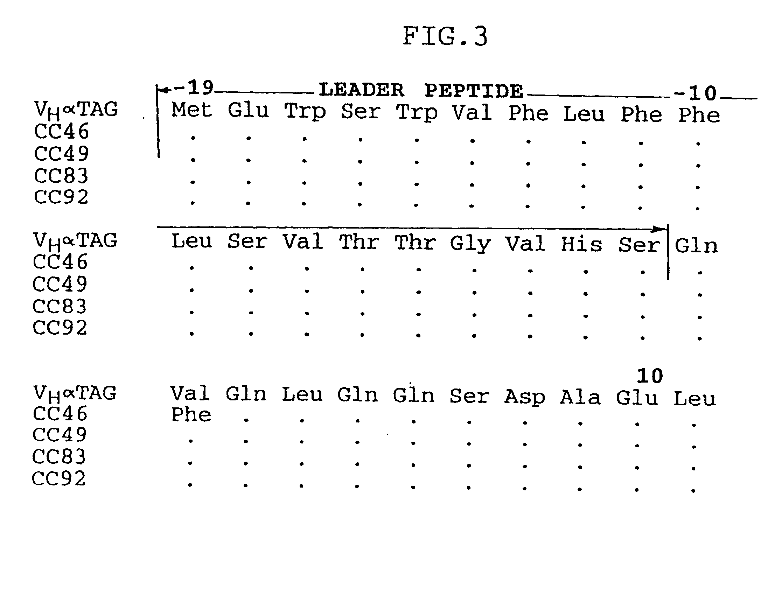

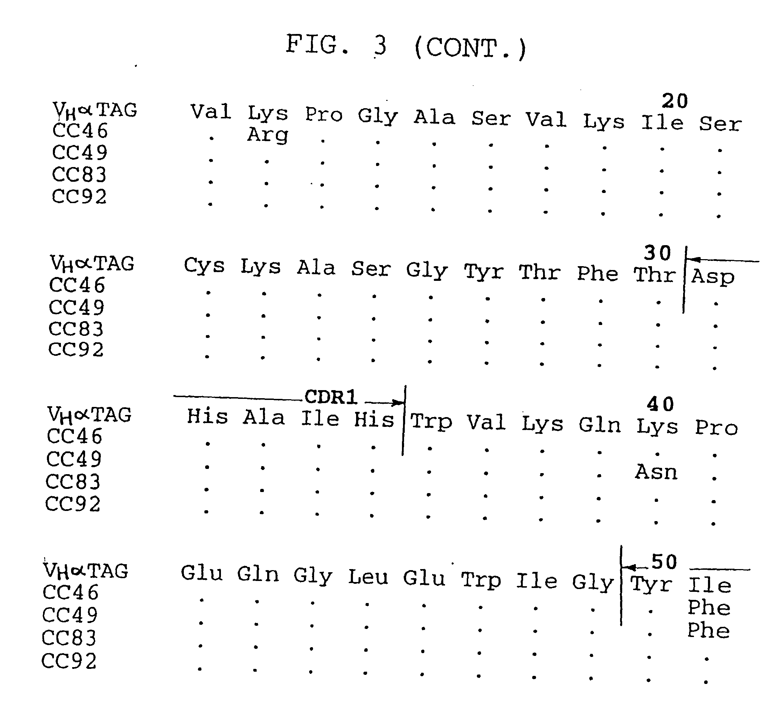

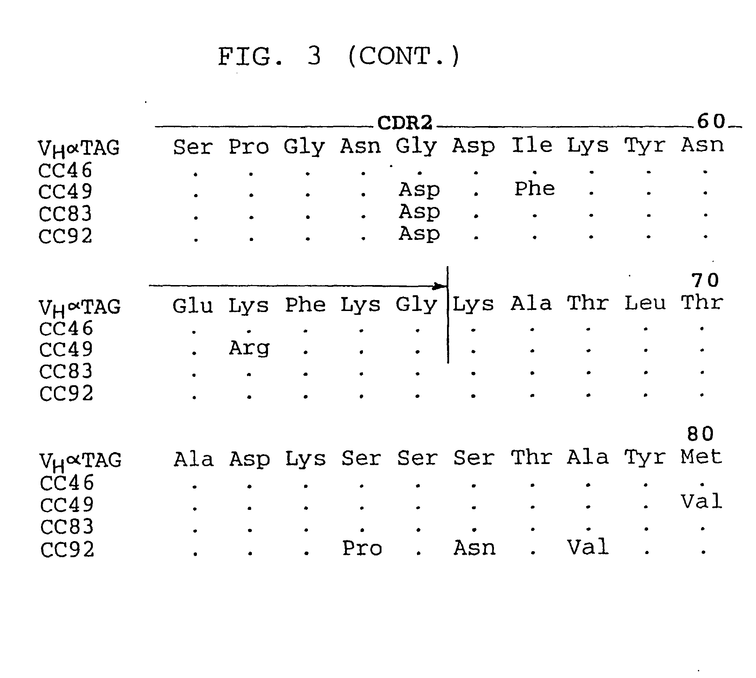

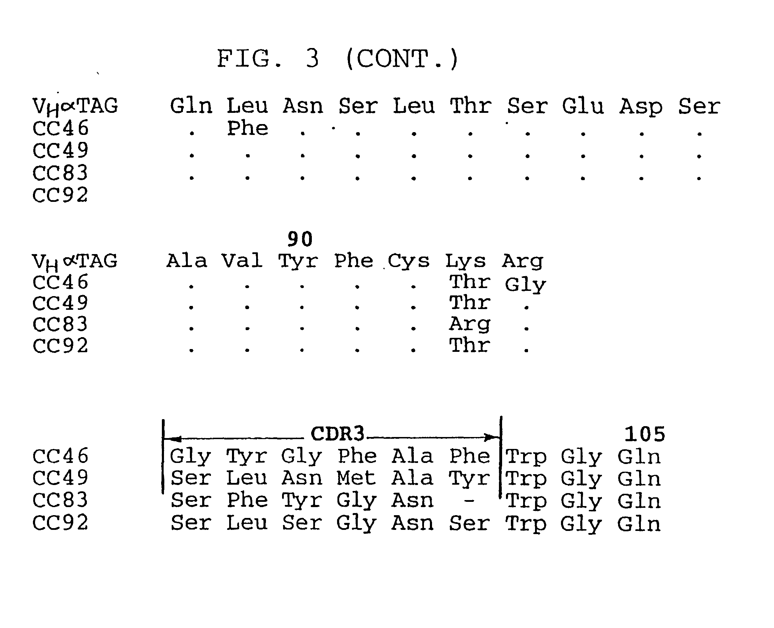

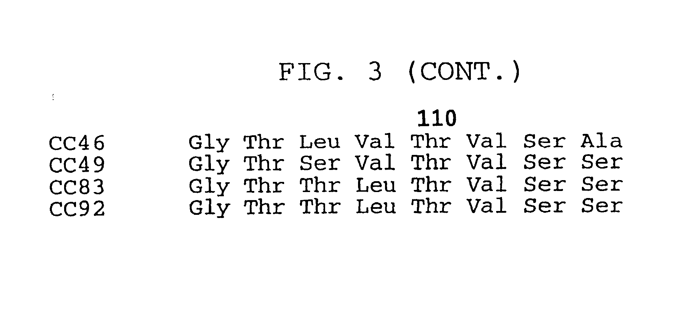

The choice of heavy chain diversity (D_{H}) segment and the heavy chain joining (J_{H}) segment of the composite Hum4 V_{L}, V_{H} antibody are not critical to the present invention. Obviously, human and murine D_{H} and J_{H} gene segments are contemplated, provided that a given combination does not significantly decrease binding to TAG-72. Specifically, when utilising CC46 V_{H}, CC49 V_{H}, CC83 V_{H} and CC92 V_{H}, the composite Hum4 V_{L}, V_{H} antibody will be designed to utilise the D_{H} and J_{H} segments which naturally associated with those V_{H} of the respective hybridomas (see Figures 2 and 3). Exemplary murine and human D_{H} and J_{H} sequences are set forth in Kabat et al (1991), supra. An exemplary technique for engineering such selected D_{H} and J_{H} segments with a V_{H} sequence of choice includes synthesizing selected oligonucleotides, annealing and ligating in a cloning procedure (see Horton et al., supra).

The V_{H}αTAG germline gene, is known in the art (see for example EP 0365 997 to Mezes et al., the Dow Chemical Company). Figure 2 shows the nucleotide sequence of V_{H}αTAG and nucleotide sequences encoding the V_{H} of the CC46, CC49, CC83 and CC92 antibodies, respectively. Figure 3 shows the corresponding amino acid sequences of V_{H}αTAG, CC46 V_{H}, CC49 V_{H}, CC83 V_{H} and CC92 V_{H}.

A comparison of the nucleotide and amino acid sequences of V_{H}αTAG, CC46 V_{H}, CC49 V_{H}, CC83 V_{H} and CC92 V_{H} shows that those CC antibodies are derived from V_{H}αTAG. Somatic mutations occurring during productive rearrangement of the V_{H} derived from V_{H}αTAG in a B cell gave rise to some nucleotide changes that may or may not result in a homologous amino acid change between the productively rearranged hybridomas (see EP 0 365 997).

The nucleotide sequences of V_{H}αTAG and Hum4 V_{L} germline genes have been provided herein. Other antibody genes may be productively rearranged from the V_{H}αTAG germline gene. Other antibodies encoded by DNA derived from V_{H}αTAG may be identified by using a hybridisation probe made from the DNA or RNA of the V_{H}αTAG or rearranged genes containing the recombined V_{H}αTAG. Specifically, the probe will include of all or part of the V_{H}αTAG germline gene and its flanking regions. By "flanking regions" is meant to include those DNA sequences from the 5' end of the V_{H}αTAG to the 3' end of the upstream gene, and from the 3' end of the V_{H}αTAG to the 5' end of the downstream gene.

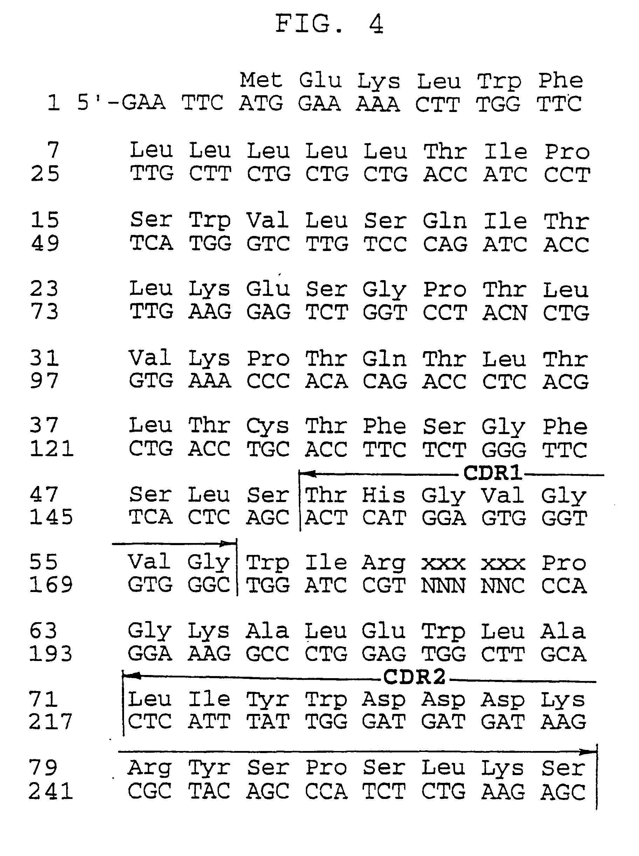

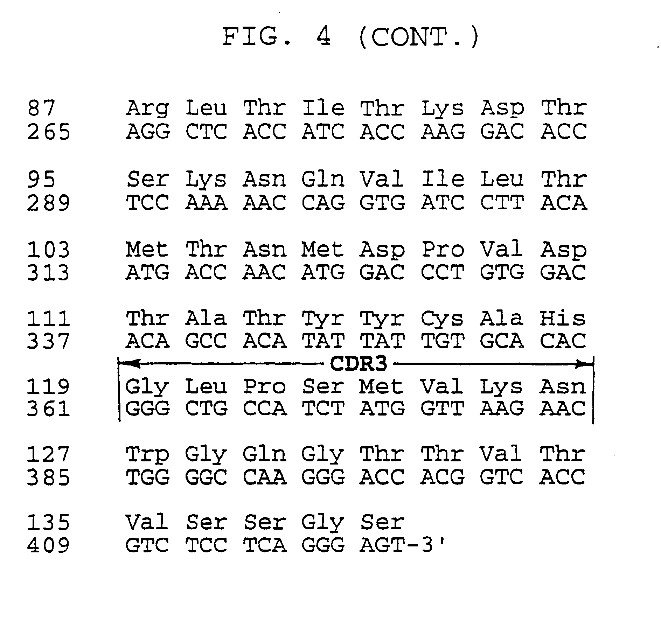

The CDR from the variable region of antibodies derived from V_{H}αTAG may be grafted on to the FR of selected V_{H}, i.e. from a human antibody (see EP 0 239 400 of Winter). For example, the cell line. B17X2, expresses an antibody utilising a variable light chain encoded by a gene derived from Hum4 V_{L} and a variable heavy chain which makes a stable V_{L} and V_{H} combination (see Marsh et al (1985), Nucleic Acid Research 13:6531-5644: and Polke et al (1982), Immunobiol. 163:95-109). The nucleotide sequence of the V_{H} chain for B17X2 is shown in Figure 4. The B17X2 cell line is publicly available from Dr Christine Polke, Universitats-Kinderklinik, Josef-Schneider-Str. 2. 8700 Wurzburg, FRG). B17X2 is directed to N-Acetyl-D-Glucosamine and is not specific for TAG-72.

However, consensus sequences of antibody derived from the CDR1 of V_{H}αTAG (amino acid residues 31 to 35 of Figure 3) may be inserted into B17X2 (amino acid residues 31 to 37 of Figure 4) and the CDR2 of V_{H}αTAG (amino residues 50 to 65 of Figure 3) may be inserted into B17X2 (amino acid residues 52 to 67 of Figure 4). The CDR3 may be replaced by any DH and JH sequence which does not affect the binding of the antibody for TAG-72 but, specifically, may be replaced by the CDR3 of an antibody having its V_{H} derived from V_{H}αTAG, e.g., CC46, CC49, CC83 and CC92. Exemplary techniques for such replacement are set forth in Horton *et al., supra*.

The C_{H} domains of immunoglobulin heavy chain derived from V_{H}αTAG genes, for example may be changed to a human sequence by known techniques (see, USP 4,816,567 to Cabilly, Genentech). C_{H} domains may be of various complete or shortened human isotypes, i.e., IgG (e.g., IgG₁, IgG₂, IgG₃, and IgG₄), IgA (e.g., IgA1 and IgA2), IgD, IgE, IgM, as well as the various allotypes of the individual groups (see Kabat *et al*. (1991), *supra*).

Given the teachings of the present invention, it should be apparent to the skilled artisan that human V_{H} genes can be tested for their ability to produce an anti-TAG-72 immunoglobilin combination with the Hum4 V_{L} gene. The V_{L} may be used to isolate a gene encoding for a V_{H} having the ability to bind to TAG-72 to test myriad combinations of Hum4 V_{L} and V_{H} that may not naturally occur in nature, e.g., by generating a combinatorial library using the Hum4 V_{L} gene to select a suitable V_{H}. Examples of these enabling technologies include screening of combinatorial libraries of V_{L}-V_{H} combinations using an Fab or single chain antibody (SCFV) format expressed on the surfaces of fd phage (Clackson, *et al*. (1991), Nature, **352**:624-628), or using a λ phage system for expression of Fv's or Fabs (Huse, *et al*. (1989), Science, **246**:1275-1281). However, according to the teachings set forth herein, it is now possible to clone SCFV antibodies in *E.coli*, and express the SCFVs as secreted soluble proteins. SCFV proteins produced in *E. coli* that contain a Hum4 V_{L} gene can be screened for binding to TAG-72 using, for example, a two-membrane filter screening system (Skerra, *et al*. (1991), Analytical Biochemistry, **196**:151-155).

The desired gene repertoire can be isolated from human genetic material obtained from any suitable source, e.g., peripheral blood lymphocytes, spleen cells and lymph nodes of a patient with tumor expressing TAG-72. In some cases, it is desirable to bias the repertoire for a preselected activity, such as by using as a source of nucleic acid, cells (source cells) from vertebrates in any one of various stages of age, health and immune response.

Cells coding for the desired sequence may be isolated, and genomic DNA fragmented by one or more restriction enzymes. Tissue (e.g., primary and secondary lymph organs, neoplastic tissue, white blood cells from peripheral blood and hybridomas) from an animal exposed to TAG-72 may be probed for selected antibody producing B cells. Variability among 3 cells derived from a common germline gene may result from somatic mutations occurring during productive rearrangement.

Generally, a probe made from the genomic DNA of a germline gene or rearranged gene can be used by those skilled in the art to find homologous sequences from unknown cells. For example, sequence information obtained from Hum4 V_{L} and V_{H}αTAG may be used to generate hybridisation probes for naturally-occurring rearranged V regions, including the 5' and 3' nontranslated flanking regions. The genomic DNA may include naturally-occurring introns for portions thereof, provided that functional splice donor and splice acceptor regions had been present in the case of mammalian cell sources.

Additionally, the DNA may also be obtained from a cDNA library. mRNA coding for heavy or light chain variable domain may be isolated from a suitable source, either mature B cells or a hybridoma culture, employing standard techniques of RNA isolation. The DNA or amino acids also may be synthetically synthesized and constructed by standard techniques of annealing and ligating fragments (see Jones et al (1986), Nature. 321:522-525; Reichmann et al., (1988), Nature, 332:323-327; Sambrook et al (1989), supra and Merrifield et al (1963), J. Amer. Chem. Soc., 85:2149-2154). Heavy and light chains may be combined in vitro to gain antibody activity (see Edelman, et al (1963), Proc. Natl. Acad, Sci. USA, 50:753).

One may also use a gene library of V_{H}αTAG homologs, preferably human homologs of V_{H}αTAG. By "homolog" is meant a gene coding for a V_{H} region homologous to the V_{H}αTAG germline gene and capable of combining with the light chain variable region encoded by the human Sub-group IV germline gene to form a three dimensional structure having the ability to bind to TAG-72.

Preferably, the gene library is produced by a primer extension reaction or combination of primer extension reactions as described herein. The V_{H}αTAG homologs are preferably in an isolated form, that is, substantially free of materials such as for example, primer extension reaction agents and/or substrates, genomic DNA segments, and the like. Thus one may clone the V_{H}αTAG-coding DNA homologs from a repertoire comprised of polynucleotide coding strands, such as genomic material containing the gene expressing the variable region or the messenger RNA (mRNA) which represents a transcript of the variable region. Nucleic acids constituting V_{H}αTAG-coding homologs can be derived from cells producing IgA, IgD, IgE, IgG or IgM, most preferably from IgM and IgG, producing cells.

The V_{H}αTAG-coding DNA homologs may be produced by primer extension. The term "primer" as used herein refers to a polynucleotide whether purified from a nucleic acid restriction digest or produced synthetically, which is capable of acting as a point of initiation of synthesis when placed under conditions in which synthesis of a primer extension product which is complementary to a nucleic acid strand is induced, i.e. in the presence of nucleotides and an agent for polymerisation such as DNA polymerase, reverse transcriptase and the like, and at a suitable temperature and pH.

Preferably, the V_{H}αTAG-coding DNA homologs may be produced by polymerase chain reaction (PCR) amplification of double stranded genomic or cDNA, wherein two primers are used for each coding strand of nucleic acid to be exponentially amplified. The first primer becomes part of the nonsense (minus or complementary) strand and hybridises to a nucleotide sequence conserved among V_{H} (plus) strands within the repertoire. PCR is described in Mullis et al (1987), Meth. Enz., 155:335-350: and PCR Technology, Erlich (ed.) (1989). PCR amplification of the mRNA from antibody-producing cells is set forth in Orlandi et al (1989), Proc. Natl. Acad. Sci. USA, 86:3387-3837.

The expression products of the V_{H}αTAG-coding DNA homologs may be connected via a linkere to form a SCFV having a three dimensional structure capable of binding TAG-72. The SCFV construct can be in a V_{L}-L-V_{H} or V_{H}-L-V_{L} configuration. For a discussion of SCFV see Bird et al (1988), Science, 242:423-426, The design of suitable peptide linker regions is described in U.S. Patent No. 4,704,692 to Ladner et al Genex.

The nucleotide sequence of a primer is selected to hybridise with a plurality of immunoglobulin heavy chain genes at a site substantially adjacent to the V_{H}αTAG-coding DNA homolog so that a nucleotide sequence coding for a functional (capable of binding) polypeptide is obtained. The choice of a primer's nucleotide sequence depends on factors such as the distance on the nucleic acid from the region coding for the desired receptor, its hybridisation site on the nucleic acid relative to any second primer to be used, the number of genes in the repertoire it is to hybridise to, and the like. To hybridise to a plurality of different nucleic acid strands of V_{H}αTAG-coding DNA homolog, the primer must be a substantial complement of a nucleotide sequence conserved among the different strands.

The peptide linker may be coded for by the nucleic acid sequences that are part of the polynucleotide primers used to prepare the various gene libraries. The nucleic acid sequence coding for the peptide linker can be made up of nucleic acids attached to one of the primers or the nucleic acid sequence coding for the peptide linker may be derived from nucleic acid sequences that are attached to several polynucleotide primers used to create the gene libraries. Additionally, noncomplementary bases or longer sequences can be interspersed into the primer, provided the primer sequence has sufficient complementarily with the sequence of the strand to be synthesized or amplified to non-randomly hybridize therewith and thereby form an extension product under polynucleotide synthesizing conditions (see Horton *et al*. (1989), Gene, **77**:61-68).

Exemplary human V_{H} sequences from which complementary primers may be synthesized are set forth in Kabat *et al*. (1991), *supra:* Humphries *et al*. (1988), Nature, **331**:446-449; Schroeder *et al*. (1990), Proc. Natl. Acad. Sci. USA, **87**:6146-6150; Berman *et al*. (1988), EMBO Journal, **7**:727-738; Lee *et al*. (1987), J. Mol. Biol., **195**:761-768); Marks *et al*. (1991), Eur. J. Immunol., **21**:985-991; Willems. *et al*. (1991), J. Immunol., **146**:3646-3651; and Person *et al*. (1991), Proc Natl. Acad. Sci. USA, **88**:2432-2436. To produce V_{H} coding DNA homologs, first primers are therefore chosen to hybridize to (i.e. be complementary to) conserved regions within the J region, CH1 region, hinge region, CH2 region, or CH3 region of immunoglobulin genes and the like. Second primers are therefore chosen to hydribidize with a conserved nucleotide sequence at the 5' end of the V_{H}αTAG-coding DNA homolog such as in that area coding for the leader or first framework region.

Alternatively, the nucleic acid sequences coding for the peptide linker may be designed as pare of a suitable vector. As used herein, the term "expression vector" refers to a nucleic acid molecule capable of directing the expression of genes to which they are operatively linked. The choice of vector to which a V_{H}αTAG-coding DNA homologs is operatively linked depends directly, as is well known in the art, on the functional properties desired, e.g., replication or protein expression, and the host cell (either procaryotic or eucaryotic) to be transformed, these being limitations inherent in the art of constructing recombinant DNA molecules. In preferred embodiments, the eucaryotic cell expression vectors used include a selection marker that is effective in an eucaryotic cell, preferably a drug resistant selection marker.

Expression vectors compatible with procaryotic cells are well known in the art and are available from several commercial sources. Typical of vector plasmids suitable for procaryotic cells are pUC8, pUC9, pBR322, and pBR329 available from BioRad Laboratories, (Richmond, CA), and pPL and pKK223 available from Pharmacia, (Piscataway, NJ).

Expression vectors compatible with eucaryotic cells, preferably those compatible with vertebrate cells, can also be used. Eucaryotic cell expression vectors are well known in the art and are available from several commercial sources. Typically, such vectors are provided containing convenient restriction sites for insertion of the desired DNA homologue. Typical of vector plasmids suitable for eucaryotic cells are pSV2neo and pSV2gpt (ATCC), pSVL and pKSV-10 (Pharmacia), pBPV-1/PML2d (International Biotechnologies, Inc.), and pTDT1 (ATCC).

The use of viral expression vectors to express the genes of the V_{H}αTAG-coding DNA homologs is also contemplated. As used herein, the term "viral expression vector" refers to a DNA molecule that includes a promoter sequences derived from the long terminal repeat (LTR) region of a viral genome. Exemplary phage include λ phage and fd phage (see, Sambrook, *et al*. (1989), Molecular Cloning: A Laboratory Manual. (2nd ed.), and McCafferty *et al*. (1990), Nature, **6301**:552-554.

The population of V_{H}αTAG-coding DNA homologs and vectors are then cleaved with an endonuclease at shared restriction sites. A variety of methods have been developed to operatively link DNA to vectors via complementary cohesive termini. For instance, complementary cohesive termini can be engineered into the V_{H}αTAG-coding DNA homologs during the primer extension reaction by use of an appropriately designed polynucleotide synthesis primer, as previously discussed. The complementary cohesive termini of the vector and the DNA homolog are then operatively linked (ligated) to produce a unitary double stranded DNA molecule.

The restriction fragments of Hum4 V_{L}-coding DNA and the V_{H}αTAG-coding DNA homologs population are randomly ligated to the cleaved vector. A diverse, random population is produced with each veccor having a V_{H}αTAG-coding DNA homolog and Hum4 V_{L}-coding DNA located in the same reading frame and under the control of the vector's promoter.

The resulting single chain construct is then introduced into an appropriate host to provide amplification and/or expression of a composite Hum4 V_{L}, V_{H}αTAG homolog single chain antibody. Transformation of appropriate cell hosts with a recombinant DNA molecule of the present invention is accomplished by methods that typically depend on the type of vector used. With regard to transformation of procaryotic host cells, see, for example, Cohen *et al.* (1972), Proceedings National Academy of Science, USA, 69:2110; and Sambrook, *et al.* (1989), *supra.* With regard to the transformation of vertebrate cells with retroviral vectors containing rDNAs, see for example. Sorge *et al.* (1984), Mol. Cell. Biol., 4:1730-1737; Graham *et al.* (1973), Virol., **52**:456; and Wigler *et al.* (1979), Proceedings National Academy of Sciences, USA,76:1373-1376.

Exemplary prokaryotic strains that may be used as hosts include *E.coli, Bacilli*, and other entero-bacteriaceae such as *Salmonella typhimurium*, and various *Pseudomonas.* Common eukaryotic microbes include *S*. *cerevisiae* and *Pichia pastoris.* Common higher eukaryotic host cells include Sp2/0, VERO and HeLa cells, Chinese hamster ovary (CHO) cell lines, and W138, BHK, COS-7 and MDCK cell lines. Furthermore, it is now also evident that any cell line producing Hum4 V_{L}, e.g., the B17X2 human cell line, can be used as a recipient human cell line for introduction of a V_{H} gene complementary to the Hum4 V_{L} which allows binding to TAG-72. For example, the B17X2 heavy chain may be genetically modified to not produce the endogenous heavy chain by well known methods; in this way, glycosylation patterns of the antibody produced would be human and not non-human derived.

Successfully transformed cells, i.e., cells containing a gene encoding a composite Hum4 V_{L}, V_{H}αTAG homolog single chain antibody operatively linked to a vector, can be identified by any suitable well known technique for detecting the binding of a receptor to a ligand. Preferred screening assays are those where the binding of the composite Hum4 V_{L}, V_{H}αTAG homolog single chain antibody to TAG-72 produces a detectable signal, either directly or indirectly. Screening for productive Hum4 V_{L} and V_{H}αTAG homolog combinations, or in other words, testing for effective antigen binding sites to TAG-72 is possible by using for example, a radiolabeled or biotinylated screening agent, e.g., antigens, antibodies (e.g., B72.3, CC49, CC83, CC46, CC92, CC30, CC11 and CC15) or anti-idiotypic antibodies (see Huse *et al*., *supra,* and Sambrook *et al*., *supra*); or the use of marker peptides to the NH₂- or COOH-terminus of the SCFV construct (see Hopp *et al.* (1988), Biotechnology, 6:1204-1210).

0f course, the Hum4 V_{L}-coding DNA and the V_{H}αTAG-coding DNA homologs may be expressed as individual polypeptide chains (e.g., Fv) or with whole or fragmented constant regions (e.g., Fab, and F(ab')₂). Accordingly, the Hum4 V_{L}-coding DNA and the V_{H}αTAG-coding DNA homologs may be individually inserted into a vector containing a C_{L} or C_{H} or fragment thereof, respectively. For a teaching of how to prepare suitable vectors see EPO 0 365 997 to Mezes *et al*., The Dow Chemical Company.

DNA sequences encoding the light chain and heavy chain of the composite Hum4 V_{L}, V_{H} antibody may be inserted into separate expression vehicles, or into the same expression vehicle. When coexpressed within the same organism, either on the same or the different vectors, a functionally active Fv is produced. When the V_{H}αTAG-coding DNA homolog and Hum4 V_{L} polypeptides are expressed in different organisms, the respective polypeptides are isolated and then combined in an appropriate medium to form a Fv. See Greene *et al*., Methods in Molecular Biology, Vol. 9, Wickner *et al*. (ed.); and Sambrook *et al*., *supra).*

Subsequent recombinations can be effected through cleavage and removal of the Hum4 V_{L}-coding DNA sequence to use the V_{H}αTAG-coding DNA homologs to produce Hum4 V_{L}-coding DNA homologs. To produce a Hum4 V_{L}-coding DNA homolog, first primers are chosen to hybridize with (i.e. be complementary to) a conserved region within the J region or constant region of immunoglobulin light chain genes and the like. Second primers become part of the coding (plus) strand and hybridize to a nucleotide sequence conserved among minus strands. Hum4 V_{L}-coding DNA homologs are ligated into the vector containing the V_{H}αTAG-coding DNA homolog, thereby creating a second population of expression vectors. The present invention thus is directed to cloning the Hum4 V_{L}-coding DNA homologs from a repertoire comprised of polynucleotide coding strands, such as genomic material containing the gene expressing the variable region or the messenger RNA (mRNA) which represents a transcript of the variable region. It is thus possible to use an iterative process to define yet further, composite antibodies, using later generation V_{H}αTAG-coding DNA homologs and Hum4 V_{L}-coding DNA homologs.

The present invention further contemplates genetically modifying the antibody variable and constant regions to include effectively homologous variable region and constant region amino acid sequences. Generally, changes in the variable region will be made in order to improve or otherwise modify antigen binding properties of the receptor. Changes in the constant region of the antigen receptor will, in general, be made in order to improve or otherwise modify biological properties, such as complement fixation, interaction with membranes, and other effector functions.

"Effectively homologous" refers to the concept that differences in the primary structure of the variable region may not alter the binding characteristics of the antigen receptor. A DNA sequence is effectively homologous to a second DNA sequence if at least 90 percent of the coding sequences are homologous. Such changes are permissible in effectively homologous amino acid sequences so long as the resultant antigen receptor region retains its desired property.

If there is only conservative difference between homologous positions of sequences, they may be regarded as equivalents under certain circumstances. General categories of potentially equivalent amino acids are set forth below, wherein, amino acids within a group may be substituted for other amino acids in that group:
(1) glutamic acid and aspartic acid; (2) hydrophobic amino acids such as alanine, valine, leucine and isoleucine; (3) asparagine and glutamine; (4) lysine, arginine; and (5) threonine and serine.

Exemplary techniques for nucleotide replacement include the addition, deletion, or substitution of various nucleotides, deletion or substitution of various nucleotides, provided that the proper reading frame is maintained. Exemplary techniques include using polynucleotide-mediated, site-directed mutagenesis, i.e., using a single strand as a template for extension of the oligonucleotide to produce a strand containing the mutation (see Zoller *et al*. (1982), Nuc. Acids Res., 10:6487-6500; Norris et al. (1983), Nuc. Acids Res., 11:5103-5112; Zoller et al. (1984), DNA. 3:479-488; Kramer *et al.* (1982), Nuc. Acids Res., 10:6475-6485 and polymerase chain reaction, i.e., exponentially amplifying DNA *in vitro* using sequence specified oligonucleotides to incorporate selected changes (see PCR Technology: Principles and Applications for DNA Amplification, Erlich, (ed.) (1989); and Horton *et al. supra*).

Further, the antibodies may have their constant region domain modified, ie., the C_{L}, CH₁, hinge, CH₂, CH₃ and/or CH₄ domains of an antibody polypeptide chain may be deleted, inserted or changed (see EPO 327 378 A1 to Morrison *et al*., the Trustees of Columbia University; USP 4,642,334 to Moore *et al*., DNAX; and USP 4,704,692 to Ladner *et al*., Genex).

Once a final DNA construct is obtained, the composite Hum4 V_{L}, V_{H} antibodies may be produced in large quantities by injecting the host cell into the peritoneal cavity of pristane-primed mice, and after an appropriate time (about 1-2 weeks), harvesting ascites fluid from the mice, which yields a very high titer of homogeneous composite Hum4 V_{L}, V_{H} antibodies, and isolating the composite Hum4 V_{L}, V_{H} antibodies by methods well known in the art (see Stramignoni, *et al*. (1983), Intl. J. Cancer, 31:543-552). The host cell are grown *in vivo*, as tumors in animals, the serum or ascites fluid of which can provide up to about 50 mg/mL of composite Hum4 V_{L}, V_{H} antibodies. Usually, injection (preferably intraperitoneal) of about 10⁶ to 10⁷ histocompatible host cells into mice or rats will result in tumor formation after a few weeks. It is possible to obtain the composite Hum4 V_{L}, V_{H} antibodies from a fermentation culture broth of procaryotic and eucaryotic cells, or from inclusion bodies of *E.coli* cells (see Buckholz and Gleeson (1991), BIO/TECHNOLOGY, 9:1067-1072. The composite Hum4 V_{L}, V_{H} antibodies can then be collected and processed by well-known methods (see generally, Immunological Methods, vols. I & II, eds. Lefkovits, I. and Pernis, B., (1979 & 1981) Academic Press, New York, N.Y.: and Handbook of Experimental Immunology, ed. Weir, D., (1978) Blackwell Scientific Publications, St. Louis, MO.)

The composite Hum4 V_{L}, V_{H} antibodies can then be stored in various buffer solutions such as phosphate buffered saline (PBS), which gives a generally stable antibody solution for further use.

### Uses

The composite Hum4 V_{L}, V_{H} antibodies provide unique benefits for use in a variety of cancer treatments. In addition to the ability to bind specifically to malignant cells and to localize tumors and not bind to normal cells such as fibroblasts, endothelial cells, or epithelial cells in the major organs, the composite Hum4 V_{L}, V_{H} antibodies may be used to greatly minimize or eliminate ANHA responses thereto. Moreover, TAG-72 contains a variety of epitopes and thus it may be desirable to administer several different composite Hum4 V_{L}, V_{H} antibodies which utilize a variety of V_{H} in combination with Hum4 V_{L}.

Specifically, the composite Hum4 V_{L}, V_{H} antibodies are useful for, but not limited to, *in vivo* and *in vitro* uses in diagnostics, therapy, imaging and biosensors.

The composite Hum4 V_{L}, V_{H} antibodies may be incorporated into a pharmaceutically acceptable, non--toxic, sterile carrier. Injectable compositions of the present invention may be either in suspension or solution form. In solution form the complex (or when desired the separate components) is dissolved in a pharmaceutically acceptable carrier. Such carriers comprise a suitable solvent, preservatives such as benzyl alcohol, if needed, and buffers. Useful solvents include, for example, water, aqueous alcohols, glycols, and phosphonate or carbonate esters. Such aqueous solutions generally contain no more than 50 percent of the organic solvent by volume.

Injectable suspensions require a liquid suspending medium. with or without adjuvants, as a carrier. The suspending medium can be, for example, aqueous polyvinyl-pyrrolidone, inert oils such as vegetable oils or highly refined mineral oils, or aqueous carboxymethlycellulose. Suitable physiologically-acceptable adjuvants, if necessary to keep the complex in suspension, may be chosen from among thickeners such as carboxymethylcellulose, polyvinylpyrrolidone, gelatin, and the alginates. Many surfactants are also useful as suspending agents, for example, lecithin, alkylphenol, polyethylene oxide adducts, naphthalenesulfonates, alkylbenzenesulfonates, and the polyoxyethylene sorbitan esters. Many substances which effect the hydrophibicity, density, and surface tension of the liquid suspension medium can assist in making injectable suspensions in individual cases. For example, silicone antifoams, sorbitol. and sugars are all useful suspending agents.

Methods of preparing and administering conjugates of the composite Hum4 V_{L}, V_{H} antibody, and a therapeutic agent are well known to or readily determined. Moreover, suitable dosages will depend on the age and weight of the patient and the therapeutic agent employed and are well known or readily determined.

Conjugates of a composite Hum4 V_{L}, V_{H} antibody and an imaging marker may be administered in a pharmaceutically effective amount for the *in vivo* diagnostic assays of human carcinomas, or metastases thereof, in a patient having a tumor that expresses TAG-72 and then detecting the presence of the imaging marker by appropriate detection means.

Administration and detection of the conjugates of the composite Hum4 V_{L}, V_{H} antibody and an imaging marker, as well as methods of conjugating the composite Hum4 V_{L}, V_{H} antibody to the imaging marker are accomplished by methods readily known or readily determined. The dosage of such conjugate will vary depending upon the age and weight of the patient. Generally, the dosage should be effective to visualize or detect tumor sites, distinct from normal tissues. Preferably, a one-time dosage will be between 0.1 mg to 200 mg of the conjugate of the composite Hum4 V_{L} antibody and imaging marker per patient.

Examples of imaging markers which can be conjugated to the composite Hum4 V_{L} antibody are well known and include substances which can be detected by diagnostic imaging using a gamma scanner or hand held gamma probe, and substances which can be detected by nuclear magnetic resonance imaging using a nuclear magnetic resonance spectrometer.

Suitable, but not limiting, examples of substances which can be detected using a gamma scanner include ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re and ^{99m}Tc, An example of a substance which can be detected using a nuclear magnetic resonance spectrometer is gadolinium.

Conjugates of a composite Hum4 V_{L}, V_{H} antibodies and a therapeutic agent may be administered in a pharmaceutically effective amount for the *in vivo* treatment of human carcinomas, or metastases thereof, in a patient having a tumor that expresses TAG-72. A "pharmaceutically effective amount" of the composite Hum4 V_{L} antibody means the amount of said antibody (whether unconjugated, i.e., a naked antibody, or conjugated to a therapeutic agent) in the pharmaceutical composition should be sufficient to achieve effective binding to TAG-72.

Exemplary naked antibody therapy includes, for example, administering heterobifunctional composite Hum4 V_{L}, V_{H} antibodies coupled or combined with another antibody so that the complex binds both to the carcinoma and effector cells, e.g., killer cells such as T cells, or monocytes. In this method, the composite Hum4 V_{L} antibody-therapeutic agent conjugate can be delivered to the carcinoma site thereby directly exposing the carcinoma tissue to the therapeutic agent. Alternatively, naked antibody therapy is possible in which antibody dependent cellular cytoxicity or complement dependent cytotoxicity is mediated by the composite Hum4 V_{L} antibody.

Examples of the antibody-therapeutic agent conjugates which can be used in therapy include antibodies coupled to radionuclides, such as ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I and ¹¹¹In; to drugs, such as methotrexate, adriamycin; to biological response modifiers, such as interferon and to toxins, such as ricin.

Methods of preparing and administering conjugates of the composite Hum4 V_{L}, V_{H} antibodies and a therapeutic agent are well known or readily determined. The pharmaceutical composition may be administered in a single dosage or multiple dosage form. Moreover, suitable dosages will depend on the age and weight of the patient and the therapeutic agent employed and are well known or readily determined.

Composite Hum4 V_{L}, V_{H} antibodies, and particularly composite Hum4 V_{L}, V_{H} single chain antibodies thereof, are particularly suitable for radioimmunoguided surgery (RIGS). In RIGS, an antibody labeled with an imaging marker is injected into a patient having a tumor that expresses TAG-72. The antibody localizes to the tumor and is detected by a hand-held gamma detecting probe (GDP). The tumor is then excised (see Martin *et al*. (1988), Amer. J. Surg., **156**:386-392; and Martin *et al*. (1986), Hybridoma, **5**:S97-S108). An exemplary GDP is the Neoprobe™ scanner, commercially available from Neoprobe Corporation, Columbus, OH. The relatively small size and human character of the composite Hum4 V_{L}, V_{H} single chain antibodies will accelerate whole body clearance and thus reduce the waiting period after injection before surgery can be effectively initiated.

Administration and detection of the composite Hum4 V_{L}, V_{H} antibody-imaging marker conjugate may be accomplished by methods well-known or readily determined.

The dosage will vary depending upon the age and weight of the patient, but generally a one time dosage of about 0.1 to 200 mg of antibody-marker conjugate per patient is administered.

### EXAMPLES

The following nonlimiting examples are merely for illustration of the construction and expression of composite Hum4 V_{L}, V_{H} antibodies. All temperatures not otherwise indicated are Centigrade. All percents not otherwise indicated are by weight.

### Example I

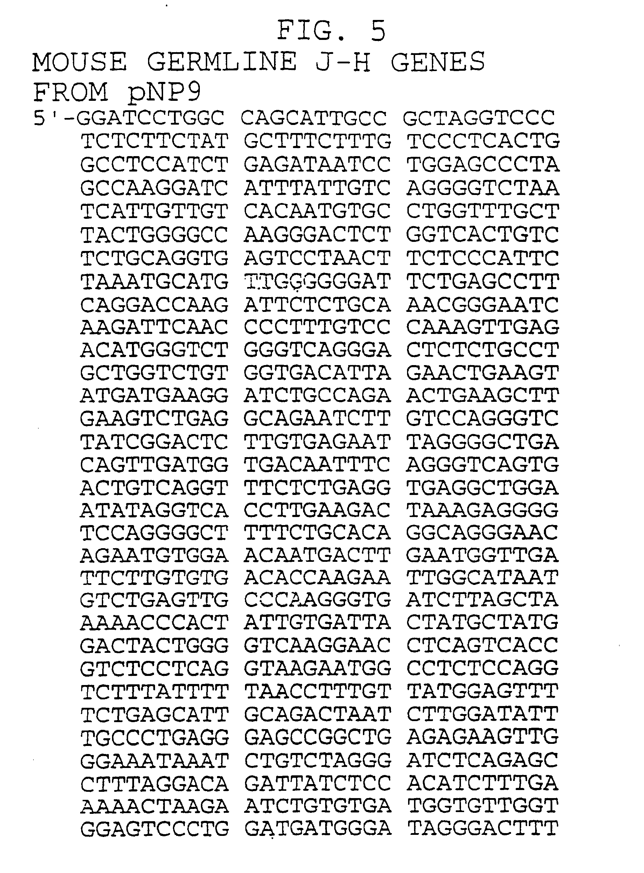

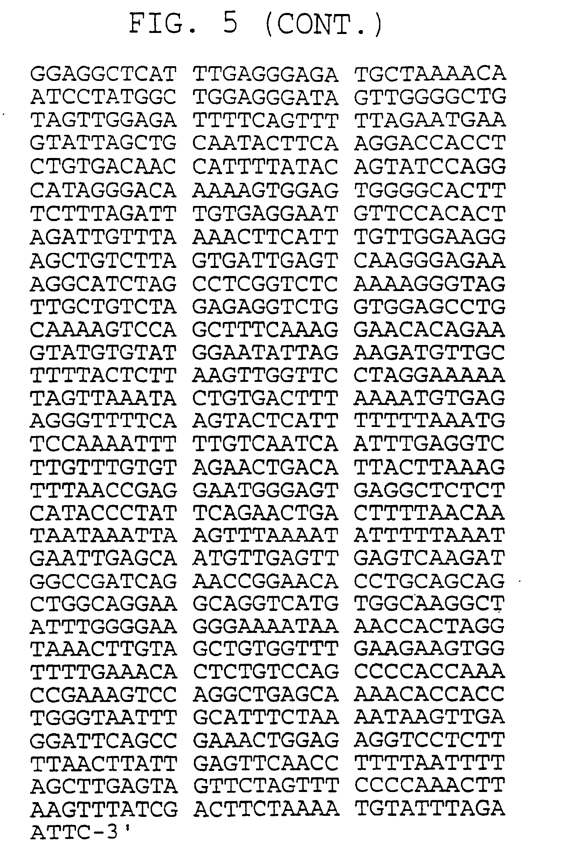

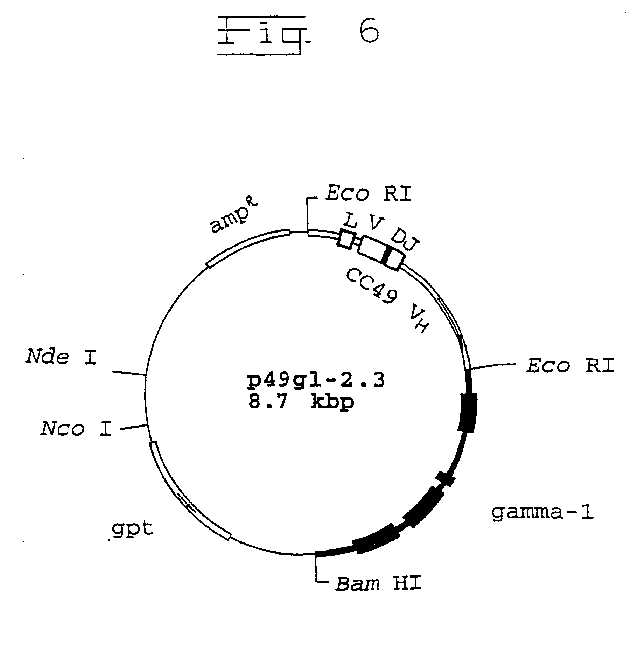

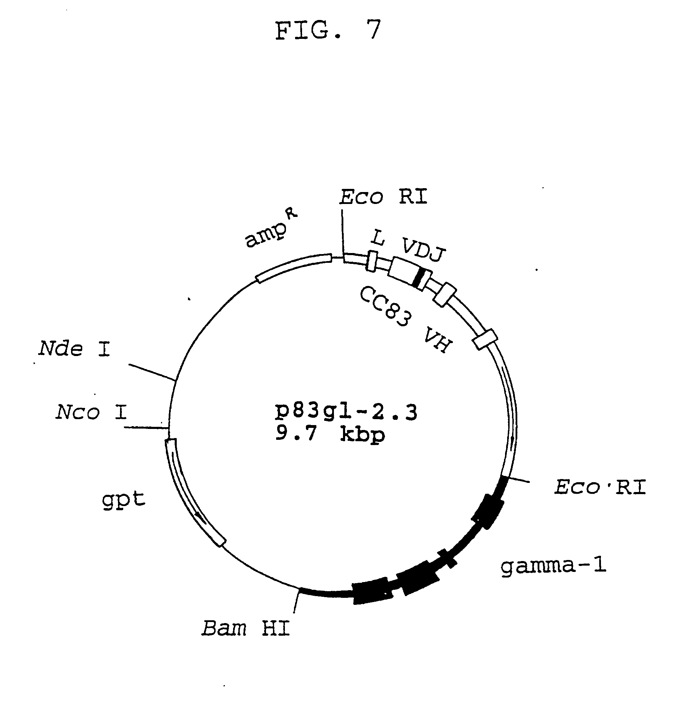

CC49 and CC83 were isolated from their respective hybridomas using pNP9 as a probe (see Figure 5). CC49 V_{H} was obtained from p49 g1-2.3 (see Figure 6) and CC83 V_{H} was obtained from p83 g1-2.3 (see Figure 7), following the procedures set forth in EPO 0 365 997.

DNA encoding an antibody light chain was isolated from a sample of blood from a human following the protocol of Madisen *et. al.* (1987), Am. J. Med. Genet., 27:379-390) with several modifications. Two 5 ml purple-cap Vacutainer tubes (containing EDTA as an anticoagulant) were filled with blood and stored at ambient temperature for 2 hours. The samples were transferred to two 4.5 mL centrifuge tubes. To each tube was added 22.5 mL of filter-sterilized erythrocycte lysate buffer (0.155 M NH₄Cl and 0.17 M Tris, pH 7.65, in a volume ratio of 9:1), and incubated at 37°C for 6.5 minutes. The tubes became dark red due to the lysed red blood cells. The samples were centrifuged at 9°C for 10 minutes, using an SS-34 rotor and a Sorvall centrifuge at 5,300 revolutions per minute (rpm) (⁻3,400 X g). The resulting white cell pellets were resuspended in 25 mL of 0.15 M NaCl solution. The white blood cells were then centrifuged as before. The pellets were resuspended in 500 µL of 0.15 M NaCl and transferred to 1.5 mL microcentrifuge tubes. The cells were pelleted again for 3 minutes, this time in the microcentrifuge at 3,000 rpm. Very few red blood cells remained on the pellet. After the supernatants were decanted from the two microcentrifuge tubes, 0.6 mL high TE buffer (100 mM Tris, pH 8.0) was added. The tubes were hand-shaken for 10 and 15 minutes. The resulting viscous solution was extracted with phenol, phenol-chloroform and finally with just chloroform as described in Sambrook *et al*., *supra.* To 3.9 mL of pooled extracted DNA solution was added 0.4 mL NaOAc (3 M, pH 5), and 10 mL 100 percent ethanol. A white stringy precipitate was recovered with a yellow pipette tip, transferred into a new Eppendorf tube, washed once with 70 percent ethanol, and finally washed with 100 percent ethanol. The DNA was dried *in vacuo* for 1 minute and dissolved in 0.75 mL deionized water. A 20 µL aliquot was diluted to 1.0 mL and the OD 260 nm value was measured and recorded. The concentration of DNA in the original solution was calculated to be 0.30 mg/mL.

Oligonucleotides (oligos) were synthesized using phosphoramidite chemistry on a 380A DNA synthesizer (Applied Biosystems, Foster, CA) starting on 0.2 µM solid support columns. Protecting groups on the final products were removed by heating in concentrated ammonia solution at 55°C for 12 hours. Crude mixtures of oligonucleotides (approximately 12 OD 260 nm units) were applied to 16 percent polyacrylamide-urea gels and electrophoresed. DNA in the gels was visualized by short wave UV light. Bands were cut out and the DNA eluted by heating the gel pieces to 65°C for 2 hours. Final purification was achieved by application of the eluted DNA solution onto C-18 Sep-Pac™ columns (Millipore) and elution of the bound oligonucleotide with a 60 percent methanol solution. The pure DNA was dissolved in deionized distilled water (ddH₂O) and quantitated by measuring OD 260 nm.

A GeneAmp™ DNA amplification kit (Cetus Corp., Emeryville, CA) was used to clone the Hum4 V_{L} germline gene by the PCR which was set up according to the manufacturer's directions. A thermal cycler was used for the denaturation (94 °C), annealing (45 °C) and elongation (72 °C) steps. Each of the three steps in a cycle were carried out for 4 minutes; there was a total of 30 cycles.

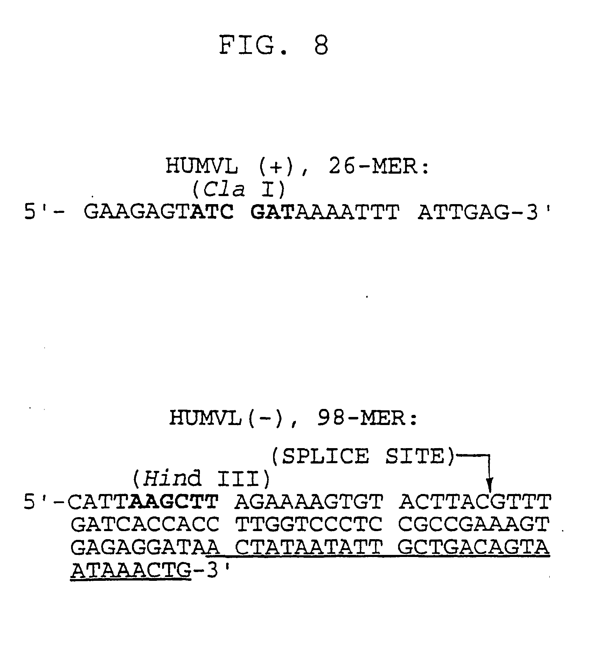

Upstream of the regulatory sequences in the Hum4 V_{L} germline gene, there is a unique *Cla* I restriction enzyme site. Therefore, the 5' end oligonucleotide for the PCR technique, called HUMVL(+) (Figure 8), was designed to include this *Cla* I site.

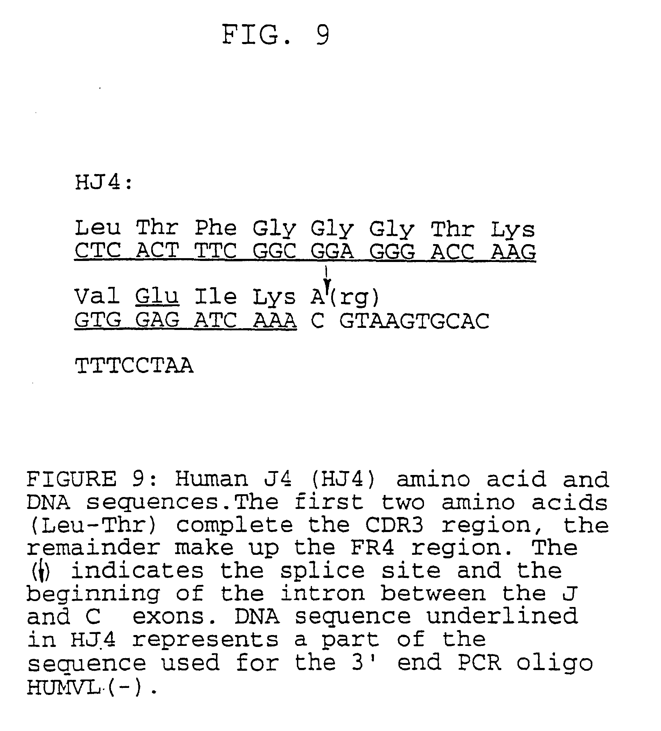

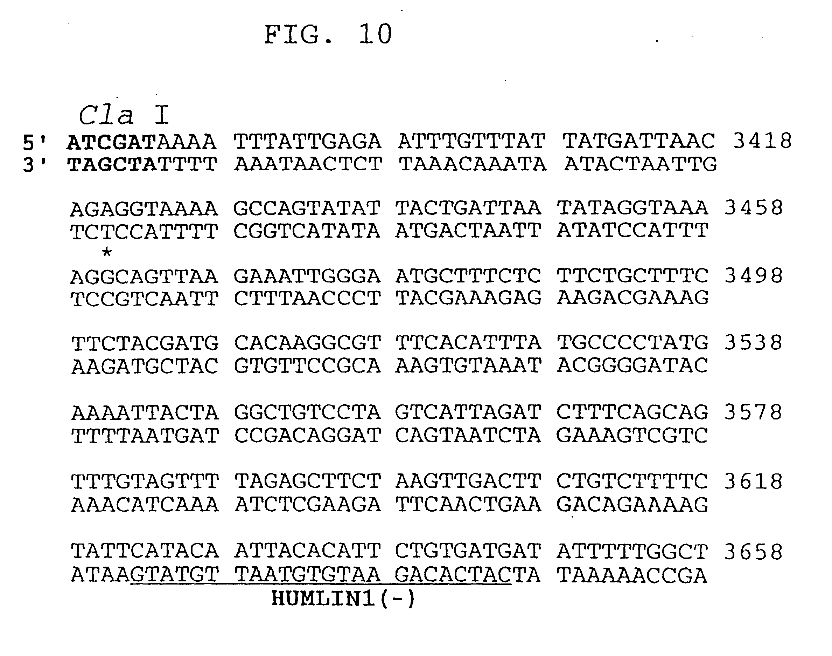

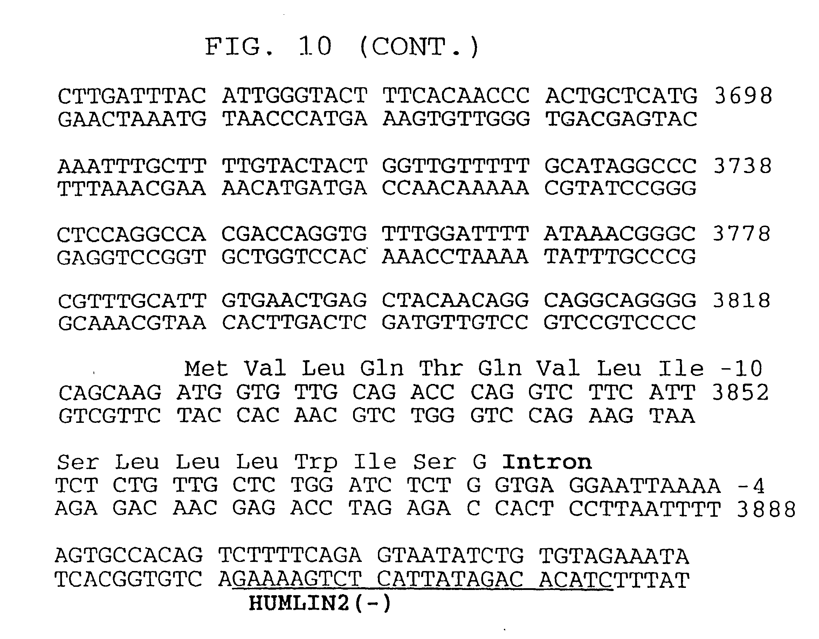

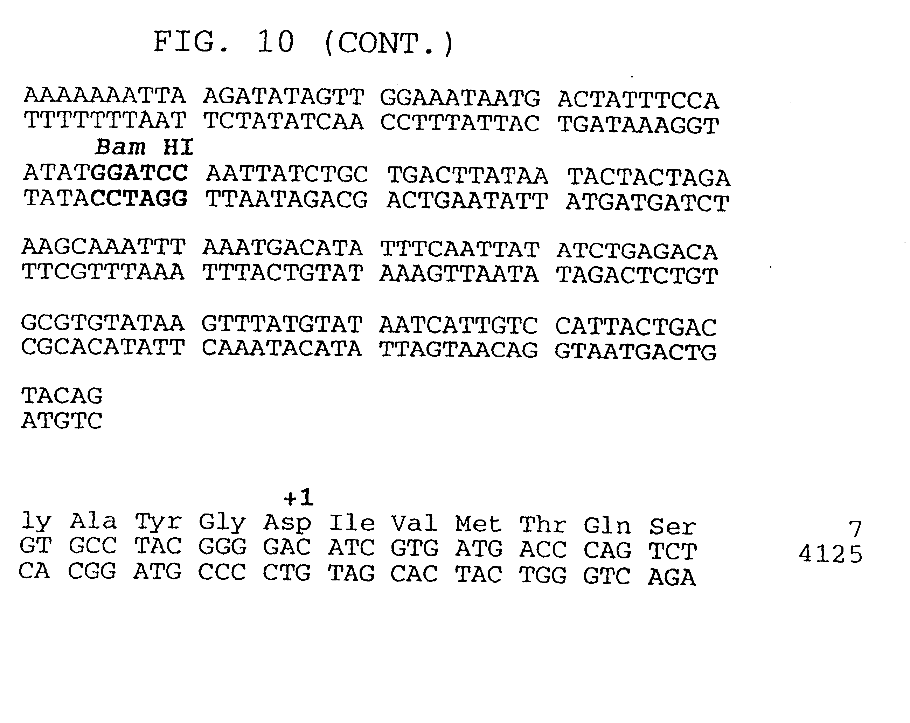

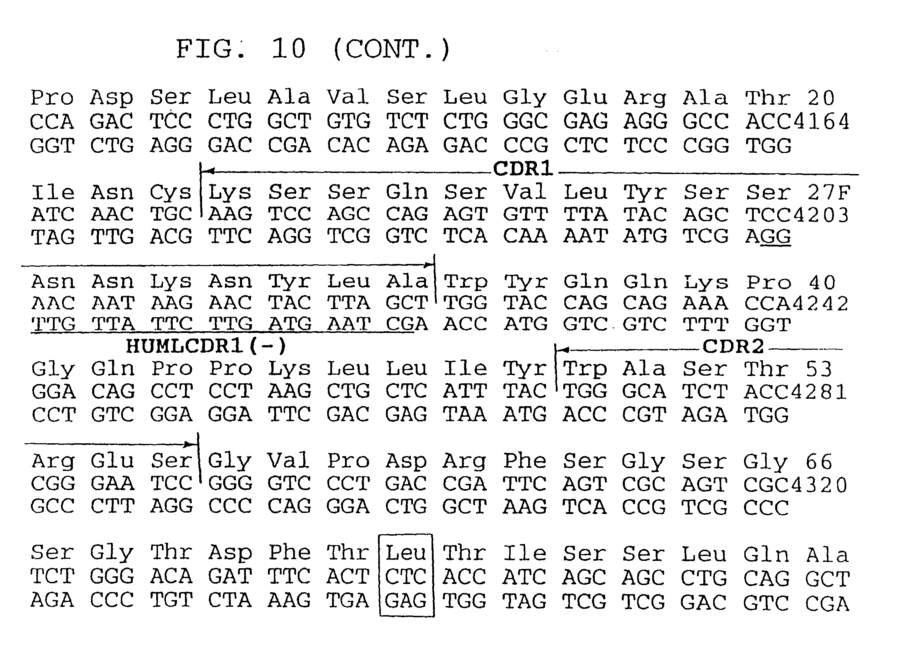

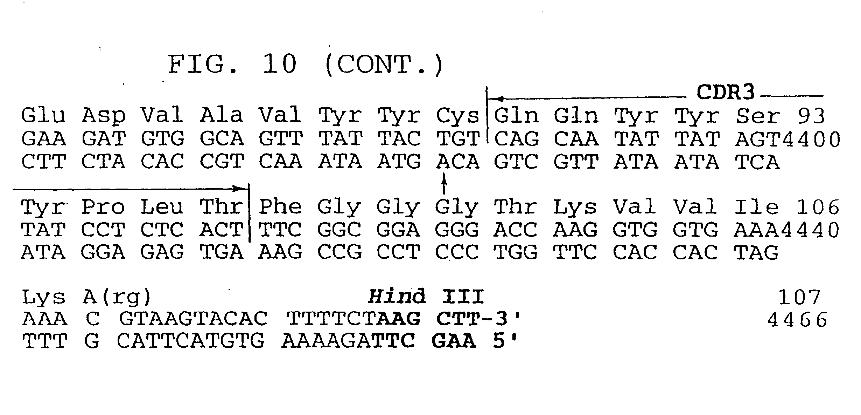

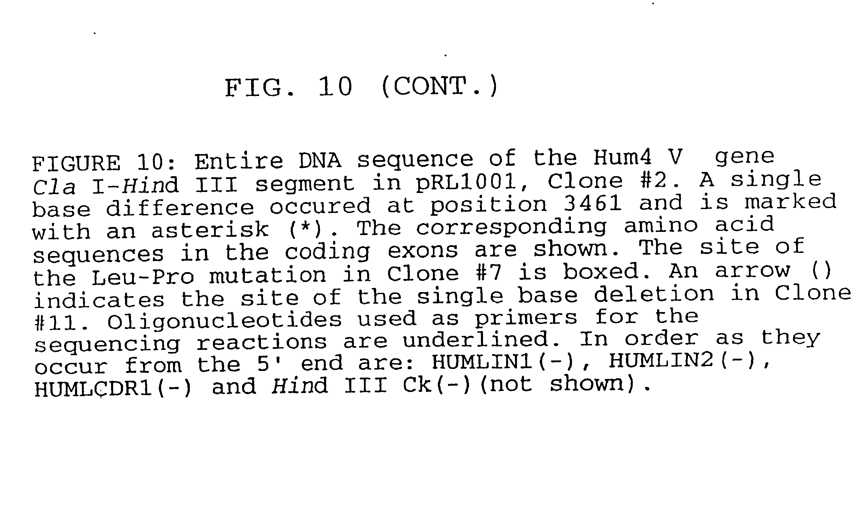

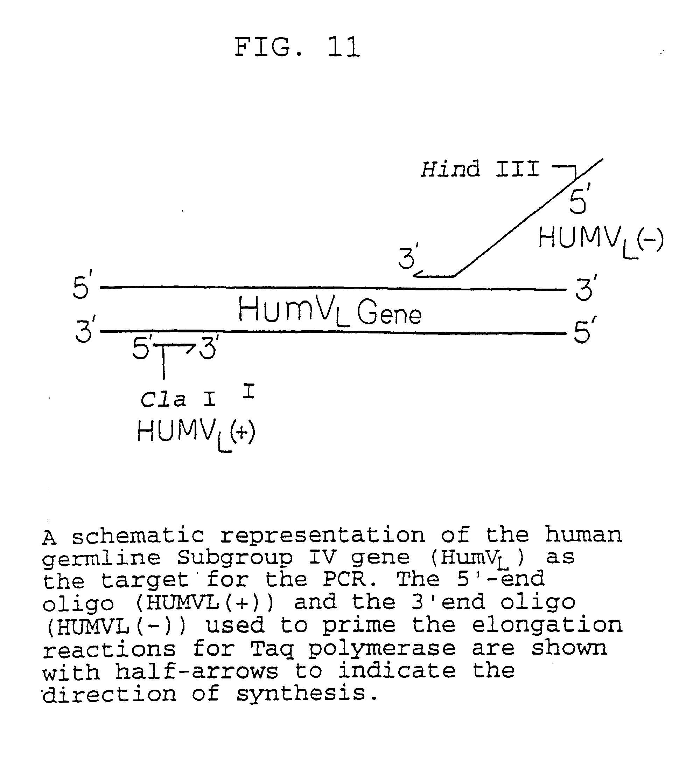

The 3' end oligonucleotide, called HUMVL(-) (Figure 8), contained a unique *Hin*d III site; sufficient mouse intron sequence past the splicing site to permit an effective splice donor function; a human J4 sequence contiguous with the 3' end of the V_{L} exon of Hum4 V_{L} to complete the CDR3 and FR4 sequences of the V_{L} domain (see Figures 9 and 10); nucleotides to encode a tyrosine residue at position 94 in CDR3; and 29 nucleotides close to the 3' end of the V_{L} exon of Hum4 V_{L} (shown underlined in the oligonucleotide HUMVL(-) in Figure 8) to anneal with the human DNA target. In total, this 3' end oligonucleotide for the PCR was 98 bases long with a non-annealing segment (a "wagging tail") of 69 nucleotides. A schematic of the Hum4 V_{L} gene target and the oligonucleotides used for the PCR are shown in Figure 11.

A PCR reaction was set up with 1 µg of total human DNA in a reaction volume of 100 µL. Primers HUMVL(-) and HUMVL(+) were each present at an initial concentratiuon of 100 pmol. Prior to the addition of *Taq* polymerase (2.5 units/reaction) 100 µLs of mineral oil were used to overlay the samples. Control samples were set up as outlined below. The samples were heated to 95 °C for 3 minutes. When the PCR was complete, 20 µL samples were removed for analysis by agarose gel electrophoresis.

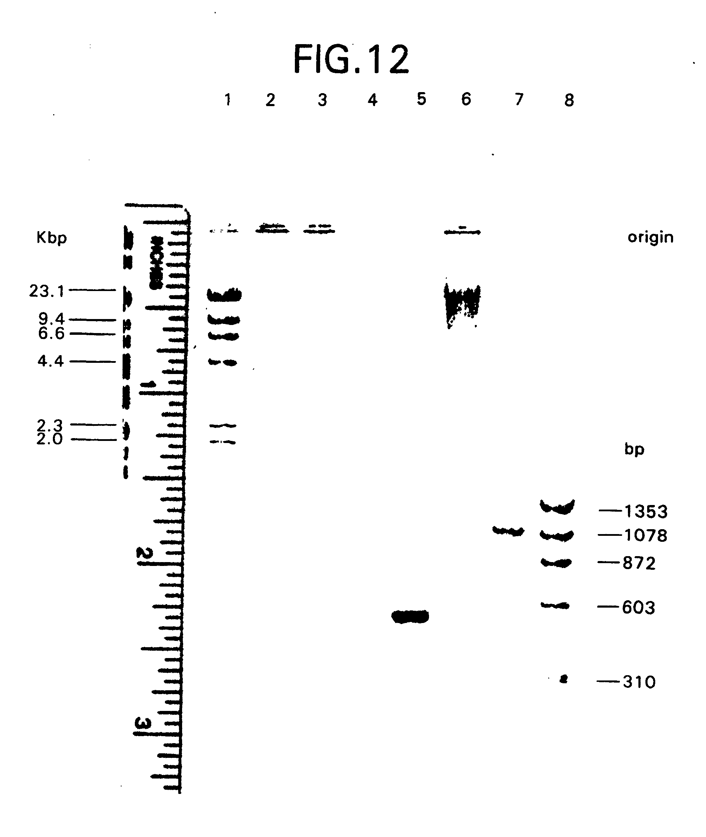

Based on the known size of the Hum4 V_{L} DNA fragment to be cloned, and the size of the oligonucleotides used to target the gene, a product of 1099 bp was expected. A band corresponding to this size was obtained in the reaction (shown in lane 7, Figure 12).

To prepare a plasmid suitable for cloning and subsequently expressing the Hum4 V_{L} gene, the plasmid pSV2neo was obtained from ATCC and subsequently modified. pSV2neo was modified as set forth below (see Figure 13).

The preparation of pSV2neo-101 was as follows. Ten micrograms of purified pSV2neo were digested with 40 units of *Hind* III at 37 °C for 1 hour. The linearized plasmid DNA was precipitated with ethanol, washed, dried and dissolved in 10 µL water. Two microliters each of 10 mM dATP, dCTP, dGTP and dTTP were added, as well as 2 µL of 10X ligase buffer. Five units (1 µL) of DNA polymerase I were added to make blunt the Hind III sticky ends. The reaction mixture was incubated at room temperature for 30 minutes. The enzyme was inactivated by heating the mixture to 65°C for 15 minutes. The reaction mixture was phenol extracted and ethanol precipitated into a pellet. The pellet was dissolved in 20 µl deionized, distilled water. A 2 µl aliquot (ca. 1 µg) was then added to a standard 20 µL ligation reaction, and incubated overnight at 4 °C.

Competent *E.coli* DH1 cells were transformed with 1 µL and 10 µL aliquots of the ligation mix (Invitrogen, San Diego, CA) according to the manufacturer's directions. Ampicillin resistant colonies were obtained on LB plates containing 100 µg/mL ampicillin. Selected clones grown in 2.0 mL overnight cultures were prepared, samples of plasmid DNA were digested with *Hin*d III and *Bam* HI separately, and a correct representative clone selected.

The resulting plasmid pSV2neo-101 was verified by size mapping and the lack of digestion with *Hin*d III.

A sample of DNA from pSV2neo-10 mini-lysate was prepared by digesting with 50 units of *Bam* HI at 37°C for 2 hours. The linearized plasmid was purified from a 4 percent DNA polyacrylamide gel by electroelution. The DNA ends were made blunt by filling in the *Bam* HI site using dNTPs and Klenow fragment, as described earlier for the *Hin*d III site of pSV2 neo-101.

A polylinker segment containing multiple cloning sites was incorporated at the *Bam* HI site of pSV2neo-101 to create pSV2neo-102. Equimolar amounts of two oligonucleotides, CH(+) and CH(-) (shown in Figure 14) were annealed by heating for 3 minutes at 90 °C and cooling to 50 °C. Annealed linker DNA and blunt ended pSV2neo-101 were added, in a 40:1 molar volume to a standard 20 µL ligation reaction. *E.coli* DH1 was transformed with 0.5 µL and 5 µL aliquots of the ligation mixture (Invitrogen). Twelve ampicillin resistant colonies were selected for analysis of plasmid DNA to determine whether the linker had been incorporated.

A *Hind* III digest of mini-lysate plasmid DNA revealed linker incorporation in six of the clones. The plasmid DNA from several clones was sequenced, to determine the number of linker units that were blunt-end ligated to pSV2neo-101 as well as the relative orientation(s) with the linker. Clones for sequencing were selected on the basis of positive digestion with *Hin*d III.

A Sequenase™ sequencing kit (United States B)iochemical Corp, Cleveland, OH was used to sequecne the DNA. A primer, NEO102SEQ, was used for sequencing and is shown in Figure 15. It is complementary to a sequence located upstream from the *Bam* HI site in the vector. Between 3 µg and 5 µg of plasmid DNA isolated from *E. coli* mini-lysates were used for sequencing. The DNA was denatured and precipitated prior to annealing, as according to the manufacturer's instructions. Electrophoresis was carried out at 1500 volts; gels were dried prior to exposure to Kodak X-ray film. Data was processed using Hitachi's DNASIS™ computer program.

From the DNA sequence data of 4 clones analyzed (see photograph of autoradiogram - Figure 16), compared to the expected sequence in Figure 14, two clones having the desired orientationwere obtained. A representative clone was selected and designated pSV2neo-102.

A human Cκ gene was inserted into pSV2neo-102 to form pRL1000. The human Cκ DNA was contained in a 5.0 kb *Hin*d III-*Bam* HI fragment (Hieter *et al*. (1980), Cell, **22**:197-207).

A 3 µg sample of DNA from a mini-lysate of pSV2neo-102 was digested with *Bam* HI and *Hin*d III. The vector DNA was separated from the small *Bam* HI-*Hin*d III linker fragment, generated in the reaction, by electrophoresis on a 3.75 percent DNA polyacrylaaide gel. The desired DNA fragment was recovered by electroelution. A pBR322 clone containing the 5.0 kb *Hin*d III-*Bam* HI fragment of the human Cκ gene (see Hieter *et al*., *supra*) was designated phumCκ. The 5.0 kb *Hin*d III-*Bam* HI fragment was ligated with pSV2neo-102r and introduced into *E.coli* DH1 (Invitrogen). Ampicillin resistant colonies were screened and a clone containing the human Ck gene was designated pRL1000.

Finally, pRL1000 clones were screened by testing mini-lysate plasmid DNA from *E.coli* with *Hin*d III and *Bam* HI. A clone producing a plasmid which gave 2 bands, one at 5.8 Kb (representing the vector) and the other at 5.0 kb (representing the human Cκ insert) was selected. Further characterization of pRL1000 was achieved by sequencing downstream from the *Hin*d III site in the intron region of the human Cκ insert. The oligonucleotide used to prime the sequencing reaction was NEO102SEQ (Figure 15). Two hundred and seventeen bases were determined (see Figure 17). A new oligonucleotide corresponding to the (-) strand near the *Hin*d III site (shown in Figure 17) was synthesized so that clones, containing the HHum4 V_{L} gene that were cloned into the *Cla* I and *Hind* III sites in pRL1000 (see Figure 13), could be sequenced.

A *Cla* I-*Hind* III DNA fragment containing Hum4 V_{L} obtained by PCR was cloned into the plasmid vector pRL1000. DNA of pRL1000 and the Hum4 V_{L} were treated with *Cla* I and *Hind* III and the fragments were gel purified by electrophoresis, as described earlier.

The pRL1000 DNA fragment and fragment containing Hum4 V_{L} gene were ligated, and the ligation mixture used to transform *E.coli* DH1 (Invitrogen), following the manufacturer's protocol. Ampicillin resistant clones were screened for the presence of the Hum4 V_{L} gene by restriction enzyme analysis and a representative clone designated pRL1001 (shown in Figure 18).

Four plasmids having the correct *Cla* I-*Hin*d III restriction pattern were analyzed further by DNA sequencing of the insert region (see Figure 19). *Hin*d III Cκ(-) (shown in Figure 17), HUMLIN1(-) (shown in Figure 10), HUMLIN2(-) (shown in Figure 10) were used as the sequencing primers. Two out of the four plasmids analyzed had the expected sequence in the coding regions (Figure 19, clones 2 and 9).

Clone 2 was chosen and used for generating sufficient plasmid DNA for cell transformations and other analysis. This plasmid was used for sequencing through the Hum4 V_{L}, and the upstream region to the *Cla* I site. Only one change at nucleotide position 83 from a C to a G (Figure 10) was observed, compared to a published sequence (Klobeck *et al*. (1985), supra). The DNA sequence data also indicates that the oligonucleotides used for the PCR had been correctly incorporated in the target sequence.

The Biorad Gene Pulser™ apparatus was used to transfect Sp2/0 cells with linearized plasmid DNAs containing the light or heavy chain constructs. The Hum4 V_{L} was introduced in Sp2/0 cells along with corresponding heavy chains by the co-transfection scheme indicated in Table 1.

**Table 1**

| Cell Line Designation | DNA Added | | |
|---|---|---|---|
| | L Chain pRL1001 | H Chain p49 g1-2.3 | H Chain p83 g1-2.3 |
| MP1-44H | 20 µg | 15 µg | 0 µg |
| MP1-84H | 20 µg | 0 µg | 15 ug |

A total of 8.0 X 10⁶ Sp2/0 cells were washed in sterile PBS buffer (0.8 mL of 1 X 107 viable cells/mL) and held on ice for 10 minutes. DNA of pRL1001, linearized at the *Cla* I site, and the DNA of either p49 g1-2.3 or p83 g1-2.3, linearized at their respective *Nde* I sites, were added, in sterile PBS, to the cells (see protocol - Table 2) and held at 0 °C for a further 10 minutes. A single 200 volt, 960 µF electrical pulse lasting between 20 and 30 milliseconds was used for the electroporation. After holding the perturbed cells on ice for 5 minutes, 25 mL of RPMI medium with 10 percent fetal calf serum were introduced, and 1.0 mL samples aliquoted in a 24 well tissue culture plate. The cells were incubated at 37 °C in a 5 percent CO₂ atmosphere. After 48 hours, the media was exchanged with fresh selection media, now containing both 1 mg/mL Geneticin (G418) (Difco) and 0.3 µg/ml mycophenolic acid/gpt medium. Resistant cells were cultured for 7-10 days.

Supernatants from wells having drug resistant colonies were tested on ELISA plates for activity against TAG-72. A roughly 10 percent pure TAG-72 solution prepared from LS147T tumor xenograft cells was diluted 1:40 and used to coat flexible polyvinyl chloride microtitration plates (Dynatech Laboratories, Inc.). Wells were air-dried overnight, and blocked the next day with 1 percent BSA. Supernatant samples to be tested for anti-TAG-72 antibody were added to the washed wells and incubated for between 1 and 2 hours at 37 °C. Alkaline phosphatase labeled goat anti-human IgG (diluted 1:250) (Southern Biotech Associates, Birmingham, AL) was used as the probe antibody. Incubation was for 1 hour. The substrate used was p-nitrophenylphosphate. Color development was terminated by the addition of 1.0 N NaOH. The plates were read spectrophotometrically at 405 nm and 450 nm, and the values obtained were 405 nm-450 nm.

Those samples producing high values in the assay were subcloned from the original 24 well plate onto 96 well plates. Plating was done at a cell density of half a cell per well (nominally 50 cells) to get pure monoclonal cell lines. Antibody producing cell lines were frozen down in media containing 10 percent DMSO.

Two cell lines were procured having the designations: MP1-44H and MP1-84H. MP1-44H has the chimeric CC49 γ1 heavy chain with the Hum4 V_{L} light chain; and MP1-84H has the chimeric CC83 g1 heavy chain with the HumVkIV light chain.

A 1.0 L spinner culture of the cell line MP1-44H was grown at 37°C for 5 days for antibody production. The culture supernatant was obtained free of cells by centrifugation and filtration through a 0.22 micron filter apparatus. The clarified supernatant was passed over a Protein A cartridge (Nygene, New York). Immunoglobulin was eluted using 0.1 M sodium citrate buffer pH 30. The pH of the eluting fractions containing the antibody was raised to neutrality by the addition of Tris base, pH 9.0. The antibody-containing fractions were concentrated and passed over a Pharmacia Superose 12 HR 10/30 gel filtration column. A protein was judged to be homogeneous by SDS polyacrylamide gel electrophoresis. Isoelectric focusing further demonstrated the purity of MP1-44H.

The biological performance of the human composite antibody, MP1-44H, was evaluated by comparing immunohistochemistry results with two other anti-TAG-72 antibdoies CC49 (ATCC No. HB 9459) and Ch44 (ATCC No. HB 9884). Sections of human colorectal tumor embedded in paraffin were tested with the three antibodies by methods familiar to those skilled in this art. All three antibodies gave roughly equivalent binding recognition of the tumor antigen present on the tumor tissue sample.

A further test of the affinity and biological integrity of the human composite antibody MP1-44H was a competition assay, based on cross-competing radioiodine--labeled versions of the antibody with CC49 and Ch44 in all combinations. From the data shown in Figure 20, it is apparent that the affinity of all 3 antibodies is equivalent and can bind effectively to tumor antigen.

MP1-44H (ATCC HB 10426) and MP1-84H (ATCC HB 10427) were deposited at the American Type Culture Collection (ATCC). The contract with ATCC provides for permanent availability of the cell lines to the public on the issuance of the U.S. patent describing and identifying the deposit or the publications or upon the laying open to the public of any U.S. or foreign patent application, which ever comes first, and for availability of the cell line to one determined by the U.S. Commissioner of Patents and Trademarks to be entitled thereto according to 35 CFR §122 and the Commissioner's rules pursuant thereto (including 37 CFR §1.14 with particular reference to 886 OG 638). The assignee of the present application has agreed that if the cell lines on deposit should die or be lost or destroyed when cultivated under suitable conditions for a period of thirty (30) years or five (5) years after the last request, it will be promptly replaced on notification with viable replacement cell lines.

### Example 2

Single-chain antibodies consist of a V_{L}, V_{H} and a peptide linker joining the V_{L} and V_{H} domains to produce SCFVs. A single chain antibody, SCFV1, was constructed to have the Hum4 V_{L} as V Domain 1 and CC49 V_{H} as V Domain 2 (see Figure 21).

The polypeptide linker which joins the two V domains was encoded by the DNA introduced at the 3' end of the V_{L} DNA during the PCR. The oligonucleotides SCFV1a and SCFV2 were designed to obtain the DNA segment incorporating part of the yeast invertase leader sequence, the Hum4 V_{L} and the SCFV linker.

The polypeptide linker for SCFV1 was encoded in oligonucleotide SCFV1b (see below). The underlined portions of the oligonucleotides SCFV1a and SCFV1b are complementary to sequences in the Hum4 V_{L} and linker respectively. The sequences of SCFV1a and SCFV1b are as follows, with the hybridizing sequences underlined:
SCFV1a with the *Hin*d III in bold: 
Hind III

5'CTGCAAGCTTCCTTTTCCTTTTGGCTGGTTTTGCAGCCAAAATATCTGCAG
ACATCGTGATGACCCAGTC-3'
SCFV1b with the *Aat* II site in bold:

5'-CGTAAGACGTCTAAGGAACGAAATTGGGCCAATTGTTCTGAGGA
GACCGAACCTGACTCCTTCACCTTGGTCCCTCCGCCG-3'

The target DNA in the PCR was pRL1001 (shown in Figure 18). The PCR was performed pursuant to the teachings of Mullis *et al*. *supra.* A DNA fragment containing the Hum4 V_{L}-linker DNA component for the construction of SCFV1 was obtained and purified by polyacrylamide gel electrophoresis according to the teachings of Sambrook *et al*., *supra.*

p49 g1-2.3, containing CC49 V_{H}, was the target DNA in the PCR. PCR was performed according to the methods of Mullis *et al., supra*. The oligonucleotides used for the PCR of CC49 V_{H} are as follows, with the hybridizing sequences underlined:
SCFV1c, with the *Aat* II site in bold: 5'-CCTTAGACGTCCAGTTGCAGCAGTCTGACGC-3'
SCFV1d, with the *Hin*d III site in bold:

5'-GATCAAGCTTCACTAGGAGACGGTGACTGAGGTTCC-3'

The purified Hum4 V_{L}-linker and VH DNA fragments were treated with *Aat* II (New England Biolabs, Beverly, MA) according to the manufacturer's protocol, and purified from a 5 percent polyacrylamide gel after electrophoresis. An equimolar mixture of the *Aat* II fragments was ligated overnight. The T4 DNA ligase was heat inactivated by heating the ligation reaction mixture at 65 °C for 10 minutes. Sodium chloride was added to the mixture to give a final concentration of 50 mM and the mixture was further with *Hin*d III. A *Hin*d III DNA fragment was isolated and purified from a 4.5 percent polyacrylamide gel and cloned into a yeast expression vector (see Carter *et al*. (1987), In: DNA Cloning, A Practical Approach, Glover (ed.) Vol. III: 141-161). The sequence of the fragment, containing the contiguous SCFV1 construct, is set forth in Figure 22.

The anti-TAG-72 SCFV1 described herein utilized the yeast invertase leader sequence (shown as positions -19 to -1 of Figure 22), the Hum4 V_{L} (shown as positions 1 to 113 of Figure 22), an 18 amino acid linker (shown as positions 114 to 132 of Figure 22) and CC49 V_{H} (shown as positions 133 to 248 of Figure 22).

The complete DNA and amino acid sequence of SCFV1 is given in Figure 22. The oligonucleotides used to sequence the SCFV1 are set forth below.

TPI:

5'-CAATTTTTTGTTTGTATTCTTTTC-3'.

HUVKF3:

5'-CCTGACCGATTCAGTGGCAG-3'.

DC113:

5'-TCCAATCCATTCCAGGCCCTGTTCAGG-3'.

SUC2T:

5'-CTTGAACAAAGTGATAAGTC-3'.

### Example 3

A plasmid, pCGS517 (Figure 23), containing a prorennin gene was digested with *Hin*d III and a 6.5 kb fragment was isolated. The plasmid pCGS517 has a triosephosphate isomerase promoter, invertase [SUC2] signal sequence, the prorennin gene and a [SUC2] terminator. The *Hind* III-digested SCFV1 insert obtained above (see Figure 23) was ligated overnight with the *Hin*d III fragment of pCGS517 using T4 DNA ligase (Stratagene, La Jolla, CA).

The correct orientation existed when the *Hind* III site of the insert containing part of the invertase signal sequence ligated to the vector DNA to form a gene with a contiguous signal sequence. *E.coli* DH1 (Invitrogen) cells were transformed and colonies screened using a filter-microwave technique (see Buluwela, *et al.* (1989), Nucleic Acids Research, 17:452). From a transformation plate having several hundred colonies, 3 positive clones were obtained. Digesting the candidate plasmids with *Sal* I and *Kpn* I, each a single cutter, differentiated between orientations by the size of the DNA fragments produced. A single clone, pDYSCFV1 (Figure 23), had the correct orientation and was used for further experimentation and cloning. The probe used was derived from pRL1001, which had been digested with *Kpn* I and *Cla* I (see Figure 18). The probe DNA was labeled with ³²p α-dCTP using a random oligcnucleotide primer labeling kit (Pharmacia LKB Biotechnology, Piscataway, NJ).

The next step was to introduce the *Bgl* II-*Sal* 1 fragment from pDYSCFV1 into the same restriction sites of another vector (ca. 9 kb), which was derived from PCGS515 (Figure 23). to give an autonomously replicating plasmid in *S. cerevisiae*.

DNA from the vector and insert were digested in separate reactions with *Bgl* II and *Sal* I using 10X buffer number 3 (50 MM Tris-HCI (pH 8.0), 100 mM NaCl, BRL). The DNA fragment from pDYSCFV1 was run in and electroeluted from a 5 percent polyacrylamide gel and the insert DNA was run and electroeluted from a 3.75 percent polyacrylamide gel. A standard ligation using T4 DNA ligase (Stratagene) and a transformation using *E. coli* DH1 (Invitrogen) was carried out. Out of 6 clones selected for screening with *Bgl* II and *Sal* II, all 6 were correctly oriented, and one was designated pCGS515/SCFV1 (Figure 23).

DNA sequencing of pCGS515/SCFVI DNA was done using a Sequenase™ kit (U.S. Biochemical, Cleveland, OH) using pCGS515/SCFV1 DNA. The results have been presented in Figure 22 and confirm the sequence expected, based on the linker, the Hum4 V_{L} and the CC49 V_{H}.

Transformation of yeast cells using the autonomosly replicating plasmid pCGS515/SCFV1 was carried out using the lithium acetate procedures described in Ito *et al.* (1983), J. Bacteriol., **153**:163-168; and Treco (1987), In: Curent Protocols in Molecular Biology, Ausebel *et al.* (eds), 2:13.71-13.7.6. The recipient strain of *S.cerevisiae* was CGY1284 having the genotype - MAT α (mating strain α), ura 3-52 (uracil auxotrophy), SSC1-1 (supersecreting 1), and PEP4⁺ (peptidase 4 positive).

Transformed clones of CGY1284 carrying SCFV plasmids were selected by their ability to grow on minimal media in the absence of uracil. Transformed colonies appeared within 3 to 5 days. The colonies were transferred, grown and plated in YEPD medium. Shake flasks were used to provide culture supernatant with expressed product.

An ELISA procedure was used to detect biological activity of the SCFV1. The assay was set up such that the SCFV would compete with biotinylated CC49 (biotin-CC49) for binding to the TAG-72 antigen on the ELISA plate .

SCFV1 protein was partially purified from a crude yeast culture supernatant, using a Superose 12 gel filtration column (Pharmacia LKB Biotechnology), and found to compete with biotinylated CC49 in the competition ELISA. These results demonstrate that the SCFV1 had TAG-72 binding activity.

The SCFV1 protein has been detected by a standard Western protocol (see Towbin *et al*. (1979), Proc. Natl. Acad. Sci., U.S.A., 76:4350-4354). The detecting agent was biotinylated FAID14 (ATCC No. CRL 10256), an anti-idiotypic monoclonal antibody prepared from mice that had been immunized with CC49. A band was visualized that had an apparent molecular weight of approximately 26,000 daltons, the expected size of the SCFV1. This result demonstrated that the SCFV1 had been secreted and properly processed.

### Example 4

The following example demonstrates the cloning of human V_{H} genes into a SCFV plasmid construct containing sequence coding for the Hum4 V_{L} and a 25 amino acid linker called UNIHOPE.

A vector was prepared from plasmid pRW 83 containing a chloramphenicol resistance (Cam^{r} ) gene for clone selection, and a *penP* gene with a *pen*P promoter and terminator (see Mezes, *et al*. (1983), J. Biol. Chem.. **258:** 11211-11218) and the *pel B* signal sequence (see Lei *et al.* (1987) *supra*). The vector was designated Fragment A. (see Figure 24). The *penP* gene was removed with a *Hin*d III/*Sal* I digest.

The *penP* promoter and *pel B* signal sequence were obtained by a PCR using pRW 83 as a template and oligonucleotides penP1 and penP2 as primers. The fragment was designated Fragment B (see Figure 24). A *Nco* I enzyme restriction site was introduced at the 3' end of the signal sequence region by the penP2 oligonucleotide.

penP1:
5'-CGATAAGCTTGAATTCCATCACTTCC-3'

penP2:
5'-GGCCATGGCTGGTTGGGCAGCGAGTAATAACAATCCAGCG GCT
GCCGTAGGCAATAGGTATTTCATCAAAATCGTCTCCCTCCGTTTGAA-3'

A SCFV comprised of a Hun4 V_{L}, a CC49 V_{H}, and an 18 amino acid linker (Lys Glu Ser Gly Ser Val Ser Ser Glu Gln Leu Ala Gln Phe Arg Ser Leu Asp) was obtained from pCGS515/SCFV1 by PCR using oligonucleotides penP3 and penP6. This fragment was designated Fragment D (see Figure 24). A *Bcl* I site was introduced at the 3' end of the V_{H} region by the penP6 oligonucleotide.

penP3:
5'-GCTGCCCAACCAGCCATGGCCGACATCGTGATGACCCAGTCTCC-3'

penP6(-):

5'-CTCTTGATCACCAAGTGACTTTATGTAAGATGATGTTTTG ACG

GATTCATCGCAATGTTTTTATTTGCCGGAGACGGTGACTGAGGTTCC-3'

Fragments B and D were joined by PCR using oligonucleotides penP1 and penP6, following the procedures of Horton *et al*., *supra*. The new fragment was designated E (See Figure 24).

Fragment C containing the *penP* termination codon was isolated by digesting pRW 83 with *Bcl* I and *Sal* I, and designated Fragment C. pRW 83 was isolated from *E. coli* strain GM161, which is DNA methylase minus or dam⁻.

Plasmid pSCFV 31 (see Figure 24) was created with a three part ligation Fragments A, C, and E.

The *Nco* I restriction enzyme site within the Cam^{r} gene and the *Hin*d III site located at the 5' end of the *penP* promoter in pSCFV 31 were destroyed through a PCR DNA amplification using oligonucleotides Nco 1.1 and Nco1.3(-) to generate an *Eco* RI-*Nco* I fragment and oligonucleotides Nco1.2 and Nco1.4c(-) to generate a *Nco* I to *Eco* RI fragment. These two fragments were joined by PCR-SOE using oligonucleotides Nco1.1 and Nco1.4c(-). The oligonucleotides are set forth below:

Nco1.1:
5'-TCCGGAATTCCGTATGGCAATGA-3'

Nco1.3(-):
5'-CTTGCGTATAATATTTGCCCATCGTGAAAACGGGGGC-3'

Nco1.2:

5'-ATGGGCAAATATTATACGCAAG-3'

Nco1.4c(-):

5'-CACTGAATTCATCGATGATAAGCTGTCAAACATGAG-3'

pSCFV 31 was digested with *Eco* RI and the larger fragment was isolated by polyacrylamide gel electrophoresis. To prevent self ligation, the DNA was dephosphorylated using calf intertinal alkaline phosphatase according to the teachings of Sambrook *et al*., *supra.*

A two part ligation of the larger pSCFV 31 digested fragment and the PCR-SOE fragment, described above, resulted in the creation of pSCFV 31b (see Figure 25).

pSCFV 31b was digested with *Nco* I and *Sal* I and a fragment containing the Cam^{r} gene was isolated.

The Hum4 V_{L} was obtained by PCR DNA amplification using pCGS515/SCFV1 as a template and oligonucleotides 104BH1 and 104BH2(-) as primers.

104BH1:

5'-CAGCCATGGCCGACATCGTGATGACCCAGTCTCCA-3'

104BH2(-):

5'-AAGCTTGCCCCATGCTGCTTTAACGTTAGTTTTATCTGCTGG
AGACAGAGTGCCTTCTGCCTCCACCTTGGTCCCTCCGCCGAAAG-3'

The CC49 V_{H} was obtained by PCR using p49 g1-2.3 (Figure 5) as a template and oligonucleotides 104B3 and 104B4(-) as primers. A *Nhe* I enzyme restriction site was introduced just past the termination codon in the 3' end (before the *Bcl* I site) by oligonucleotide 104B4(-).

104B3:

5'-GTTAAAGCAGCATGGGGCAAGCTTATGACTCAGTTGCAGCAGTCTGACGC-3'

104B4(-):

5'-CTCTTGATCACCAAGTGACTTTATGTAAGATGATGTTTTGACGGATT
CATCGCTAGCTTTTTATTTGCCATAATAAGGGGAGACGGTGACTGAGGTTCC-3'

In the PCR which joined these two fragments using oligonucleotides 104BH1 and 104B4(-) as primers, a coding region for a 22 amino acid linker was formed.

A fragment C (same as above) containing the *penP* termination codon was isolated from pRW 83 digested with *Bcl* I and *Sal* I.

Plasmid pSCFV 33H (see figure 25) was created with a three part ligation of the vector, fragment C, and the SCFV fragment described above.

pSCFV 33H was digested with *Nco*I and *Nhe*I, and the DNA fragment containing the Cam^{r} gene was isolated as a vector.

Hum4 V_{L} was obtained by PCR DNA amplification using pRL1001 (see Figure 18) as a template and oligonucleotides UNIH1 and UNIH2(-) as primers. Oligonucleotides for the PCR were: 
UNIH1:
5'-CAGCCATGGCCGACATTGTGATGTCACAGTCTCC-3'
 The *Nco* I site is in bold and the hybridizing sequence is underlined. 
UNIH2(-):
5'-GAGGTCCGTAAGATCTGCCTCGCTACCTAGCAAA
AGGTCCTCAAGCTTGATCACCACCTTGGTCCCTCCGC-3'
 The *Hin*d III site is in bold.

The CC49 V_{H} was obtained by a PCR using p49 g1-2.3 (see Figure 6) as a template and oligonucleotides UNI3 and UNI4(-) as primers. 
UNI3:
5'-AGCGAGGCAGATCTTACGGACCTCGAGGTTCAGTTGCAGCAGTCTGAC-3'.
 The *Xho* I site is in bold and the hybridizing sequence is underlined. 
UNI4(-):

5'-CATCGCTAGCTTTTTATGAGGAGACGGTGACTGAGGTTCC-3'.
 The *Nhe* I site is in bold and the hybridizing sequence is underlined.

Oligonucleotides UNIH1 and UNI4(-) were used in the PCR-SOE amplification which joined the Hum4 V_{L} and CC49 V_{H} fragments and formed a coding region for a negatively charged fifteen amino acid linker. The DNA was digested with *Nhe* I and *Nco* I and ligated with the vector fragment from the *Nco* I-*Nhe* I digest of pSCFV 33H. The resultant plasmid was designated pSCFV UNIH (shown in Figure 25).

With the construction of pSCFV UNIH, a universal vector for any SCFV was created with all the desired restriction enzyme sites in place.

pSCFV UNIH was digested with *Hin*d III/*Xho* I. and the large DNA fragment containing the Cam^{r} gene. Hum4 V_{L} and CC49 V_{H} was isolated.

A fragment coding for a 25 amino acid linker, was made by annealing the two oligonucleotides shown below. The linker UNIHOPE is based on 205C SCA™ linker (see Whitlow, (1990) Antibody Engineering: New Technology and Application Implications, IBC USA Conferences Inc, MA), but the first amino acid was changed from serine to leucine and the twenty-fifth amino acid were was changed from glycine to leucine, to accomodate the *Hin*d III and *Xho* I restriction sites. The nucleotide sequence encoding the linker UNIHOPE is set forth below:

UNIHOPE (Figure 26):
5'-TATAAAGCTTAGTGCGGACGATGCGAAAAAGGATGCTGCGAAG
AAGGATGACGCTAAGAAAGACGATGCTAAAAAGGACCTCGAGTCTA-3'

UNIHOPE(-) (Figure 26):
5'TAGACTCGAGGTCCTTTTTAGCATCGTCTTTCTTAGCGT CAT
CCTTCTTCGCAGCATCCTTTTTCGCATCGTCCGCACTAAGCTTTATA-3'

The resulting strand was digested with *Hin*d III/*Xho* I and ligated into the vector, thus generating the plasmid pSCFV UHH (shown in Figure 27). Plasmid pSCFV UHH expresses a biologically active, TAG-72 binding SCFV consisting of the Hum4 V_{L} and CC49 V_{H}. The expression plasmid utilizes the β-lactamase *penP* promoter, pectate lyase *pel*B signal sequence and the *pen*P terminator region. Different immunoglobulin light chain variable regions can be inserted in the *Nco* I-*Hin*d III restriction sites, different SCFV linkers can be inserted in the *Hin*d III-*Xho* I sites and different immunoglobulin heavy chain variable regions can be inserted in the *Xho* I-*Nhe* I sites.

*E.coli* AG1 (Stratagene) was transformed with the ligation mix, and after screening, a single chloramphenicol resistant clone, having DNA with the correct restriction map, was used for further work.

The DNA sequence and deduced amino acid sequence of the SCFV gene in the resulting plasmid are shown in Figure 26.

*E.coli* AG1 containing pSCFV UHH were grown in 2 ml of LB broth with 20 µg/mL chloramphenicol (CAM 20). The culture was sonicated and assayed using a competition ELISA. The cells were found to produce anti-TAG-72 binding material. The competition assay was set up as follows: a 96 well plate was derivatized with a TAG-72 preparation from LS174T cells. The plate was blocked with 1% BSA in PBS for 1 hour at 31 °C and then washed 3 times with 200 µL of biotinylated CC49 (1/20,000 dilution of a 1 mg/mL solution) were added to the wells and the plate was incubated for 30 minutes at 31 °C. The relative amounts of TAG-72 bound to the plate, biotinylated CC49, streptavidin-alkaline phosphatase, and color development times were determined empirically in order not to have excess of either antigen or biotinylated CC49, yet have enough signal to detect competition by SCFV. Positive controls were CC49 at 5 µg/mL and CC49 Fab at 10 µL/mL. Negative controls were 1% BSA in PBS and/or concentrated LB. Unbound proteins were washed away.

Fifty microliters of a 1:1000 dilution of streptavidin conjugated with alkaline phosphatase (Southern Biotechnoiogy Associates, Inc., Birmingham, AL) were added and the plate was incubated for 30 minutes at 31 °C. The plate was washed 3 more times. Fifty microliters of a para-nitrophenylphosphate solution (Kirkegaard & Perry Laboratories, Inc., Gaithersburg, MD) were added and the color reaction was allowed to develop for a minimum of 20 minutes. The relative amount of SCFV binding was measured by optical density scanning at 405-450 nm using a microplate reader (Molecular Devices Corporation, Menio Park, CA). Binding of the SCFV resulted in decreased binding of the biotinylated CC49 with a concomitant decrease in color development. The average value for triplicate test samples is shown in the table below:

| Sample (50 µL) (mixed 1:1 with CC49 Biotin) | OD 405 nm - OD 450 nm Value at 50 minutes |
|---|---|
| Sonicate *E.coli* AG1/ pSCFVUHH clone 10 | 0.072 |
| Sonicate *E.coli* AG1/ pSCFVUHH clone 11 | 0.085 |
| CC49 at 5 mg/mL | 0.076 |
| CC49 Fab at 10 mg/mL | 0.078 |
| LB (negative control) | 0.359 |

The data indicates that there was anti-TAG-72 activity present in the *E.coli* AGI/pSCFVUHH clone sonicate.

### Example 7

The plasmid pSCFVUHH may be used to host other V_{H} genes on *Xho* I-*Nhe* I fragments and test in a SCFV format, following the procedures set forth below. A schematic for this process is shown here.

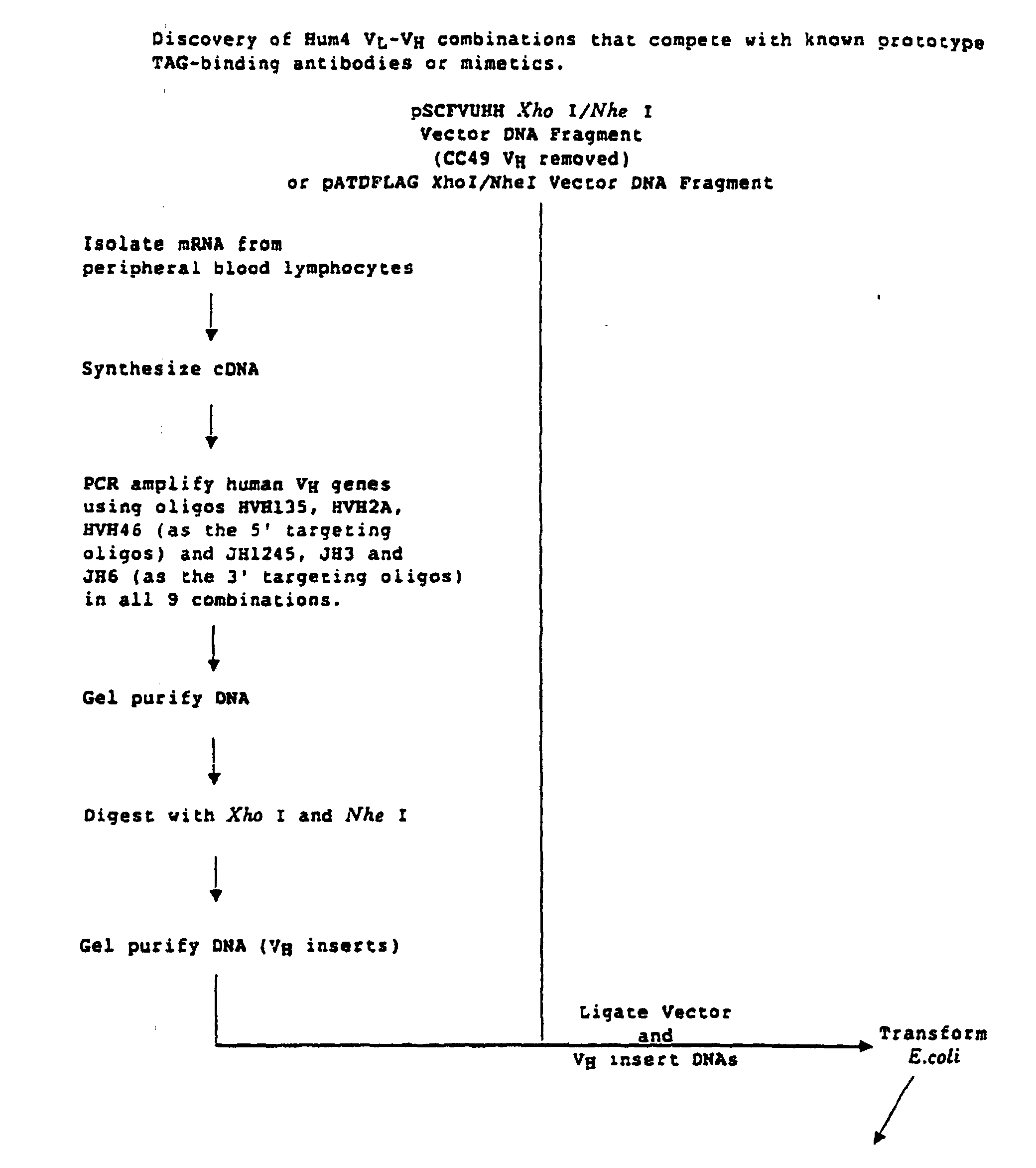

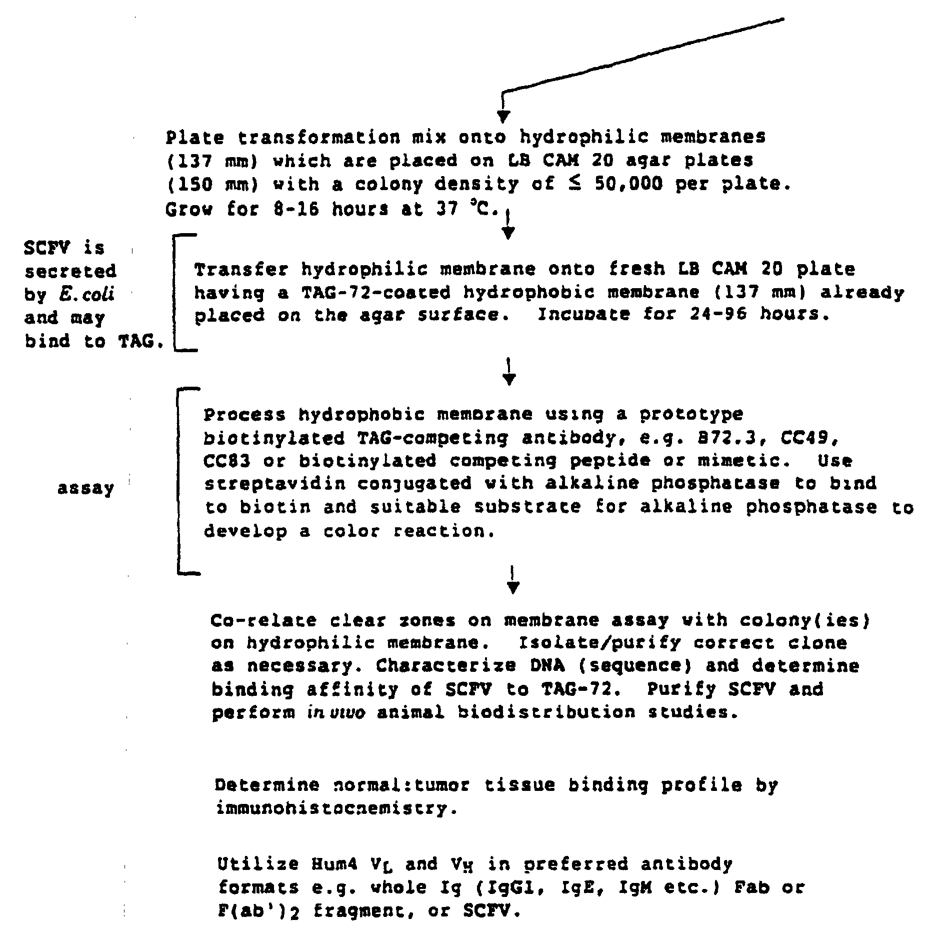

### Isolating total RNA from peripheral blood lymphocytes:

Blood from a normal, healthy donor is drawn into three 5 mL purple-cap Vacutainer tubes. Seven mL of blood are added to two 15 mL polypropylene tubes. An equal volume of lymphoprep (cat# AN5501, Accurate) is added and the solution is mixed by inversion. Both tubes are centrifuged at 1000 rpm and 18 °C for 20 minutes. The resulting white area near the top of the liquid (area not containing red blood cells) is removed from each sample and placed into two sterile polypropylene centrifuge tube. Ten mL of sterile PBS are added and the tube mixed by inversion. The samples are centrifuged at 1500 rpm and 18 °C for 20 minutes Total RNA is isolated from resulting pellet according to the RNAzol B Method (Chomczynski and Sacchi (1987), Analytical Biochemistry, **162**:156-159). Briefly, the cell pellets are lysed in 0.4 mL RNAzol solution (cat#:CS-105, Cinna/Biotecx). RNA is solubilized by passing the cell pellet through a 1 mL pipet tip. Sixty µL of chloroform are added and the solution is shaken for 15 seconds. RNA solutions are then placed on ice for 5 minutes. Phases are separated by centrifugation at 12000 x g and 4 °C for 15 minutes. The upper (aqueous) phases are transferred to fresh RNase-free microcentrifuge tubes. One volume of isopropanol is added and the samples placed at -20 °C for 1 hour. The samples are then placed on dry ice for 5 minutes and finally centrifuged for 40 seconds at 14,000 x g and 4 °C. The resulting supernatant is removed from each sample and the pellet is dissolved in 144 µL of sterile RNase-free water. Final molarity is brought to 0.2M NaCL. The DNA is reprecipitated by adding 2 volumes of 100% ethanol, leaving on dry ice for 10 minutes, and centrifugation at 14,000 rpm and 4 °C for 15 minutes. The supernatants are then removed, the pellets washed with 75% ethanol and centrifuged for 8 minutes at 12000 x g and 4 °C. The ethanol is then removed and the pellets dried under vacuum. The resulting RNA is then dissolved in 20 sterile water containing 1 µl RNasin (cat#:N2511, Promega).

### cDNA synthesis:

cDNA synthesis is performed using a Gene Amp™ PCR kit (cat#: N808-0017 Perkin Elmer Cetus), RNasin™ (cat#; N2511, Promega), and AMV reverse transcriptase (cat#: M9004, Promega). The following protocol is used for each sample:

| Components | Amount |
|---|---|
| MgCl₂ solution | 4 µl |
| 10 µl PCR buffer II | 2 µl |
| dATP | 2 µl |
| dCTP | 2 µl |
| dGTP | 2 µl |
| dTTP | 2 µl |
| 3' primer (random hexamers) | 1 µl |
| RNA sample | 2 µl |
| RNasin | 1 µl |
| AMV RT | 1.5 µl |

Samples are heated at 80 °C for 3 minutes then slowly cooled to 48 °C. The samples are then centrifuged for 10 seconds. AMV reverse transcriptase is added to the samples which are then incubated for 30 minutes at 37 °C. After incubation, 0.5 µl of each dNTP and 0,75 reverse transcriptase (cat#:109118, Boehringer Mannheim) are added. The samples are incubated for an additional 15 minutes at 37 °C.

### PCR Reaction:

Oligonucleotides are designed to amplify human VH genes by polymerase chain reaction. The 5' oligonucleotides are set forth below: 
HVH 135:
5'-TATTCTCGAGGTGCA(AG)CTG(CG)TG(CG)AGTCTGG-3'
 
HVH2A:
5'-TATTCTCGAGGTCAA(CG)TT(AG)A(AG)GGAGTCTGG-3'
 
HVH46:
5'-TATTCTCGAGGTACAGCT(AG)CAG(CG)(AT)GTC(ACG)GG-3'
 The 3' oligonucleotides are set forth below:

JH1245:
5'-TTATGCTAGCTGAGGAGAC(AG)GTGACCAGGG-3'

JH3:
5'-TTATGCTAGCTGAAGAGACGGTGACCATTG

JH6:
5'-TTATGCTAGCTGAGGAGACGGTGACCGTGG-3'

PCR reactions are performed with a GeneAmp™ PCR kit (cat#:N808-0017, Perkin Elmer Cetus). Components are listed below:

| Components | Amount |
|---|---|
| ddH₂O | 75 µl |
| 10 x buffer | 10 µl |
| dATP | 2 µl |
| dCTP | 2 µl |
| dGTP | 2 µl |
| dTTP | 2 µl |
| 1* Target DNA | 1 µl |
| 2* 5' primer | 2.5 µl |
| 3' primer | 2.0 µl |
| 3* AmpliTaq™ | 1.3 µl |
| Polymerase | |

| | |
|---|---|
| *components added in order at 92 °C of first cycle | |

| | | |
|---|---|---|
| PCR program: | step 1 | 94 °C for 30 seconds |
| | step 2 | 60 °C for 1 minutes |
| | step 3 | 72 °C for 45 seconds |

Approximately 35 cycles are completed for each reaction. All PCR reactions are performed using a Perkin Elmer Cetus PCR System 9600 thermal cycler.

### Treatment of Human V_{H} inserts with Xho I and Nhe I:

Human V_{H} genes are digested with *Xho* I (cat#: 131L, New England Biolabs) and *Nhe* I (cat#: 146L, New England Biolabs). The following protocol is used for each sample:

| SUBSTANCE | AMOUNT |
|---|---|
| DNA | 20 µl |
| NEB Buffer #2 | 4.5 µl |
| *Nhe* I | 2 µl |
| *Xho* I | 2 µl |
| ddH₂O | 16.5 µl |

Samples are incubated at 37 °C for one hour. After this incubation, an additional 1.5 µL *Nhe* I is added and samples are incubated an additional two hours at 37 °C.

### Purification of DNA:

After the restrictive enzyme digest, DNA is run on a 5 percent polyacrylamide gel (Sambrook *et al*. (1989), *supra).* Bands of 390-420 bp in size are excised from the gel. DNA is electroeluted and ethanol precipitated according to standard procedures.

PCR products resulting from oligonucleotide combinations are pooled together: JH1245 with HVH135, HVH2A and HVH46; JH3 with HVH135, HVH2A and HVH46; JH6 with HVH135, HVH2A and HVH46. The volume of the resulting pools are reduced under vacuum to 50 microliters. The pools are then purified from a 4 percent polyacrylamide gel (Sambrook *et al*. (1989), *supra*) to isolate DNA fragments. Bands resulting at 390-420 bp are excised from the gel. The DNA from excised gel slices is electroeluted according to standard protocols set forth in Sambrook, *supra*.

### Isolation of pSCFVUHH Xho I/Nhe I Vector Fragment

Approximately 5 µg in 15 µL of pSCFVUHH plasmid is isolated using the Magic Mini-prep™ system (Promega). To this is added 5.4 µL OF 10X Buffer #2 (New England Biolabs), 45 units of *Xho* I (New England Biolabs), 15 units of *Nhe* I and 24 µL of ddH₂O. The reaction is allowed to proceed for 1 hour at 37 °C. The sample is loaded on a 4% polyacrylamide gel, electrophoresed and purified by electroelution, as described earlier. The DNA pellet is dissolved in 20 µL of ddH₂O.

One hundred nanograms of pSCFVUHH digested with *Xho* I/*Nhe* I is ligated with a 1:1 molar ratio of purified human V_{H} inserts digested with *Xho* I and *Nhe* I using T4 DNA ligase (Stratagene). Aliquots are used to transform competent *E.coli* AG1 cells (Stratagene) according to the supplier's instructions.

GVWP hydrophilic membranes (cat# GVWP14250, Millipore) are placed on CAM 20 LB agar plates (Sambrook *et al*., 1989). One membrane is added to each plate. Four hundred microliters of the *E.coli* AG1 transformation suspension from above are evenly spread over the surface of each membrane. The plates are incubated for 16 hours at 37 °C ambient temperatures.

### Preparation of TAG-72-coated membranes:

A 1% dilution of partially purified tumor associated glycoprotein-72 (TAG-72) produced in LS174 T-cells is prepared in TBS (cat# 28376, Pierce). Ten milliliters of the TAG dilution are placed in a petri plate (cat# 8-757-14, Fisher) for future use. Immobilon-P PVDF transfer membranes (cat# SE151103, Millipore) are immersed in methanol. The membranes are than rinsed three times in sterile double distilled water. After the final wash, the excess water is allowed to drain. Each of the membranes are placed in 10 milliliters of dilute TAG-72. The membranes are incubated at ambient temperature from 1 hour with gentle shaking. After incubation, the membranes are blocked with Western blocking solution (25 mM Tris, 0.15 M NaCl, pH 7.6; 1% BSA) for about 1 hour at ambient temperature.

Blocking solution is drained from the TAG membranes. With the side exposed to TAG-72 facing up, the membranes are placed onto fresh CAM 20 plates. Resulting air pockets are removed. The bacterial membranes are then added, colony side up, to a TAG membrane. The agar plates are incubated for 24 to 96 hours at ambient temperatures.

The orientation of the TAG-72 and bacterial membranes are marked with permanent ink. Both membranes are removed from the agar surface. The TAG-72 membrane is placed in 20 ml of Western antibody buffer (TBS in 0.05% Tween-20, cat# P-1379, Sigma Chemical Co.; 1% BSA, cat#3203, Biocell Laboratories) containing 0.2 ng of CC49-Biotin probe antibody. The bacterial membranes are replaced on the agar surface in their original orientation and set aside. CC49-Biotin is allowed to bind to the TAG membranes for 1 hour at 31 °C with gentle shaking. The membranes are then washed three times with TTBS (TBS, 0.05% Tween-20) for 5 minutes on an orbital shaker at 300 rpm. Streptavidin alkaline phosphatase (cat# 7100-04, Southern Biotechnology Associates) is added to Western antibody buffer to produce a 0.1% solution. The TAG-72 membranes are each immersed in 16 milliliters of the streptavidin solution and allowed to incubate for 30 minutes at 31 °C with gentle shaking. After incubation, the membranes are washed as previously described. A final wash is then performed using Western alkaline phosphate buffer (8.4 g NaCO₃, 0.203 g MgCl₂-H₂O, pH 9.8), for 2 minutes at 200 rpm at ambient temperature. To develop the membranes, Western blue stabilized substrate (cat# S384B, Promega) is added to each membrane surface. After 30 minutes at ambient temperatures, development of the membranes is stopped by rinsing the membranes three times with ddH₂O. The membranes are then photographed. The membranes are then photograhed and clear zones are corelated with colonies on the hydrophilic membrane, set aside earlier. Colony(ies) are isolated for growth in culture and used to prepare plasmid DNA for sequencing and protein preparation to evaluate specificity and affinity.

### Identification of Hum4 V_{L}, human V_{H} combinations using pATDFLAG.

In a second assay system, Hum4 V_{L} - human V_{H} combinations are discovered that bind to TAG-72 according to the schematic, *supra,* except for the following: at the assay step, IBI MII antibody is used as a probe to detect any Hum4 V_{L} - V_{H} SCFV combinations that have bound to the hydrophobic membrane coated with TAG-72.

The plasmid pATDFLAG was generated from pSCFVUHH (see Figure 29) to incorporate a flag-coating sequence 3' of any human V_{H} genes to be expressed continguously with Hum4 V_{L}. The plasmid pATDFLAG, when digested with *Xho* I and *Nhe* I and purified becomes the human C_{H} discovery plasmid containing Hum4 V_{L} in this SCFV format. The plasmid pATDFLAG was generated as follows. Plasmid pSCFVUHH treated with *Xho* I and *Nhe* I (isolated and described above) was used in a ligation reaction with the annealed FLAG and FLAGNC oligonucleotides.

FLAGC:
5'-TCGAGACAATGTCGCTAGCGACTACAAGGACGATGATGACAAATAAAAAC-3'

FLAGNC:
5'-CTAGGTTTTTATTTGTCATCATCGTCCTTGTAGTCGCTAGCGACATTGTC-3'

Equimolar amounts (1 x 10⁻¹⁰ moles of each of the oligonucleotides FLAGC and FLAGNC were mixed together using a ligation buffer (Stratagene). The sample is heated to 94 °C and is allowed to cool to below 35 °C before use in the ligation reaction below.

### Ligation Reaction to Obtain pATDFLAG

| COMPONENT | AMOUNT |
|---|---|
| pSCFVUHH *Xho* I*lNhe* I vector | 1.5 µl |
| ANNEALED FLAGC/FLAGNC | 0.85 µl |
| 10X Ligation buffer | 2 µl |
| T4 DNA LIGASE | 1 µl |
| 10 MM ATP | 2 µl |
| ddH₂O | 12.65 µl |

The reaction is carried out using the following components and amounts according the ligation protocol disclosed above. *E coli* AG1 cells (Stratagene) are transformed with 3 µl of the above ligation reaction and colonies selected using CAM 20 plates. Clones having appropriate *Nhe* I, *Xho* I and *Nhe* I/*Xho* I restriction patterns are selected for DNA sequencing.

The oligonucleotide used to verify the sequence of the FLAG linker in PATDFLAG (see Figure 28) is called PENPTSEQ: 5'-CTTTATGTAAGATGATGTTTTG-3. This oligonucleotide is derived from the non-coding strand of the *pen*P terminator region. DNA sequencing is performed using Sequenase™ sequencing kit (U.S. Biochemical, Cleveland, OH) following the manufacturer's directions. The DNA and deduced amino acid sequences of the Hum4 V_{L} - UNIHOPE linker - FLAG peptide is shown in Figure 28.

### Generating pSC49FLAG

The CC49V_{H} is inserted into the sites of *Xho* I - *Nhe* I pATDFLAG (see Figure 29) and evaluated for biological activity with the purpose of serving as a positive control for the FLAG assay system to detect binding to TAG-72. The new plasmid, called pSC49FLAG (see Figure 29) is generated as follows. The plasmid pATDFLAG (5 mg, purified from a 2.5 ml culture by the Magic Miniprep™ system (Promega) is treated with *Xho* I and *Nhe* I and the large vector fragment purified as described above for pSCFVUHH. The CC49 V_{H} insert DNA fragment is obtained by PCR amplification from pSCFVUHH and oligonucleotides UNI3 as the 5' end oligonucleotide and SC49FLAG as the 3' end oligonucleotide. The resulting DNA and amino acid sequences of this SCFV antibody, with the FLAG peptide at the C-terminus, is shown in Figure 30. The PCR reaction is carried out using 100 pmol each of the oligonucleotides, 0.1 ng of pSCFVUHH target DNA (uncut) and the standard protocol and reagents provided by Perkin Elmer Cetus. The DNA is first gel purfied, then treated with *Xho* I and *Nhe* I to generate sticky ends and purified from a 4% polyacrylamide gel and electroeluted as described earlier. The DNA vector (pATDFLAG treated with *Xho* I and *Nhe* I) and the insert (CC49 V_{H} PCR product from pSCFVUHH treated with *Xho* I and *Nhe* I) are ligated in a 1:1 molar ratio, using 100 ng vector DNA (Stratagene kit) and used to transform *E.coli* AG1 competent cells (Stratagene) according to the manufacturer's directions. A colony with the correct plasmid DNA is picked as the pSC49FLAG clone.

### Ligation of pATDFLAG Vector with PCR Amplified Hum4 V_{H} Inserts

The protocol for the ligation reaction is as follows:

| COMPONENT | AMOUNT |
|---|---|
| DNA vector:pATDFLAG *Xho* I/*Nhe* I | 2.5 µL |
| Hum V_{H} (X) DNA inserts: *Xho* I/*Nhe* I | 6 µL |
| 10 mM ATP (Stratagene) | 2 µL |
| 10X buffer (Stratagene) | 2 µL |
| T4 DNA ligase (Stratagene) | 1 µL |
| ddH₂O | 6.5 µL |

DNA vector, ATP, 10X buffer and ddH₂O are combined. DNA insert and T4 DNA ligase are then added. Ligation reactions are then placed in a 4 L beaker containing H₂O at 18 °C. The temperature of the water is gradually reduced by refrigeration at 4 °C overnight. This ligation reaction generates pHum4 V_{L} - hum V_{H} (X).

### Transformation of E.coli AG1 with pHum4 V_{L}-Hum V_{H} (X) Ligation Mix

Transformation of pATDFLAG into competent *E.coli* AG1 cells (Stratagene, La Jolla) is achieved following the supplier's protocol.

### IBI MII Anti-FLAG Antibody Plate Assay

The first three steps, preparation of TAG-coated membranes, plating of bacterial membranes, and assembly of TAG and bacterial membranes, are the same as those described in the CC49-Biotin Competition Plate Assay.

After the 24 hour incubation at ambient temperatures, the membranes are washed with TTBS three times at 250 rpm for four minutes. The MII antibody (cat# IB13010, International Biotechnologies, Inc.) is then diluted with TBS to a concentration ranging from 10.85 µg/ml to 0.03 µg/ml. Ten millilters of the diluted antibody are added to each membrane. The membranes are then incubated for 1 hour at ambient temperatures and shaken on a rotary shaker at 70 rpm. After incubation, the MII antibody is removed and the membranes are washed three times at 250 rpm and ambient temperatures for 5 minutes. The final wash is removed and 20 milliters of a 1:2000 dilution of sheep anti-mouse horseradish peroxidase linked whole antibody (cat# NA931, Amersham) is prepared with TBS and added to each membrane. The membranes are again incubated for 1 hour at ambient temperatures and 70 rpm. Following incubation, the membranes are washed three times at 250 rpm and ambient temperature for 5 minutes each. Enzygraphic Webs (cat# IB8217051, International Biotechnologies, Inc.) are used according to develop the membranes, according to the manufacturer's instructions. The membranes are then photographed.

Instead of seeing a clear zone on the developed membrane for a positive Hum4 V_{L}-V_{H} (X) clone producing an SCFV that binds to TAG-72, (as seen with the competition screening assay) in this direct FLAG - detecting assay, a blue-purple spot is indicative of a colony producing a SCFV that has bound to the TAG-72 coated membrane. The advantage of using the FLAG system is that any Hum4 V_{L} - V_{H} SCFV combination that has bound to TAG-72 will be detected. Affinities can be measured by Scatchard analysis (Scatchard (1949), *supra*) and specificity by immunohistochemistry. These canidates could then be checked for binding to a specific epitope by using the competition assay, *supra,* and a competing antibody or mimetic, if desired.

## Claims

1. A composite Hum4 V_{L}, V_{H} antibody or immunoreactive fragment thereof having binding affinity for TAG-72, comprising:-
a) a light chain variable region (V_{L}) of human kappa Subgroup IV, said V_{L} containing the human Subgroup IV germline gene (Hum4 V_{L}) encoded amino acid sequence, 
Asp Ile Val Met Thr Gln Ser Pro Asp Ser Leu Ala Val
Ser Leu Gly Glu Arg Ala Thr Ile Asn Cys Lys Ser Ser
Gln Ser Val Leu Tyr Ser Ser Asn Asn Lys Asn Tyr Leu
Ala Trp Tyr Gln Gln Lys Pro Gly Gln Pro Pro Lys Leu
Leu Ile Tyr Trp Ala Ser Thr Arg Glu Ser Gly Val Pro
Asp Arg Phe Ser Gly Ser Gly Ser Gly Thr Asp Phe Thr
Leu Thr Ile Ser Ser Leu Gln Ala Glu Asp Val Ala Val
Tyr Tyr Cys Gln Gln Tyr Tyr Ser Tyr Pro Leu Thr Phe
Gly Gly Gly Thr Lys Val Val Ile Lys,
 and
b) a heavy chain variable region (V_{H}), said V_{H} being encoded by a DNA coding sequence at least 90% homologous to the V_{H}αTAG germline gene (V_{H}αTAG) coding sequence,
wherein the V_{H} is combined with the V_{L} to form a three dimensional antigen binding structure retaining at least the same ability to bind TAG-72, as does a three dimensional antigen binding structure formed by combination of the V_{H} encoded by V_{H}αTAG and the V_{L} encoded by Hum4V_{L}.

2. The composite Hum4V_{L}, V_{H} antibody or immunoreactive fragment thereof of Claim 1, wherein the V_{L} is further encoded by a human J gene segment.

3. The composite Hum4 V_{L} V_{H} antibody or immunoreactive fragment thereof of claim 1 or claim 2, wherein the V_{H} is further encoded by a non-human animal D gene segment and an animal J gene segment.

4. The composite Hum4 V_{L} V_{H} antibody or immunoreactive fragment thereof of any one of Claims 1 to 3, wherein the heavy chain variable region (V_{H}) is from the heavy chain variable region of any one of CC46, CC49, CC83 or CC92.

5. The composite Hum4 V_{L}, V_{H} antibody or immunoreactive fragment thereof of any preceding claim, wherein the V_{H} comprises (1) complementarity determining regions (CDR) being encoded by V_{H}αTAG and (2) framework segments, adjacent to the CDR segments, encoded by human genes.

6. The composite Hum4 V_{L}, V_{H} antibody or immunoreactive fragment thereof of any preceding claim, wherein the light chain further comprises a human light chain constant region (C_{L}) and the heavy chain further comprises an animal heavy chain region (C_{H}).

7. The composite Hum4 V_{L}, V_{H} antibody or immunoreactive fragments thereof of Claim 6, wherein the C_{H} is IgG1-4, IgM, IgA1, IgA2, IgD or IGE.

8. The composite Hum4 V_{L}, V_{H} antibody or immunoreactive fragment thereof of Claim 6, wherein C_{L} is kappa or lambda.

9. A composite Hum4 V_{L}, V_{H} single chain antibody or immuno-reactive fragment thereof having binding affinity for TAG-72, comprising (a) a light chain having a variable region (V_{L}) as defined in Claim 1, (b) a heavy chain variable region (V_{H}) as defined in Claim 1 and (c) a polypeptide linker linking the V_{H} and V_{L}, wherein the polypeptide linker properly folds the V_{H} and V_{L} into a single chain antibody having the ability to bind TAG-72.

10. A composite Hum4 V_{L}, V_{H} antibody conjugate comprising the composite Hum4 V_{L}, V_{H} antibody or immunoreactive fragment thereof of any one of Claims 1 to 9, conjugated to an imaging marker or a therapeutic agent.

11. The composite Hum4 V_{L}, V_{H} antibody conjugate of Claim 10, wherein the imaging marker is ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁶Re, or ^{99m}Tc.

12. The composite Hum4 V_{L}, V_{H} antibody conjugate of Claim 10, wherein the therapeutic agent is a drug or biological response modifier, radionuclide, or toxin.

13. The composite Hum4 V_{L}, V_{H} antibody conjugate of Claim 12, wherein the drug is methotrexate, adriamycin or interferon.

14. The composite Hum4 V_{L}, V_{H} antibody conjugate of Claim 12, wherein the radionuclide is ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I or ¹¹¹In.

15. A composition comprising a composite Hum4 V_{L}, V_{H} antibody or immunoreactive fragment thereof of any one of Claims 1 to 9, in a pharmaceutically acceptable, non-toxic, sterile carrier.

16. A composition comprising a composite Hum4 V_{L}, V_{H} antibody conjugate of any one of Claims 10 to 14 in a pharmaceutically acceptable, non-toxic, sterile carrier.

17. A composition as claimed in Claim 15 or Claim 16 for use as a diagnostic or therapeutic agent.

18. A cell capable of expressing a composite Hum4 V_{L}, V_{H} antibody or immunoreactive fragment thereof of any one of Claims 1 to 9, said cell being transformed with
(a) a first DNA sequence encoding at least a light chain variable region (V_{L}) as defined in Claim 1; and
(b) a second DNA sequence encoding at least a heavy chain variable region (V_{H}) as defined in Claim 1, capable of combining with the V_{L} to form a three-dimensional structure having the ability to bind TAG-72.

19. The cell of Claim 18 wherein the first and second DNA sequences are contained within at least one biologically functional expression vector.

20. A process for producing a composite Hum4 V_{L}, V_{H} antibody comprising at least the variable domains of the antibody heavy and light chains, in a single host cell, comprising the steps of:
(a) transforming at least one host cell with
(i) a first DNA sequence encoding at least a light chain variable region (V_{L}) as defined in Claim 1; and
(ii) a second DNA sequence encoding at least a heavy chain variable region (V_{H}) as defined in Claim 1 which is capable of combining with the V_{L} to form a three-dimensional structure having the ability to bind TAG-72, and
(b) expressing said first DNA sequence and said second DNA sequence in said transformed single host cell.

21. The process according to Claim 20, wherein said first and second DNA sequences are present in at least one vector.

22. The process according to Claim 20 or Claim 21, wherein the antibody heavy and light chains of the composite Hum4 V_{L}, V_{H} antibody are expressed in the host cell and are secreted therefrom as an immunologically functional antibody molecule or antibody fragment.

23. The process of any one of Claims 20 to 22, wherein the second DNA sequence encodes the V_{H} of CC46, CC49, CC83 or CC92.

24. A process for preparing an antibody or antibody fragment conjugate which comprises contacting:
a composite Hum4 V_{L}, V_{H} antibody of any one of Claims 1 to 9 with an imaging marker or therapeutic agent.

25. The process of Claim 24, wherein the imaging marker is ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re, or ^{99m}Tc.

26. The process of Claim 24, wherein the therapeutic agent is a radionuclide, drug or biological response modifier, toxin or another antibody.

27. The use of a composition as claimed in Claim 15 or Claim 16 in the manufacture of a diagnostic agent or medicament for *in vivo* diagnosis or therapy of cancer.

## Patentansprüche

1. Zusammengesetzter Hum4-V_{L}/V_{H}-Antikörper oder immunreaktives Fragment davon mit Bindeaffinität für TAG-72 umfassend:
a) eine variable Region einer leichten Kette (V_{L}) der humanen Kappa-Untergruppe IV, wobei die V_{L} die von dem humanen Untergruppe-IV-Keimbahngen (Hum4-V_{L}) kodierte Aminosäuresequenz enthält, 
Asp Ile Val Met Thr Gln Ser Pro Asp Ser Leu Ala Val
Ser Leu Gly Glu Arg Ala Thr Ile Asn Cys Lys Ser Ser
Gln Ser Val Leu Tyr Ser Ser Asn Asn Lys Asn Tyr Leu
Ala Trp Tyr Gln Gln Lys Pro Gly Gln Pro Pro Lys Leu
Leu Ile Tyr Trp Ala Ser Thr Arg Glu Ser Gly Val Pro
Asp Arg Phe Ser Gly Ser Gly Ser Gly Thr Asp Phe Thr
Leu Thr Ile Ser Ser Leu Gln Ala Glu Asp Val Ala Val
Tyr Tyr Cys Gln Gln Tyr Tyr Ser Tyr Pro Leu Thr Phe
Gly Gly Gly Thr Lys Val Val Ile Lys,
 und
b) eine variable Region einer schweren Kette (V_{H}), wobei die V_{H} von einer DNA-kodierenden Sequenz kodiert wird, die mindestens zu 90% homolog zur kodierenden Sequenz des V_{H}αTAG-Keimbahngens (V_{H}αTAG) ist,
wobei die V_{H} mit der V_{L} kombiniert wird, so dass eine dreidimensionale Antigen-bindende Struktur gebildet wird, die mindestens die gleiche Fähigkeit, TAG-72 zu binden, beibehält wie eine dreidimensionale Antigen-bindende Struktur, die durch Kombination der durch V_{H}αTAG kodierten V_{H} und der durch Hum4-V_{L} kodierten V_{L} gebildet wird.

2. Zusammengesetzter Hum4-V_{L}/V_{H}-Antikörper oder immunreaktives Fragment davon nach Anspruch 1, wobei die V_{L} des Weiteren durch ein humanes J-Gen-Segment kodiert wird.

3. Zusammengesetzter Hum4-V_{L}/V_{H}-Antikörper oder immunreaktives Fragment davon nach Anspruch 1 oder Anspruch 2, wobei die V_{H} des Weiteren durch ein nicht-humanes, tierisches D-Gen-Segment und ein tierisches J-Gen-Segment kodiert wird.

4. Zusammengesetzter Hum4-V_{L}/V_{H}-Antikörper oder immunreaktives Fragment davon nach einem der Ansprüche 1 bis 3, wobei die variable Region einer schweren Kette (V_{H}) von der variablen Region einer schweren Kette von CC46, CC49, CC83 oder CC92 abstammt.

5. Zusammengesetzter Hum4-V_{L}/V_{H}-Antikörper oder immunreaktives Fragment davon nach einem der vorhergehenden Ansprüche, wobei die V_{H} (1) Komplementaritätbestimmende Regionen (CDR), die durch V_{H}αTAG kodiert sind, und (2) den CDR-Segmenten benachbarte Rahmensegmente, die durch humane Gene kodiert werden, umfasst.

6. Zusammengesetzter Hum4-V_{L}/V_{H}-Antikörper oder immunreaktives Fragment davon nach einem der vorhergehenden Ansprüche, wobei die leichte Kette des Weiteren eine konstante Region einer humanen leichten Kette (C_{L}) umfasst und die schwere Kette des Weiteren eine Region einer tierischen schweren Kette (C_{H}) umfasst.

7. Zusammengesetzter Hum4-V_{L}/V_{H}-Antikörper oder immunreaktives Fragment davon nach Anspruch 6, wobei die C_{H} IgG1-4, IgM, IgA1, IgA2, IgD oder IgE ist.

8. Zusammengesetzter Hum4-V_{L}/V_{H}-Antikörper oder immunreaktives Fragment davon nach Anspruch 6, wobei C_{L} Kappa oder Lambda ist.

9. Einkettiger zusammengesetzter Hum4-V_{L}/V_{H}-Antikörper oder immunreaktives Fragment davon mit Bindeaffinität für TAG-72, umfassend (a) eine leichte Kette mit einer variablen Region (V_{L}), wie in Anspruch 1 definiert, (b) eine variable Region einer schweren Kette (V_{H}), wie in Anspruch 1 definiert, und (c) einen Polypeptid-Linker, der die V_{H} und V_{L} verbindet, wobei der Polypeptid-Linker die V_{H} und V_{L} in passender Weise zu einem einkettigen Antikörper faltet, der die Fähigkeit, TAG-72 zu binden, aufweist.

10. Zusammengesetztes Hum4-V_{L}/V_{H}-Antikörper-Konjugat umfassend den zusammengesetzten Hum4-V_{L}/V_{H}-Antikörper oder das immunreaktive Fragment davon nach einem der Ansprüche 1 bis 9, mit einem bildgebenden Marker oder einem therapeutischen Mittel konjugiert.

11. Zusammengesetztes Hum4-V_{L}/V_{H}-Antikörper-Konjugat nach Anspruch 10, wobei der bildgebende Marker ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re oder ^{99m}Tc ist.

12. Zusammengesetztes Hum4-V_{L}/V_{H}-Antikörper-Konjugat nach Anspruch 10, wobei das therapeutische Mittel ein Wirkstoff oder Modifikator einer biologischen Antwort, ein Radionuklid oder ein Toxin ist.

13. Zusammengesetztes Hum4-V_{L}/V_{H}-Antikörper-Konjugat nach Anspruch 12, wobei der Wirkstoff Methotrexat, Adriamycin oder Interferon ist.

14. Zusammengesetztes Hum4-V_{L}/V_{H}-Antikörper-Konjugat nach Anspruch 12, wobei das Radionuklid ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I oder ¹¹¹In ist.

15. Zusammensetzung umfassend einen zusammengesetzten Hum4-V_{L}/V_{H}-Antikörper oder ein immunreaktives Fragment davon nach einem der Ansprüche 1 bis 9 in einem pharmazeutisch akzeptablen, nicht-toxischen, sterilen Träger.

16. Zusammensetzung umfassend ein zusammengesetztes Hum4-V_{L}/V_{H}-Antikörper-Konjugat nach einem der Ansprüche 10 bis 14 in einem pharmazeutisch akzeptablen, nicht-toxischen, sterilen Träger.

17. Zusammensetzung nach Anspruch 15 oder Anspruch 16 zur Verwendung als diagnostisches oder therapeutisches Mittel.

18. Zelle, die einen zusammengesetzten Hum4-V_{L}/V_{H}-Antikörper oder ein immunreaktives Fragment davon nach einem der Ansprüche 1 bis 9 exprimieren kann, wobei die Zelle transformiert ist mit
(a) einer ersten DNA-Sequenz, die mindestens eine variable Region einer leichten Kette (V_{L}), wie in Anspruch 1 definiert, kodiert, und
(b) einer zweiten DNA-Sequenz, die mindestens eine variable Region einer schweren Kette (V_{H}), wie in Anspruch 1 definiert, kodiert, die mit der V_{L} kombinieren kann, um eine dreidimensionale Struktur mit der Fähigkeit, TAG-72 zu binden, auszubilden.

19. Zelle nach Anspruch 18, wobei die ersten und zweiten DNA-Sequenzen in mindestens einem biologisch funktionalen Expressionsvektor enthalten sind.

20. Verfahren zur Herstellung eines zusammengesetzten Hum4-V_{L}/V_{H}-Antikörpers, der mindestens die variablen Domänen der schweren und leichten Ketten des Antikörpers umfasst, in einer einzigen Wirtszelle, umfassend die Schritte:
(a) Transformieren mindestens einer Wirtszelle mit
(i) einer ersten DNA-Sequenz, die mindestens eine variable Region einer leichten Kette (V_{L}), wie in Anspruch 1 definiert, kodiert, und
(ii) einer zweiten DNA-Sequenz, die mindestens eine variable Region einer schweren Kette (V_{H}), wie in Anspruch 1 definiert, kodiert, die mit der V_{L} kombinieren kann, um eine dreidimensionale Struktur mit der Fähigkeit, TAG-72 zu binden, auszubilden, und
(b) Exprimieren der ersten DNA-Sequenz und der zweiten DNA-Sequenz in der transformierten einzigen Wirtszelle.

21. Verfahren nach Anspruch 20, wobei die ersten und zweiten DNA-Sequenzen in mindestens einem Vektor vorhanden sind.

22. Verfahren nach Anspruch 20 oder Anspruch 21, wobei die schweren und leichten Ketten des zusammengesetzten Hum4-V_{L}/V_{H}-Antikörpers in der Wirtszelle exprimiert und daraus als ein immunologisch funktionales Antikörper-Molekül oder Antikörper-Fragment sezerniert werden.

23. Verfahren nach einem der Ansprüche 20 bis 22, wobei die zweite DNA-Sequenz die V_{H} von CC46, CC49, CC83 oder CC92 kodiert.

24. Verfahren zur Herstellung eines Antikörper- oder Antikörperfragment-Konjugates, das das Kontaktieren eines zusammengesetzten Hum4-V_{L}/V_{H}-Antikörpers nach einem der Ansprüche 1 bis 9 mit einem bildgebenden Marker oder therapeutischen Mittel umfasst.

25. Verfahren nach Anspruch 24, wobei der bildgebende Marker ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re oder ^{99m}Tc ist.

26. Verfahren nach Anspruch 24, wobei das therapeutische Mittel ein Radionuklid, Wirkstoff oder Modifikator einer biologischen Antwort, ein Toxin oder ein anderer Antikörper ist.

27. Verwendung einer Zusammensetzung, wie in Anspruch 15 oder Anspruch 16 beansprucht, für die Herstellung eines diagnostischen Mittels oder Medikaments zur in vivo Diagnose oder Therapie von Krebs.

## Revendications

1. Anticorps composite Hum4-V_{L},V_{H} ou fragment immunoréactif d'un tel anticorps, présentant une affinité de liaison pour l'anti-gène TAG-72 et comportant :
a) une région variable de chaîne légère (région V_{L}) du sous-groupe IV humain de chaîne kappa, laquelle région V_{L} contient la séquence d'acides aminés codés par le gène "germline" Hum4-V_{L} du sous-groupe IV humain : 
Asp Ile Val Met Thr Gln Ser Pro Asp Ser Leu Ala Val
Ser Leu Gly Glu Arg Ala Thr Ile Asn Cys Lys Ser Ser
Gln Ser Val Leu Tyr Ser Ser Asn Asn Lys Asn Tyr Leu
Ala Trp Tyr Gln Gln Lys Pro Gly Gln Pro Pro Lys Leu
Leu Ile Tyr Trp Ala Ser Thr Arg Glu Ser Gly Val Pro
Asp Arg Phe Ser Gly Ser Gly Ser Gly Thr Asp Phe Thr
Leu Thr Ile Ser Ser Leu Gln Ala Glu Asp Val Ala Val
Tyr Tyr Cys Gln Gln Tyr Tyr Ser Tyr Pro Leu Thr Phe
Gly Gly Gly Thr Lys Val Val Ile Lys,
b) et une région variable de chaîne lourde (région V_{H}), laquelle région V_{H} est codée par une séquence codante d'ADN présentant au moins 90 % d'homologie avec la séquence codante du gène "germline" V_{H}αTAG,
et dans lequel anticorps composite la région V_{H} est combinée avec la région V_{L} de manière à constituer une structure tridimensionnelle de liaison à l'antigène qui conserve une capacité à se lier à l'antigène TAG-72 au moins identique à celle d'une structure tridimensionnelle de liaison à l'antigène constituée par combinaison de la région V_{H} codée par le gène V_{H}αTAG et de la région V_{L} codée par le gène Hum4-V_{L}.

2. Anticorps composite Hum4-V_{L},V_{H} ou fragment immunoréactif d'un tel anticorps, conforme à la revendication 1, dans lequel la région V_{L} est en outre codée par un segment de gène J humain.

3. Anticorps composite Hum4-V_{L},V_{H} ou fragment immunoréactif d'un tel anticorps, conforme à la revendication 1 ou 2, dans lequel la région V_{H} est en outre codée par un segment de gène D animal non-humain et un segment de gène J animal.

4. Anticorps composite Hum4-V_{L},V_{H} ou fragment immunoréactif d'un tel anticorps, conforme à l'une des revendications 1 à 3, dans lequel la région variable de chaîne lourde V_{H} vient de la région variable de chaîne lourde de l'un des anticorps CC46, CC49, CC83 et CC92.

5. Anticorps composite Hum4-V_{L},V_{H} ou fragment immunoréactif d'un tel anticorps, conforme à l'une des revendications précédentes, dans lequel la région V_{H} comporte
1) des régions CDR (Complementarity Determining Regions) codées par le gène V_{H}αTAG,
2) des segments FR (Framework Regions), adjacents aux segments CDR, codés par des gènes humains.

6. Anticorps composite Hum4-V_{L},V_{H} ou fragment immunoréactif d'un tel anticorps, conforme à l'une des revendications précédentes, dans lequel la chaîne légère comporte en outre une région constante C_{L} de chaîne légère humaine et la chaîne lourde comporte en outre une région constante C_{H} de chaîne lourde animale.

7. Anticorps composite Hum4-V_{L},V_{H} ou fragment immunoréactif d'un tel anticorps, conforme à la revendication 6, dans lequel la région C_{H} est celle de l'une des immunoglobulines IgG1 à IgG4, IgM, IgA1, IgA2, IgD ou IgE.

8. Anticorps composite Hum4-V_{L},V_{H} ou fragment immunoréactif d'un tel anticorps, conforme à la revendication 6, dans lequel la région C_{L} est celle d'une chaîne kappa ou lambda.

9. Anticorps composite Hum4-V_{L},V_{H} à chaîne unique ou fragment immunoréactif d'un tel anticorps, présentant une affinité de liaison pour l'antigène TAG-72 et comportant :
a) une chaîne légère comportant une région variable V_{L} définie dans la revendication 1,
b) une région variable de chaîne lourde V_{H} définie dans la revendication 1,
c) et un raccord polypeptidique qui relie les régions V_{H} et V_{L} et qui permet à ces régions V_{H} et V_{L} de se replier de manière appropriée en un anticorps à chaîne unique qui peut se lier à l'antigène TAG-72.

10. Conjugué d'anticorps composite Hum4-V_{L},V_{H}, comportant un anticorps composite Hum4-V_{L},V_{H} ou un fragment immunoréactif d'un tel anticorps, conforme à l'une des revendications 1 à 9, conjugué avec un marqueur d'imagerie ou un agent thérapeutique.

11. Conjugué d'anticorps composite Hum4-V_{L},V_{H}, conforme à la revendication 10, dans lequel le marqueur d'imagerie est ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re ou ^{99m}Tc.

12. Conjugué d'anticorps composite Hum4-V_{L},V_{H}, conforme à la revendication 10, dans lequel l'agent thérapeutique est un médicament, un modificateur de réponse biologique, un radionucléide ou une toxine.

13. Conjugué d'anticorps composite Hum4-V_{L},V_{H}, conforme à la revendication 12, dans lequel le médicament est du méthotrexate, de l'adriamycine ou un interféron.

14. Conjugué d'anticorps composite Hum4-V_{L},V_{H}, conforme à la revendication 12, dans lequel le radionucléide est ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I ou ¹¹¹In.

15. Composition comprenant un anticorps composite Hum4-V_{L},V_{H} ou un fragment immunoréactif d'un tel anticorps, conforme à l'une des revendications 1 à 9, dans un véhicule admissible en pharmacie, non-toxique et stérile.

16. Composition comprenant un conjugué d'anticorps composite Hum4-V_{L},V_{H}, conforme à l'une des revendications 10 à 14, dans un véhicule admissible en pharmacie, non-toxique et stérile.

17. Composition conforme à la revendication 15 ou 16, utilisable comme agent diagnostique ou thérapeutique.

18. Cellule capable d'exprimer un anticorps composite Hum4-V_{L},V_{H} ou un fragment immunoréactif d'un tel anticorps, conforme à l'une des revendications 1 à 9, laquelle cellule a été transformée avec
a) une première séquence d'ADN codant au moins une région variable de chaîne légère V_{L} définie dans la revendication 1,
b) et une deuxième séquence d'ADN codant au moins une région variable de chaîne lourde V_{H} définie dans la revendication 1, capable de se combiner avec la région V_{L} pour former une structure tridimensionnelle qui peut se lier à l'antigène TAG-72.

19. Cellule conforme à la revendication 18, dans laquelle les première et deuxième séquences d'ADN se trouvent au sein d'au moins un vecteur d'expression biologiquement fonctionnel.

20. Procédé de production d'un anticorps composite Hum4-V_{L},V_{H} comprenant au moins les régions variables des chaînes lourdes et légères d'anticorps, dans une cellule hôte unique, lequel procédé comporte les étapes suivantes :
a) transformer au moins une cellule hôte avec
- une première séquence d'ADN codant au moins une région variable de chaîne légère V_{L} définie dans la revendication 1,
- et une deuxième séquence d'ADN codant au moins une région variable de chaîne lourde V_{H} définie dans la revendication 1, capable de se combiner avec la région V_{L} pour former une structure tridimensionnelle qui peut se lier à l'antigène TAG-72 ;
b) et faire exprimer ces première et deuxième séquences d'ADN dans ladite cellule hôte unique transformée.

21. Procédé conforme à la revendication 20, dans lequel les première et deuxième séquences d'ADN se trouvent au sein d'au moins un vecteur.

22. Procédé conforme à la revendication 20 ou 21, dans lequel les chaînes lourdes et les chaînes légères de l'anticorps composite Hum4-V_{L},V_{H} sont exprimées dans la cellule hôte et sont sécrétées par celle-ci sous la forme d'une molécule d'anticorps ou d'un fragment d'anti-corps, fonctionnel du point de vue immunologique.

23. Procédé conforme à l'une des revendications 20 à 22, dans lequel la deuxième séquence d'ADN code la région V_{H} de l'un des anticorps CC46, CC49, CC83 et CC92.

24. Procédé de préparation d'un conjugué d'anticorps ou de fragment d'anticorps, qui comporte le fait de mettre un anticorps composite Hum4-V_{L},V_{H} conforme à l'une des revendications 1 à 9 en contact avec un marqueur d'imagerie ou un agent thérapeutique.

25. Procédé conforme à la revendication 24, dans lequel le marqueur d'imagerie est ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re ou ^{99m}Tc.

26. Procédé conforme à la revendication 24, dans lequel l'agent thérapeutique est un radionucléide, un médicament, un modificateur de réponse biologique, une toxine ou un autre anticorps.

27. Emploi d'un composition conforme à la revendication 15 ou 16 dans la fabrication d'un agent de diagnostic ou d'un médicament, destiné au diagnostic *in vivo* ou au traitement d'un cancer.
